# EUROPÄISCHE PATENTSCHRIFT

(11) **EP 2 274 390 B1**
(45) Veröffentlichungstag und Bekanntmachung des Hinweises auf die Patenterteilung: **30.07.2014**
(21) Anmeldenummer: 09738219.6
(22) Anmeldetag: 30.04.2009
(51) Int. Cl.: C09J 7/00

(54) **KLEBEBAND**
ADHESIVE TAPE
BANDE ADHÉSIVE

(30) Priorität: 30.04.2008 DE 102008021739; 30.04.2008 DE 102008021742; 30.04.2008 DE 102008021744; 30.04.2008 DE 102008021743; 30.04.2008 DE 102008021741; 30.05.2008 DE 102008025979; 30.05.2008 DE 102008025984; 30.05.2008 DE 102008025980; 30.05.2008 DE 102008025982
(43) Veröffentlichungstag der Anmeldung: 19.01.2011
(73) Patentinhaber: TESA SE, 20253 Hamburg (DE)
(72) Erfinder: MÜSSIG, Bernhard, 21218 Seevetal (DE); SEITZER, Dennis, 72764 Reutlingen (DE); KÜLPER, Klaus, 25421 Pinneberg (DE); BEHRENS, Nicole, 81245 München (DE)
(86) Internationale Anmeldenummer: PCT/EP2009/055275
(87) Internationale Veröffentlichungsnummer: WO 2009/133175

(56) Entgegenhaltungen:
- EP-A- 1 308 271
- EP-A- 1 318 180
- EP-A- 1 719 808
- WO-A-02/14448
- WO-A-99/38960

## Beschreibung

Die Erfindung betrifft ein Klebeband und die Verwendung desselben.

Klebebänder werden üblicherweise mit Klebemassen auf Basis Naturkautschuk, Styrolblockcopolymer oder Acrylat gefertigt.

Kautschukklebemassen bestehen üblicherweise aus einem Elastomer, einem Klebharz, einem Weichmacher und einem phenolischen Antioxidans. Das häufigste Elastomer ist Naturkautschuk, und die gängigsten synthetische Elastomere sind Styrol-Dien-Blockcopolymere, insbesondere Styrol-Isopren-Styrol-Blockcopolymere. Als Weichmacher wird in der Regel ein Mineralöl eingesetzt, meistens ein Weißöl oder seltener ein aromatisches Öl. Für einige Anwendungen sind solche Öle unerwünscht, zum Beispiel für Oberflächenschutzprodukte (Ghosting auf dem Lack nach dem Entfernen), für den Kraftfahrzeuginnenbereich (Fogging) oder in Papierklebebändern (Durchfettung des Papierträgers nach Lagerung), in diesen Fällen verwendet man ein Flüssig- oder Weichharz mit einem Schmelzpunkt von 10 °C bis 40 °C, welches die teuerste Komponente der Rezeptur darstellt.
Kautschukklebemassen sind relativ wenig alterungs- und UV-beständig und weisen eine schlechte Verträglichkeit mit Drahtisolierungen auf. Hydrierte Styrol-Dien-Blockcopolymere schaffen hier Abhilfe, sind aber extrem teuer und erreichen nur relativ geringe Klebkräfte.

Die Naturkautschuk-Klebemassen sind lösungsmittelhaltig und weisen eine geringe alterungs- und UV-Stabilität auf.

Styrolblockcopolymer-Klebemassen, in der Regel basierend auf Styrol-Isopren-Sytrol-Blockcopolymeren, sind lösungsmittelfrei verarbeitbar, weisen aber ebenfalls eine geringe alterungs- und UV-Stabilität auf. Darüber hinaus sind sie sehr hart, so dass diese Klebebänder nur mit einem lauten Abrollgeräusch verarbeitbar sind.

Acrylatklebemassen sind Dispersionen und somit lösungsmittelfrei und weisen eine gute alterungs- und UV-Stabilität auf, zeigen jedoch eine erhöhte Empfindlichkeit gegen Wasser und vor allem eine schwache Anfangshaftung (Tack) bei Verklebungen auf Karton oder Papier sowie eine schlechte Haftung auf unpolaren Untergründen. Sie sind daher für viele Permanentanwendungen ungeeignet. Sie sind von sehr polaren Untergründen wie Aluminium oder PVC nicht wieder entfernbar und daher für solche Maskierungsanwendungen ungeeignet. Acrylatklebemassen sind nicht preisgünstig.

Es besteht schon lange der Wunsch nach einer Klebmasse, welche die positiven Eigenschaften aller dieser Klebmassen miteinander kombiniert:
Lösungsmittelfreiheit, Wasserbeständigkeit, hohe Anfangshaftung, hohe Haftung auf niederenergetischen Oberflächen, Abrollverhalten und Wiederablösbarkeit wie Naturkautschuk-Klebemassen und alterungs- und UV-Stabilität wie Acrylatklebemassen.

Aufgabe der Erfindung ist es, ein Klebeband mit einer derartigen Klebemasse zur Verfügung zu stellen.

Gelöst wird die Aufgabe durch ein Klebeband, wie es im Hauptanspruch niedergelegt ist. Vorteilhafte Weiterbildungen des Erfindungsgegenstands sowie Verwendungen des Klebebands finden sich in den Unteransprüchen.

Demgemäß betrifft die Erfindung ein Klebeband aus einem Träger und einer darauf zumindest einseitig beschichteten Klebemasse aus einem Olefinpolymer mit einer Dichte zwischen 0,86 und 0,89 g/cm³ und einem Kristallitschmelzpunkt von mindestens 105 °C und aus einem Klebharz, wobei die Menge an Klebharz 130 bis 350 phr beträgt.

Dem Fachmann galten Olefinpolymere für Klebmassen unter anderem wegen der Härte oder des niedrigen Schmelzpunktes der Rohstoffe als ungeeignet. Dennoch können überraschenderweise aus Olefinpolymeren mit einer Dichte zwischen 0,86 und 0,89 g/cm³, vorzugsweise zwischen 0,86 und 0,88 g/cm³, besonders bevorzugt zwischen 0,86 und 0,87 g/cm³, und einem Kristallitschmelzpunkt von mindestens 105 °C, vorzugsweise mindestens 115 °C, besonders bevorzugt mindestens 135 °C, Klebmassen für Klebebänder mit hervorragenden Klebeigenschaften hergestellt werden, beispielsweise hohe Klebkraft, hoher Tack und hohe Scherfestigkeit.

Das erfindungsgemäße Olefinpolymer weist vorzugsweise einen Schmelzindex von weniger als 8 g/10 min, besonders bevorzugt weniger als 1,5 g/10 min auf. Der Biegemodul des Olefinpolymers beträgt vorzugsweise weniger als 50 MPa, besonders bevorzugt weniger als 26 MPa und ganz besonders bevorzugt weniger als 17 MPa.

Das Olefinpolymer ist zum Beispiel ein Polypropylenharz und kann auf verschiedene Weise aufgebaut sein, zum Beispiel als Blockcopolymer, als Pfropfpolymer oder als sogenannter Reaktorblend wie bei heterophasischen Polypropylenen (auch Impact Polypropylen oder (nicht ganz richtig aber üblich) Polypropylenblockcopolymer genannt). Das bevorzugte Polypropylenharz ist bevorzugt kein klassisches nicht-heterophasisches Polypropylenrandomcopolymer, welches die Monomere Propylen und das weitere Olefin (zum Beispiel Ethylen oder Buten) statistisch verteilt enthält, da diese Polymere nur geringe Scherfestigkeiten, Klebkräfte und Wärmebeständigkeiten erreichen können. Ein heterophasisches Polypropylen darf jedoch in der kristallinen Komponente geringe Mengen eines Comonomers enthalten, soweit der Kristallitschmelzpunkt noch im erfindungsgemäßen Bereich liegt.

Das Olefinpolymer enthält vorzugsweise Ethylen oder Propylen und mindestens ein weiteres Comonomer ausgewählt aus den C₂- bis C₁₀-Olefinen, vorzugsweise C₂- bis C₁₀-α-Olefinen. Besonders geeignet sind Copolymere aus Ethylen und Propylen, Ethylen und Buten-(1), Ethylen und Octen-(1), Propylen und Buten-(1) oder ein Terpolymer aus Ethylen, Propylen und Buten-(1).

Die Dichte des Polypropylens beziehungsweise Polyethylens wird nach ISO 1183 ermittelt und in g/cm³ ausgedrückt. Der Schmelzindex wird nach ISO 1133 und 2,16 kg geprüft und in g/10 min ausgedrückt. Die Prüftemperatur beträgt, wie es dem Fachmann geläufig ist, bei propylenbasierten Polyolefinen 230 °C und bei ethylenbasierten Polymeren 190 °C.

Der Biegemodul (flexural modulus) ist nach ASTM D 790 (Sekantenmodul bei 2 % Dehnung) zu bestimmen.
Der Kristallitschmelzpunkt (T_{cr}) und die Schmelzwärme werden mit DSC (Mettler DSC 822) bei einer Aufheizrate von 10 °C/min nach ISO 3146 ermittelt, beim Auftreten mehrerer Schmelzpeaks wird der mit der höchsten Temperatur gewählt, weil nur Schmelzpeaks oberhalb von 100 °C in Klebstoffformulierungen erhalten bleiben und wirksam werden, wohingegen Schmelzpeaks erheblich unter 100 °C nicht erhalten bleiben und keine Auswirkung auf die Produkteigenschaften haben. Die Schmelzwärme bestimmt einerseits die Klebkraft und den Tack der Formulierung und andererseits die Scherfestigkeit insbesondere in der Wärme (also 70 °C und darüber).
Die Schmelzwärme des Olyolefinharzes ist daher für den optimalen Kompromiss der klebtechnischen Eigenschaften von Bedeutung, sie liegt vorzugsweise zwischen 3 und 18 J/g, besonders bevorzugt zwischen 5 und 15 J/g.
Ebenso spielt die Schmelzwärme des Klebstoffs für den optimalen Kompromiss der klebtechnischen Eigenschaften eine Rolle, sie liegt vorzugsweise zwischen 1 und 6 J/g, besonders bevorzugt zwischen 2 und 5 J/g.

Das erfindungsgemäße Olefinpolymer kann mit Elastomeren wie Naturkautschuk oder Synthesekautschuken kombiniert werden. Vorzugsweise werden ungesättigte Elastomere wie Naturkautschuk, SBR, NBR oder ungesättigte Styrolblockcopolymere nur in geringen Mengen oder besonders bevorzugt gar nicht verwendet. In der Hauptkette gesättigte Synthesekautschuke wie Polyisobutylen, Butylkautschuk, EPM, HNBR, EPDM oder hydrierte Styrolblockcopolymere werden für den Fall einer gewünschten Modifikation bevorzugt.

Es stellte sich heraus, dass das Olefinpolymer der Klebmasse erhebliche Mengen (über 100 phr) an Klebharz aufnehmen und somit ein sehr gutes Klebverhalten erreichen kann. Die Polydispersität ist das Verhältnis von Gewichtsmittel zu Zahlenmittel der Molmassenverteilung und kann durch Gelpermeationschromatographie ermittelt werden, sie spielt für die Eigenschaften eine wichtige Rolle. Als Klebharz werden daher solche mit einer Polydispersität von weniger als 2,1, vorzugsweise weniger als 1,8, besonders bevorzugt weniger als 1,6 eingesetzt. Der höchste Tack ist mit Harzen mit einer Polydispersität von 1,0 bis 1,4 zu erreichen.

Als Klebharz hat sich herausgestellt, dass Harze auf Basis von Kolophonium (zum Beispiel Balsamharz) oder Kolophoniumderivaten (zum Beispiel disproportioniertes, dimerisiertes oder verestertes Kolophonium), nicht, partiell oder vollständig hydriert, gut geeignet sind. Sie weisen von allen Klebharzen den höchsten Tack (Klebrigkeit, Anfassvermögen) auf. Vermutlich liegt das an der geringen Polydispersität von 1,0 bis 1,2. Terpenphenolharze zeichnen sich wie die hydrierten Harze durch eine besonders hohe Alterungsbeständigkeit aus.

Bevorzugt werden ebenfalls Kohlenwasserstoffharze, die vermutlich aufgrund ihrer Polarität gut verträglich sind. Dies sind zum Beispiel aromatische Harze wie Cumaron-Inden-Harze oder Harze auf Basis Styrol oder α-Methylstyrol oder cycloaliphatische Kohlenwasserstoffharze aus der Polymerisation von C₅-Monomeren wie Piperylen aus oder C₅- oder C₉-Fraktionen von Crackern oder Terpenen wie ß-Pinen oder δ-Limonen oder Kombinationen hiervon, vorzugsweise partiell oder vollständig hydriert, und Kohlenwasserstoffharze gewonnen durch Hydrierung von aromatenhaltigen Kohlenwasserstoffharzen oder Cyclopentadien-Polymeren.
Weiterhin können Harze auf Basis von Polyterpenen, vorzugsweise partiell oder vollständig hydriert, und/oder Terpenphenolharze zur Verwendung kommen.

Die Menge an Klebharz beträgt 130 bis 350 phr, bevorzugt 200 bis 240 phr (phr bedeutet Gewichtsteile bezogen auf 100 Gewichtsteile Resin beziehungsweise Rubber, das heißt hier Olefinpolymer).

Die Klebmasse enthält zur Einstellung der gewünschten Eigenschaften vorzugsweise einen flüssigen Weichmacher wie beispielsweise aliphatische (paraffinische oder verzweigte) und cycloaliphatische (naphthenische) Mineralöle, Ester der Phthal-, Trimellit-, Zitronen- oder Adipinsäure, Wachse wie Wollwachs, flüssige Kautschuke (zum Beispiel niedermolekulare Nitril-, Butadien- oder Polyisoprenkautschuke), flüssige Polymerisate aus Isobutenhomopolymer und/oder Isobuten-Buten-Copolymer, Flüssig-und Weichharze mit einem Schmelzpunkt unter 40 °C auf Basis der Rohstoffe von Klebharzen, insbesondere der oben aufgeführten Klassen an Klebharz.
Besonders bevorzugt werden davon flüssige Polymerisate aus Isobuten und/oder Buten und Ester der Phthal-, Trimellit-, Zitronen- oder Adipinsäure, insbesondere deren Ester von verzweigten Octanolen und Nonanolen.

Anstelle eines flüssigen Weichmachers kann auch ein sehr weiches und kaum kristallines Olefinpolymer verwendet werden. Dieses ist vorzugsweise ein Copolymer aus Ethylen, Propylen, Buten-(1), Hexen-(1) und/oder Octen-(1), die zum Beispiel unter den Handelsnamen Exact®, Engage®, Versify® oder Tafmer® bekannt sind, oder ein Terpolymer aus Ethylen, Propylen, Buten-(1), Hexen-(1) und/oder Octen-(1), wobei der Biegemodul vorzugsweise unter 10 MPa und der Kristallitschmelzpunkt vorzugsweise unter 50 °C liegt.
Weitere bevorzugte Olefinpolymere sind gegebenenfalls ölfreie EPM oder EPDM, also Co- oder Terpolymere aus Ethylen und Propylen und optional einem Dien wie Ethylidennorbornen, vorzugsweise mit einem Ethylengehalt von 40 bis 70 Gew.-%, einer Mooney-Viskosität (Bedingungen 1+4, 125 °C) unter 50 und/oder einer Dichte unter 0,88 g/cm³, besonders bevorzugt unter 0,87 g/cm³. Da solche Ethylenpolymere zwar sehr weich - verglichen mit einem flüssigen Weichmacher - sind, sollte die Menge im Verhältnis zum erfindungsgemäßen Olefinpolymer sehr hoch sein, also deutlich über 100 phr.

Dem Schmelzpunkt des Klebharzes (Bestimmung nach DIN ISO 4625) kommt ebenfalls eine Bedeutung zu. Üblicherweise steigt die Klebkraft einer Kautschukmasse (auf Basis Natur- oder Synthesekautschuk) mit dem Schmelzpunkt des Klebharzes an. Bei dem erfindungsgemäßen Olefinpolymer scheint sich das umgekehrt zu verhalten. Klebharze mit hohem Schmelzpunkt von 115 °C bis 140 °C sind deutlich ungünstiger als solche mit Schmelzpunkt unter 105 °C, welche bevorzugt werden. Harze mit einem Schmelzpunkt von unter 85 °C sind wenig im Handel erhältlich, da die Flakes oder Pastillen bei Transport und Lagerung zusammenbacken.
Daher wird erfindungsgemäß vorzugsweise ein gängiges Klebharz (zum Beispiel mit einem Schmelzpunkt aus dem Bereich 85 °C bis 105 °C) mit einem Weichmacher kombiniert, um faktisch den Harzschmelzpunkt zu senken. Der Mischschmelzpunkt wird an einer homogenisierten Mischung aus Klebharz und Weichmacher ermittelt, wobei die beiden Komponenten im gleichen Verhältnis vorliegen wie in der Klebmasse. Er liegt vorzugsweise im Bereich von 45 °C bis 95 °C.

Konventionelle Klebmassen auf Basis Naturkautschuk oder ungesättigten Styrolblockcopolymeren als Elastomerkomponente enthalten üblicherweise ein phenolisches Antioxidans zur Vermeidung des oxidativen Abbaus dieser Elastomerkomponente mit Doppelbindungen in der Polymerkette.

Die erfindungsgemäße Klebemasse enthält jedoch ein Olefinpolymer ohne oxidationsempfindliche Doppelbindungen und kann daher ohne Antioxidans auskommen.

Zur Optimierung der Eigenschaften kann die zum Einsatz kommende Selbstklebemasse mit weiteren Additiven wie auch primären oder sekundären Antioxidantien, Füllstoffen, Flammschutzmitteln, Pigmenten, UV-Absorbern, Antiozonantien, Antioxidantien, Metalldesaktivatoren, Lichtschutzmitteln wie HALS, Flamm-, Photoinitiatoren, Vernetzungsmitteln oder Vernetzungspromotoren abgemischt sein. Geeignete Füllstoffe und Pigmente sind beispielsweise Mikroballons Zinkoxid, Titandioxid, Ruß, Titandioxid, Calciumcarbonat, Zinkcarbonat, Zinkoxid, Silicate oder Kieselsäure.

Bei Mikroballons handelt es sich um elastische Hohlkugeln, die eine thermoplastische Polymerhülle aufweisen. Diese Kugeln sind mit niedrigsiedenden Flüssigkeiten oder verflüssigtem Gas gefüllt. Als Hüllenmaterial finden insbesondere Polyacrylnitril, PVDC, PVC oder Polyacrylate Verwendung. Als niedrigsiedende Flüssigkeit sind insbesondere Kohlenwasserstoffe der niederen Alkane, beispielsweise Isobutan oder Isopentan geeignet, die als verflüssigtes Gas unter Druck in der Polymerhülle eingeschlossen sind. Durch ein Einwirken auf die Mikroballons, insbesondere durch eine Wärmeeinwirkung erweicht einerseits die äußere Polymerhülle. Gleichzeitig geht das in der Hülle befindliche flüssige Treibgas in seinen gasförmigen Zustand über. Dabei dehnen sich die Mikroballons irreversibel aus und expandieren dreidimensional. Die Expansion ist beendet, wenn sich der Innen- und der Außendruck ausgleichen. Da die polymere Hülle erhalten bleibt, erzielt man so einen geschlossenzelligen Schaum.

Es ist eine Vielzahl an Mikroballontypen kommerziell erhältlich wie zum Beispiel von der Firma Akzo Nobel die Expancel DU-Typen (dry unexpanded), welche sich im Wesentlichen über ihre Größe (6 bis 45 µm Durchmesser im unexpandierten Zustand) und ihre zur Expansion benötigten Starttemperatur (75 °C bis 220 °C) differenzieren. Wenn der Mikroballontyp beziehungsweise die Schäumungstemperatur auf das zur Massecompoundierung benötigte Temperaturprofil und die Maschinenparameter abgestimmt ist, können Massecompoundierung und Schäumung auch gleichzeitig in einem Schritt erfolgen.
Weiterhin sind unexpandierte Mikroballontypen auch als wässrige Dispersion mit einem Feststoff- beziehungsweise Mikroballonanteil von ca. 40 bis 45 Gew.-% erhältlich, weiterhin auch als polymergebundende Mikroballons (Masterbatche), zum Beispiel in Ethylvinylacetat mit einer Mikroballonkonzentration von ca. 65 Gew.-%.

Die Klebemasse enthält gemäß einer bevorzugten Ausführungsform
- ein primäres Antioxidans vorzugsweise in einer Menge von mindestens 2, besonders bevorzugt mindestens 6 phr und/oder mit einer sterisch gehinderten phenolischen Gruppe und/oder
- ein sekundäres Antioxidans in einer Menge von 0 bis 5, vorzugsweise in einer Menge von 0,5 bis 1 phr und/oder aus der Klasse der Schwefelverbindungen oder der Klasse der Phosphite.

Die erfindungsgemäße Klebmasse kann absorbierende Füllstoffe wie zum Beispiel Zellulosederivate wie Carboxymethylcellulose, Pectin, Gelatine, Polyvinylalkohol, Polyvinylacetat, Polyethylenoxid, Polyvinylpyrrolidon, Collagen, Alginat als Hydrocolloide oder Hydrogele enthalten, insbesondere im Hinblick auf die später beschriebene Verwendung zur Hautverklebung.

Die erfindungsgemäße Klebmasse kann des Weiteren antimikrobielle Additive wie zum Beispiel Additive auf Basis von Silbersalzen, Iod, Chloramin, Chlorohexidine oder Zinksalze enthalten, um eine keimabtötende Wirkung zu erzielen und um Infektionen vorzubeugen, wieder insbesondere im Hinblick auf die später beschriebene Verwendung zur Hautverklebung.

Besonders vorteilhaft ist eine Ausführungsform des Klebebands mit einem Träger und einer mindestens einseitig aus der Schmelze auf den Träger beschichteten, im Wesentlichen Mineralöl freien Klebemasse aus einem Ethylenpolymer mit einer Dichte zwischen 0,86 und 0,89 g/cm³ und einem Kristallitschmelzpunkt von mindestens 105 °C und einem Klebharz. Auf Mineralöl wird dabei als Weichmacher verzichtet.

Mineralöle eignen sich zwar sehr gut, um das erfindungsgemäße Ethylenpolymer klebrig einzustellen, sind jedoch zu flüchtig, um gute Fogging-Werte (DIN 75201), also zum Beispiel > 60, zu erreichen oder um Ghosting (Rückstände bei Masken- und Oberflächenschutzbändern) oder Durchfetten von Papierträgern bei Wärmelagerung der Rollen zu vermeiden. Daher ist die Klebemasse im Wesentlichen frei von Mineralölen. Die Haftklebemasse ist alterungs- und UV-stabil. Bei dieser ist die Haftung für polare und unpolare Untergründe adjustierbar und sie ist auch lösungsmittelfrei verarbeitbar.
Dies Klebeband weist gegenüber analogen Klebebändern auf Basis von Naturkautschuk oder ungesättigten Styrolblockcopolymeren nicht nur Vorteile in der Kabelverträglichkeit auf, sondern auch in der Verträglichkeit gegenüber Rillrohren aus Polypropylen und Polyamid, wie sie in Kabelbäumen im Automobilbau üblich sind.

Das Ethylenpolymer weist vorzugsweise einen Schmelzindex von weniger als 6 g/10 min, besonders bevorzugt weniger als 1,5 g/10 min auf. Der Biegemodul des Ethylenpolymers beträgt vorzugsweise weniger als 26 MPa, besonders bevorzugt weniger als 17 MPa. Das Ethylenpolymer enthält vorzugsweise ein C₃- bis C₁₀-Olefin, insbesondere 1-Octen als Comonomer. Das Ethylenpolymer weist bevorzugt eine Struktur aus kristallinen Polyethylenblöcken und im Wesentlichen amorphen Blöcken aus Ethylen und einem C₃-bis C₁₀-Olefin auf.

Klassische Klebebänder mit textilem Träger oder Papierträger neigen bei Lagerung einerseits zu Deformation (Bildung von Nasen und Hohlstellen) und andererseits steigt durch kalten Fluss der Klebemasse die Abrollkräfte immer weiter an, bis das Abrollen für den Anwender zu schwer wird oder die Klebemasse oder der Papierträger beim Abrollversuch gar aufspaltet. Daher ist ein weiterer überraschender Vorteil die Lagerstabilität der erfindungsgemäßen Klebebandrollen. Selbst nach einem Monat Lagerung bei 70 °C ist der Erfindungsgegenstand noch gut abwickelbar und der Papierträger fettet nicht durch Ölmigration durch. Maskenbänder zum Lackieren oder Oberflächenschutzbänder können nach mehrwöchiger Außenbewitterung noch rückstandfrei entfernt werden.

Als Klebharze haben sich Harze auf Basis von Kolophonium (zum Beispiel Balsamharz) oder Kolophoniumderivaten (zum Beispiel disproportioniertes, dimerisiertes oder verestertes Kolophonium), vorzugsweise partiell oder vollständig hydriert, als gut geeignet erwiesen.

Die Klebemasse enthält vorzugsweise einen flüssigen Mineralöl freien Weichmacher, wie sie ausführlich beschrieben sind.

Konventionelle Klebmassen auf Basis Naturkautschuk oder ungesättigten Styrolblockcopolymeren als Elastomerkomponente enthalten üblicherweise ein phenolisches Antioxidans zur Vermeidung des oxidativen Abbaus dieser Elastomerkomponente mit Doppelbindungen in der Polymerkette. Die erfindungsgemäße Klebemasse enthält jedoch ein Ethylenpolymer ohne oxidationsempfindliche Doppelbindungen und sollte daher ohne Antioxidans auskommen. Überraschenderweise wurde festgestellt, dass Antioxidantien die Kompatibilität der Klebemasse mit den Drahtisolierungen verbessern. Daher werden vorzugsweise ein primäres Antioxidans und besonders bevorzugt auch ein sekundäres Antioxidans verwendet.

Der Masseauftrag (Beschichtungsstärke) liegt in dieser Ausführungsform vorzugsweise zwischen 10 und 120 g/m², besonders bevorzugt zwischen 20 und 70 g/m².

Besonders vorteilhaft ist die erfindungsgemäße Ausführungsform des Klebebands mit einem Träger und einer mindestens einseitig aus der Schmelze auf den Träger beschichteten Klebemasse aus einem Ethylenpolymer mit einer Dichte zwischen 0,86 und 0,89 g/cm³ und einem Kristallitschmelzpunkt von mindestens 105 °C und einem Klebharz zur Verklebung auf niederenergetischen Oberflächen geeignet, und zwar zum Verkleben auf Haftgründen aus unpolaren Lacken oder Olefinpolymeren, besonders bevorzugt zum Schließen oder Gurten von Polyolefinbeuteln, oder zum Fixieren von Teilen aus olefinischen Kunststoffen oder Elastomeren, insbesondere zum Fixieren von Teilen in Kraftfahrzeugen.

Klebebänder zur Verklebung niederenergetischer Oberflächen werden üblicherweise mit Klebemassen auf Basis Naturkautschuk, Styrolblockcopolymer und Acrylat gefertigt. Beide Sorten von Kautschukmassen weisen auf niederenergetischen Oberflächen gute Haftung auf. Klebemassen auf Basis hydrierter Styrolblockcopolymere sind sehr teuer und kleben auf anderen Untergründen schlecht. Sie erweichen ebenfalls schon deutlich unter 100 °C.
Acrylat-Klebemassen haben eine gute Alterungs- und UV-Stabilität haften aber auf unpolaren wie zum Beispiel olefinischen Polymeren trotz aller bisherigen Bemühungen nur schlecht, weshalb die zu verklebenden Oberflächen mit lösungsmittelhaltigen Primern vorbehandelt werden müssen.

Silikonhaftkleber haben eine gute Alterungs- und UV-Stabilität und gute Haftung auf niederenergetischen Oberflächen, sind aber extrem teuer und lassen sich nicht mit den üblichen silikonisierten Linern abdecken (beziehungsweise sich davon nicht wieder abziehen). Die erfindungsgemäße Klebemasse weist eine Lösungsmittelfreiheit, eine hohe Haftung auf niederenergetischen Oberflächen und eine Alterungs- und UV-Stabilität wie Acrylat-Klebemassen auf.

Die Klebemasse zeigt auf sehr vielen Untergründen eine hervorragende Haftung und insbesondere auch auf niederenergetischen Oberflächen wie unpolaren Lacken oder Olefinpolymeren.

Die Zusammensetzung der Klebemasse orientiert sich an derjenigen, wie sie für die Mineralöl freie Klebemasse aus einem Ethylenpolymer beschrieben worden ist.

Als Beschichtungsverfahren für den Auftrag der Klebemasse werden Extrusionsbeschichtung mit Breitschlitzdüsen und Kalanderbeschichtung bevorzugt.

Das erfindungsgemäße Klebeband, insbesondere bei Verwendung zur Verklebung auf niederenergetischen Oberflächen, ist vorzugsweise beidseitig klebend.

Bei mehrschichtigem Aufbau können mehrere Schichten durch Coextrusion, Kaschierung oder Beschichtung übereinander gebracht werden. Die Beschichtung kann direkt oder auf einen Liner oder auf einen Prozessliner erfolgen.

Die Haftklebemasse kann
- einseitig auf einem Träger vorliegen und wobei auf der anderen Seite eine nicht erfindungsgemäße Haftklebemasse vorzugsweise auf Basis Polyacrylat oder eine nicht erfindungsgemäße Siegelschicht vorhanden ist oder
- beidseitig auf einem Träger vorliegen, wobei die beiden Haftklebemassen gleiche oder verschiedene Zusammensetzung aufweisen können.

Das Klebeband ist vorzugsweise ein- oder beidseitig mit einem Liner abgedeckt. Der Liner für das Produkt oder der Prozessliner ist zum Beispiel ein Trennpapier oder eine Trennfolie, vorzugsweise mit Silikonbeschichtung. Als Träger kommen zum Beispiel Folien aus Polyester oder Polypropylen oder kalandrierte Papiere mit oder ohne Dispersions- oder Polyolefinbeschichtung in Frage.

Der Masseauftrag (Beschichtungsstärke) einer Schicht liegt vorzugsweise Schicht zwischen 30 und 200 g/m², vorzugsweise zwischen 50 und 75 g/m². Die Gesamtdicke des Klebebandes ohne Liner beträgt vorzugsweise 600 bis 1500 µm, besonders bevorzugt 700 bis 5000 µm.

Vorzugsweise ist mindestens eine Schicht besonders vorzugsweise die erfindungsgemäße vernetzt. Dies kann vermittels energiereicher Strahlen vorzugsweise Elektronenstrahlen, oder durch Peroxid- oder Silanvernetzung erfolgen.

Das erfindungsgemäße Klebeband wird dadurch gebildet, dass auf den Träger partiell oder vollflächig vorzugsweise ein- oder gegebenenfalls beidseitig die Klebemasse aufgetragen wird. Die Beschichtung kann auch in Form eines oder mehrerer Streifen in Längsrichtung (Maschinenrichtung) erfolgen, gegebenenfalls in Querrichtung, sie ist insbesondere aber vollflächig. Weiterhin können die Klebemassen rasterpunktförmig mittels Siebdruck, wobei die Klebstoffpünktchen auch unterschiedlich groß und/oder unterschiedlich verteilt sein können, durch Tiefdruck in Längs- und Querrichtung zusammenhängenden Stegen, durch Rasterdruck oder durch Flexodruck aufgebracht werden. Die Klebemasse kann in Kalottenform (hergestellt durch Siebdruck) vorliegen oder auch in einem anderen Muster wie Gitter, Streifen, Zickzacklinien. Ferner kann sie beispielsweise auch aufgesprüht sein, was ein mehr oder weniger unregelmäßiges Auftragsbild ergibt.

Die Herstellung und Verarbeitung der Haftklebemassen kann aus Lösung sowie aus der Schmelze erfolgen. Bevorzugte Herstell- und Verarbeitungsverfahren erfolgen aus der Schmelze. Für den letzteren Fall umfassen geeignete Herstellprozesse sowohl Batchverfahren als auch kontinuierliche Verfahren. Besonders bevorzugt ist die kontinuierliche Fertigung der Haftklebemasse mit Hilfe eines Extruders und anschließender Beschichtung direkt auf das zu beschichtende Substrat bei entsprechend hoher Temperatur der Klebmasse. Als Beschichtungsverfahren werden Extrusionsbeschichtung mit Breitschlitzdüsen, Kalanderbeschichtung, Sprühbeschichtung und Schmelzsiebdruck bevorzugt. Weiterhin kann die Beschichtung auch auf beide Seiten des Trägermaterials erfolgen, so dass ein doppelseitiges Klebeband entsteht.

Die Klebemasse kann gleichmäßig auf dem Trägermaterial verteilt sein, sie kann aber auch funktionsgerecht für das Produkt über die Fläche unterschiedlich stark oder dicht aufgetragen sein.

Der prozentuale Anteil der mit der Klebemasse beschichteten Fläche sollte mindestens 20 % betragen und kann bis zu 95 % reichen, für spezielle Produkte bevorzugt 40 % bis 60 % sowie 70 % bis 95 %. Dieses kann gegebenenfalls durch Mehrfachapplikation erreicht werden, wobei gegebenenfalls auch Klebemassen mit unterschiedlichen Eigenschaften eingesetzt werden können.

Das Klebeband weist gemäß einer vorteilhaften Ausführungsform der Erfindung eine Klebkraft auf der Trägerrückseite von mindestens 1,5 N/cm auf, besonders eine Klebkraft zwischen 2,5 N/cm und 5 N/cm. Auf anderen Untergründen können höhere Klebkräfte erreicht werden.

Je nach Trägermaterial und dessen Temperaturempfindlichkeit kann die Selbstklebemasse direkt aufgetragen sein oder zuerst auf einen Hilfsträger aufgebracht und dann auf den endgültigen Träger transferiert werden.

Als Trägermaterialien eignen sich alle starren und elastischen Flächengebilde aus synthetischen und natürlichen Rohstoffen. Bevorzugt sind Trägermaterialien, die nach Applikation der Klebemasse so eingesetzt werden können, dass sie Eigenschaften eines funktionsgerechten Verbandes erfüllen.
Als Trägermaterial können eingesetzt werden zum Beispiel Textilien wie Gewebe, Gewirke, Gelege, Vliese, Laminate, Netze, Folien, Papiere, Tissues, Schäume und geschäumte Folien. Geeignete Folien sind aus Polypropylen vorzugsweise orientiertem Polyester, Hart- und Weich-PVC vorzugsweise mit einem Flächengewicht von unter 50 g/m² und bei Folien vorzugsweise weniger 15 µm, damit das Klebeband nicht zu wenig anschmiegsam ist. Besonders bevorzugt sind Polyolefin-, Polyurethan-, EPDM und Chloroprenschaum. Unter Polyolefin wird Polyethylen und Polypropylen verstandne, wobei Polyethylen wegen der Weichheit bevorzugt wird. Der Begriff Polyethylen schließt LDPE aber auch Ethylencopolymere wie LLDPE und EVA ein. Insbesondere sind vernetzte Polyethylenschäume oder viskoelastische Schäume geeignet. Letztere sind vorzugsweise aus Polyacrylat, besonders bevorzugt gefüllt mit hohlen Körpern aus Glas oder Polymeren wie Mikroballons.

Als Trägermaterial können Kunststoff-Folien wie zum Beispiel Folien aus Polyolefin wie Polyethylen, Polypropylen, Polybuten, deren Copolymeren, Blends dieser Polymeren zum Beispiel mit Polyethylenvinylacetat oder lonomeren sowie Folien aus Polyvinylchlorid oder Polyester eingesetzt werden. Dehnbare Folien können durch eine Armierung, vorzugsweise ein Fadengelege, verstärkt werden. Weiterhin ist der Einsatz von Papier-Kunststoff-Verbünden, die zum Beispiel durch Extrusionsbeschichtung oder Laminierung erhalten werden, möglich. Textilmaterialien können je nach Anwendung offenporig oder als Textil-Kunststoff-Verbund als Trägermaterial verwendet werden. Die verwendeten Kunststoffe können Flammschutzmittel wie zum Beispiel Antimontrioxid oder bromhaltige Flammschutzmittel wie zum Beispiel Saytex® 8010 enthalten. Das Trägermaterial kann Dicken zwischen 30 und 150 µm aufweisen, bevorzugt zwischen 50 und 100 µm.

Die Träger können vor dem Zusammenbringen mit der Klebemasse (auf der Streich-, auch Beschichtungsseite genannt) chemisch wie durch Primerung oder durch eine physikalische Vorbehandlung wie Corona vorbereitet werden. Die Rückseite derselben kann einer antiadhäsiven physikalischen Behandlung oder Beschichtung unterzogen sein.

Vernetzte Polyethylenschäume werden für doppelseitig klebende Klebebänder derart behandelt, dass eine Haftung von Acrylathaftklebemassen darauf sehr schlecht ist und selbst mit einer Behandlung nicht sehr zufrieden stellend, da diese Träger aufgrund des Herstellprozesses Gleitmittel wie Erucamid enthalten.

Es ist daher völlig überraschend, dass die erfindungsgemäßen Massen selbst ohne Behandlung hervorragend auf solchen Schäumen haften, das heißt, beim gewaltsamen Versuch, sie abzulösen, wird der Schaum zerstört.

Weiter können diese Materialien vor- beziehungsweise nachbehandelt werden. Gängige Vorbehandlungen sind Corona und Hydrophobieren, geläufige Nachbehandlungen sind Kalandern, Tempern, Kaschieren, Stanzen und Eindecken.

Vorteilhaft hat sich auch das Laminieren des Trägers mit zumindest einer zusätzlichen Schicht aus Textilien, Schäumen oder Folien herausgestellt, da sich hierdurch eine Kombination von Eigenschaften besonderer Art ergibt. Ein Schaum hat eine wesentlich höhere Dämpfungseigenschaft auf als ein nicht laminierter Träger. Folien können zum Beispiel zum Versiegeln der Oberfläche verwendet werden.

Die Herstellung und Verarbeitung der Haftklebemassen kann aus Lösung sowie aus der Schmelze erfolgen. Der Vorteil der Verarbeitung der Haftklebemasse aus der Schmelze liegt in der Möglichkeit, sehr hohe Schichtdicken (Masseaufträge) in sehr kurzer Zeit erreichen zu können, da nach der Beschichtung kein Lösungsmittel entfernt werden muss. Bevorzugte Herstell- und Verarbeitungsverfahren erfolgen daher aus der Schmelze. Für den letzteren Fall umfassen geeignete Herstellprozesse sowohl Batchverfahren als auch kontinuierliche Verfahren. Besonders bevorzugt ist die kontinuierliche Fertigung der Haftklebemasse mit Hilfe eines Extruders und anschließender Beschichtung direkt auf das zu beschichtende Substrat oder ein Trennpapier beziehungsweise eine Trennfolie bei entsprechend hoher Temperatur der Klebmasse. Als Beschichtungsverfahren werden Extrusionsbeschichtung mit Breitschlitzdüsen und Kalanderbeschichtung bevorzugt.

Der Masseauftrag (Beschichtungsstärke) liegt vorzugsweise zwischen 10 oder 15 und 300 g/m², weiter vorzugsweise zwischen 20 und 250 g/m², besonders bevorzugt zwischen 70 und 160 g/m².

Für die Verwendung als Haftklebeband können die ein- oder doppelseitigen Haftklebebänder mit einem oder zwei Trennfolien oder Trennpapieren abgedeckt sein. In einer bevorzugten Auslegung werden silikonisierte oder fluorierte Folien oder Papiere, wie zum Beispiel Glassine, HPDE oder LDPE gecoatete Papiere eingesetzt, die wiederum mit einer Releaseschicht basierend auf Silikonen oder fluorierten Polymeren versehen sind.

Der allgemeine Ausdruck "Klebeband" umfasst im Sinne dieser Erfindung alle flächigen Gebilde wie in zwei Dimensionen ausgedehnte Folien oder Folienabschnitte, Bänder mit ausgedehnter Länge und begrenzter Breite, Bandabschnitte, Stanzlinge, Etiketten und dergleichen.

Das Klebeband kann in Form einer Rolle, also in Form einer archimedischen Spirale auf sich selbst aufgerollt, hergestellt werden.

Im Folgenden wird die Erfindung durch einige Beispiele näher erläutert, ohne die Erfindung damit einschränken zu wollen.

### Rohstoffe der Beispiele:

| | |
|---|---|
| IN FUSE 9107: | Copolymer aus Ethylen und Octen-(1), Schmelzindex 1 g/10min, Dichte 0,866 g/cm³, Biegemodul 15,5 MPa, Kristallitschmelzpunkt 121 °C |
| IN FUSE 9507: | Copolymer aus Ethylen und Octen-(1), Schmelzindex 5 g/10min, Dichte 0,866 g/cm³, Biegemodul 13,9 MPa, Kristallitschmelzpunkt 119 °C |
| NOTIO PN-0040: | Copolymer aus Propylen und Buten-(1) (evtl. mit geringen Mengen auch an Ethylen), Schmelzindex 4 g/10min, Dichte 0,868 g/cm³, Biegemodul 42 MPa, Kristallitschmelzpunkt 159 °C, Schmelzwärme 5,2 J/g |
| Softell CA02: | Copolymer aus Propylen und Ethylen, Schmelzindex 0,6 g/10min Dichte 0,870 g/cm³, Biegemodul 20 MPa, Kristallitschmelzpunkt 142 °C, Schmelzwärme 9,9 J/g |
| Engage 7467: | Copolymer aus Ethylen und Buten-(1), Schmelzindex 1,2 g/10min Dichte 0,862 g/cm³, Biegemodul 4 MPa, Kristallitschmelzpunkt 34 °C |
| LD 251: | LDPE, Schmelzindex 8 g/10min, Dichte 0,9155 g/cm³, Biegemodul 180 MPa, Kristallitschmelzpunkt 104 °C |
| PB 0300 M: | Polybuten, Schmelzindex 4 g/10min, Dichte 0,915 g/cm³, Biegemodul 450 MPa, Kristallitschmelzpunkt 116 °C |
| Buna EP G 3440: | EPDM, Dichte von 0,86 g/cm³, Mooney-Viskosität 28, 48 Gew.-% Ethylen, 48 Gew.-% Propylen und 4 Gew.-% Dien |
| Ondina 933: | Weißöl (paraffinisch-naphthenisches Mineralöl) |
| Wingtack 10: | flüssiges C₅-Kohlenwasserstoffharz |
| Escorez 1310: | nicht hydriertes C₅-Kohlenwasserstoffharz, Schmelzpunkt von 94 °C, Polydispersität 1,5 |
| Escorez 1102: | nicht hydriertes C₅-Kohlenwasserstoffharz mit einem Schmelzpunkt von 100 °C und einer Polydispersität von 2,6 |
| Escorez 5400: | voll hydriertes Cyclopentadienharz mit einem Schmelzpunkt von 103 °C und einer Polydispersität von 2,3 |
| Wingtack extra: | aromatenmodifiertes C₅-Kohlenwasserstoffharz, Schmelzpunkt 97 °C, Polydispersität 1,6 |
| Regalite R1100: | hydriertes aromatisches Kohlenwasserstoffharz, Schmelzpunkt 100 °C, Polydispersität 1,9 |
| Eastotac C 130 L: | voll hydriertes C₅-Kohlenwasserstoffharz (im Gegensatz zu Eastotac H 130 R als nicht voll hydriertem Harz mit einer Polydispersität von 2,1) mit einem Schmelzpunkt von 130 °C und einer Polydispersität von 2,0 |
| Eastotac C 115 L: | voll hydriertes C₅-Kohlenwasserstoffharz mit einem Schmelzpunkt von 115 °C und einer Polydispersität von 1,9 |
| Irganox 1726: | phenolisches Antioxidans mit Schwefel basierter Funktion eines sekundären Antioxidans |
| Irganox 1076: | phenolisches Antioxidans |
| Irganox PS 802: | Schwefel-basiertes sekundäres Antioxidans |
| Oppanol B 10: | flüssiges Poly-iso-Buten |
| Foral 85: | voll hydrierter Glycerinester des Kolophoniums mit einem Schmelzpunkt von 85 °C und einer Polydispersität von 1,2 |
| PRO 10493: | nicht hydriertes C₅-Kohtenwasserstoffharz mit einem Schmelzpunkt von 98 °C und einer Polydispersität von 2,0 |
| Tinuvin 622: | UV-Stabilisator auf HALS-Basis |
| TOTM: | Tris-(2-ethylhexyl)-trimellitat |

### Prüfmethoden

Die Messungen werden, wenn nicht anders angegeben, bei einem Prüfklima von 23 ± 1 °C und 50 ± 5 % rel. Luftfeuchte durchgeführt.

Die Abrollkraft wird bei 300 mm/min nach DIN EN 1944 gemessen.

Die Alterungsprüfungen werden gemäß Automobilnorm LV 312-1 "Schutzsysteme für Leitungssätze in Kraftfahrzeugen, Klebebänder; Prüfrichtlinie" (02/2008), gemeinsame Norm der Firmen Daimler, Audi, BMW und Volkswagen, durchgeführt.

Die Klebkräfte werden bei einem Abzugswinkel von 180° nach AFERA 4001 an 15 mm breiten Teststreifen bestimmt. Hierbei werden als Prüfuntergrund Stahlplatten nach AFERA-Norm beziehungsweise die Rückseite des Klebebandes verwendet.

Die Bestimmung der Klebkraft bei der Ausführungsform mit einem Gewebeträger für die Außenanwendung wird in Anlehnung an AFERA 5001 wie folgt durchgeführt. Als definierter Haftgrund werden eine Stahlfläche, eine Polyethylenfläche (PE) und ein Sandpapier der Körnung 150 eingesetzt. Das zu untersuchende verklebbare Flächenelement wird auf eine Breite von 20 mm und eine Länge von etwa 25 cm zugeschnitten, mit einem Handhabungsabschnitt versehen und unmittelbar danach fünfmal mit einer Stahlrolle von 4 kg bei einem Vorschub von 10 m/min auf den jeweils gewählten Haftgrund aufgedrückt. Unmittelbar im Anschluss daran wird das verklebt Flächenelement in einem Winkel von 180° vom Haftgrund mit einem Zugprüfungsgerät (Firma Zwick) abgezogen und die hierfür bei Raumtemperatur benötigte Kraft gemessen. Der Messwert (in N/cm) ergibt sich als Mittelwert aus drei Einzelmessungen.

Zur Messung der UV-Stabilität (UV-Test) werden die Muster in 20 mm Breite und 25 cm Länge auf eine Glasplatte mit einer Stärke von 4 mm verklebt und fünfmal mit einer 2 kg Rolle angerollt. Die Muster werden mit der Glasseite nach oben in einer UV-Kammer mit Xenonlampe bei einer Bestrahlungsstärke von 300 W/m² gelagert. Jeden Tag wird ein jeweils neuer Streifen pro Beispiel aus der UV-Kammer genommen und nach Konditionierung auf Raumtemperatur für 1 h von der Glasplatte abgezogen.
Dabei wird die Haftung beurteilt sowie registriert, ob es merkliche Veränderungen, Reißer oder Klebmasserückstände auf der Glasplatte gibt.

Als Bewitterungstest in Form eines Schnelltests anstelle der langwierigen Freibewitterung wird der so genannte "Suntest" in Anlehnung an die ISO 4892-2 (2006) nach Methode A durchgeführt. Dazu werden Muster auf Hart-PVC, Glas und PE verklebt und einer Kombination von UV-Bestrahlung mittels einer 765 Watt Xenonlampe und temporärer Bewässerung unterworfen. In den zweistündigen Zyklen folgt nach 18 min einer Kombination aus Bewässerung und Bestrahlung ein Zeitraum von 102 min Bestrahlung ohne Bewässerung.
Nach der Bewitterungszeit werden die Streifen nach Rekonditionierung auf Raumtemperatur visuell beurteilt, anschließend unter 90° und 180° abgezogen. Nach Herstellerangaben (zum Beispiel Firma Atlas) entspricht eine Woche Suntester ca. 3 Monaten Freibewitterung in Mitteleuropa.
Soweit die abgezogenen Teststreifen es zulassen, wird hiervon die Klebkraft nach Lagerung ermittelt.
Sporadisch durchgeführte Dauerteste unter realen Außenbedingungen (Freibewitterung) erfolgten in Hamburg auf denselben Haftgründen auf einem Gebäudedach mit Südausrichtung unter einem Neigungswinkel von 45°. Die Ergebnisse waren vergleichbar zu den oben genannten Schnelltesten.

Die Dichte der Polymeren wird nach ISO 1183 ermittelt und in g/cm³ ausgedrückt.

Der Kristallitschmelzpunkt (T_{cr}) wird mit DSC nach MTM 15902 (Basell-Methode) beziehungsweise ISO 3146 ermittelt.

Die Dicke wird nach DIN 53370 bestimmt, wobei der Taster plan (nicht gewölbt) ist. Bei strukturierten Folien wird jedoch die Dicke vor dem Prägen zugrunde gelegt. Dies ist auch nachträglich über das Flächengewicht (ermittelt nach DIN 53352) und Umrechnung mit der Dichte möglich. Die Prägetiefe ist die Differenz zwischen den Dicken mit und ohne Prägung.

Die Bestimmung der Klebkräfte auf Stahl bei der Ausführungsform für Bauanwendungen werden bei einem Abzugswinkel von 180° in Anlehnung an AFERA 4001 an (nach Möglichkeit) 20 mm breiten Teststreifen bestimmt. Hierbei werden Stahl-Platten nach AFERA-Norm als Prüfuntergrund verwendet, auf die ein Streifen des zu prüfenden Klebebands aufgebracht wird. Klebebänder mit weichen Trägerfolien, das heißt Klebebänder, bei denen die Folie bei Kräften unterhalb der Klebkraft auf Stahl verdehnt wird, werden mit einem 20 mm breiten Streifen tesa® 4224 (ein 83 µm Klebeband auf Basis einer PP-Folie mit einer Kautschukklebemasse, das eine Klebkraft von 8,25 N/25 mm aufweist) verstärkt. Bei der Prüfung von doppelseitigen Klebebändern wird die nicht zu prüfende Seite mit einem 20 mm breiten und 30 µm dicken Streifen aus Hart-PVC abgedeckt. Die Prüfung erfolgt gemäß AFERA 4001.

Klebkräfte auf Polyethylen werden an 20 mm breiten Verklebungen einer 190 µm dicken Polyethylen-Folie und des Klebebands ohne vorherige Lagerung ermittelt. Die Folie ist dabei senkrecht nach unten befestigt, das Klebeband wird senkrecht nach oben mit einer Geschwindigkeit von 300 mm/min abgezogen. Für Klebebänder mit weichen Trägerfolien oder doppelseitigen Klebebändern gilt das gleiche Vorgehen wie bei der Bestimmung der Klebkraft auf Stahl.

Zur Bestimmung der Alterungsbeständigkeit werden Verklebungen des Klebebands auf handelsüblichen Winddichtungen, Dampfbremsen oder Dampfsperren geprüft. Verwendet werden Prüflinge wie in der Methode zur Bestimmung der Klebkraft auf Polyethylen beschrieben. Die Lagerung erfolgt für 20 Wochen bei 65 ± 1 °C und 85 ± 5 % rel. Luftfeuchte.

Der Fogging-Wert wird nach DIN 75201 bestimmt.

Das Anfassverhalten wird bestimmt, indem man eine Probe analog wie für die Klebkraftbestimmung beschrieben auf Kraftpapier appliziert und die Probe zügig abzieht. Das Anfassverhalten ist gut wenn auf mindestens auf 50 % der Verklebungsfläche die Papierfasern herausgerissen werden oder das Papier spaltet.

Die Erfindung wird im Anschluss durch mehrere Beispiele näher beschreiben, ohne dass diese in irgendeiner Form einschränkend wirken sollen. Bei den verschiedenen, als vorteilhaft erkannten Verwendungsmöglichkeiten finden sich weitere, speziell auf die jeweilige Verwendungsart zugeschnittene Beispiele, die ebenfalls nur zur Erläuterung dienen sollen.

### Beispiel 1

Die Klebemasse besteht aus folgenden Komponenten: 100 phr IN FUSE 9107, 100 phr Engage 7467, 425 phr Escorez 1310, 16 phr Irganox 1726.

Die Klebemasse wird in einem Extruder kontinuierlich hergestellt und mit 70 g/m² auf einem Polyestergewebe mittels Düsenbeschichtung aus der Schmelze aufgetragen. Das Filamentgewebe hat ein Flächengewicht von 130 g/m² aus Polyestergarn von 167 dtex mit 45 Fäden pro cm in Kettrichtung und 25 Fäden pro cm in Schussrichtung. Der beschichtete Ballen wird durch Schneiden in Rollen in 19 mm Breite und 10 m Lauflänge konfektioniert, der Kerninnendurchmesser beträgt 38 mm.

Klebkraft auf Stahl 5 N/cm, Klebkraft auf Rückseite 2,5 N/cm.
Rollenlagerung 1 Monat bei 70 °C: Die Rolle ist leicht deformiert und gut abrollbar.
Verträglichkeitsprüfung: Das fertige Klebeband wird gemäß LV 312 um ein Drahtpaar mit unterschiedlichen Isolierungsmaterialien gewickelt und bei entsprechender Temperatur gelagert. Sechs solcher Prüflinge werden pro Isolierungsmaterial hergestellt. Alle 500 Stunden wird je eines der Muster kontrolliert, das Klebeband wieder abgewickelt, und das Kabel um einen Dorn von 2 mm Durchmesser gewickelt. Es wird untersucht, ob die Isolierung beschädigt ist und ob die Klebemasse eine Haftklebrigkeit ausweist. Prüftemperaturen: PVC 105 °C und auf vernetztem PE bei 125 °C. Nach 3000 Stunden sind alle Drahtisolierungen noch unbeschädigt. Nach 3000 Stunden bei 105 °C ist die Masse kaum in den Träger eingedrungen und weist noch eine gute Haftklebrigkeit auf. Nach 3000 Stunden bei 125 °C ist die Masse teilweise in den Träger eingedrungen ist aber noch haftklebrig.
Fogging-Wert nach DIN 75201: 85.

### Beispiel 2

Die Klebemasse besteht aus folgenden Komponenten:
100 phr IN FUSE 9107, 100 phr Buna EP G 3440, 425 phr Regalite 1100, 8 phr Irganox 1076 und 8 phr Irganox PS 802. Die Beschichtung erfolgt wie in Beispiel 1 mit 40 g/m² auf einem fertig ausgerüsteten Papierträger SC/042 P (Gessner, 60 g/m²).
Das Klebeband wird auf einem Blech mit 2K-PU-Lack, wie es für die KFZ-Industrie gebräuchlich ist, verklebt und einer Außenbewitterung in Hamburg unterzogen, nach 4 Wochen lässt sich das Klebeband rückstandfrei wieder abziehen. Nach 4 Wochen Lagerung der Rollen bei 70 °C ist das Papier nicht durchfettet und die Rolle nur geringfügig deformiert.

### Beispiel 3

Die Klebemasse besteht aus folgenden Komponenten:
100 phr IN FUSE 9107, 100 phr Buna EP G 3440, 425 phr Escorez 1310, 8 phr Irganox 1076 und 8 phr Irganox PS 802. Die Beschichtung erfolgt wie in Beispiel 1 mit 68 g/m² Die Klebemasse wird auf folgendem Träger aufgebracht: Maliwatt-Nähgewirke aus Polyesterfasern mit ca. 3,4 dtex und einer Faserlänge von ca. 80 mm, einem Flächengewicht von 72 g/m² und einer Feinheit F 22 mit einer Stichlänge von 1 mm eines Polyestergarns von 50 dtex.

Klebkraft auf Stahl 6,2 N/cm, Klebkraft auf Rückseite 2,4 N/cm.
Rollenlagerung 1 Monat bei 70 °C: Die Rolle ist leicht deformiert und gut abrollbar. Verträglichkeitsprüfung auf PVC bei 105 °C und auf vernetztem PE und PP bei 125 °C: Nach 3000 Stunden sind alle Drahtisolierungen noch unbeschädigt. Nach 3000 Stunden bei 105 °C ist die Masse kaum in den Träger eingedrungen und weist noch eine gute Haftklebrigkeit auf. Nach 3000 Stunden bei 125 °C ist die Masse teilweise in den Träger eingedrungen ist aber noch haftklebrig.

### Beispiel 4

Die Ausführung erfolgt wie in Beispiel 1 beschrieben, jedoch besteht die Masse aus 100 phr IN FUSE 9507, 140 phr Oppanol B 10, 250 phr Foral 85, 8 phr Irganox 1076 und 5 phr Tinuvin 622. Die Beschichtung erfolgt mit 15 g/m² auf der Basisschicht einer Trägerfolie. Diese besteht aus einer 50 µm dicken Basisschicht aus 59,7 Gew.-Teilen aus PP-Homopolymer, 30 Gew.-Teilen LLDPE, 10 Gew.-Teilen anorganisch gecoatetem Titandioxid und 0.3 Gew.-Teilen eines HALS-Stabilisators (Tinuvin 622) und einer 15 µm dicken Deckschicht aus 30 Gew.-Teilen aus PP-Homopolymer und 70 Gew.-Teilen LDPE (LD 251).
Das resultierende Produkt wird auf einem Bleck mit 2K-PU-Lack wie für KFZ gebräuchlich verklebt und einer UV-Alterung unterzogen (1750 h Xenotest 150 entsprechend 97 kLy), nach dem nachfolgendem Abziehen traten keine Klebemasserückstände auf.

### Beispiel 5

Die Klebemasse bestehend aus folgenden Komponenten: 100 phr IN FUSE 9107, 100 phr Engage 7467, 425 phr Escorez 1310, 16 phr Irganox 1726.
Die Klebemasse wird in einem Extruder kontinuierlich hergestellt mittels Düsenbeschichtung aus der Schmelze beidseitig mit 70 g/m² auf ein Tissue von 25 g/m² aufgetragen. Das Produkt ist mit einem polyethylenbeschichteten Trennpapier abgedeckt. Klebkraft auf Stahl der offenen und auf der abgedeckten Seite beträgt jeweils 5 N/cm. Die Klebkraft auf einer Polypropylenplatte ist jeweils > 10 N/cm.
Die Klebkräfte werden bei einem Abzugswinkel von 180° nach AFERA 4001 an 15 mm breiten Teststreifen bestimmt. Die nicht auf Stahl bzw. Polypropylen verklebte Seite wird vor der Klebkraftmessung mit einer 25 µm starken geätzten Polyesterfolie kaschiert.

### Beispiel 6

Die Herstellung erfolgt analog zu Beispiel 5, die Klebemasse bestehend aus folgenden Komponenten: 100 phr IN FUSE 9107, 212 phr Foral 85, 78 phr Ondina 933, 2 phr Irganox 1726. Die Beschichtung erfolgt mit 65 g/m² auf einem vernetztem Polyethylenschaum Alveolith THL SR0701.

Klebkraft auf Stahl der offenen und auf der abgedeckten Seite beträgt jeweils 9 N/cm. Die Klebkraft auf einer Polypropylenplatte ist jeweils > 10 N/cm. Klebt man zwei Lagen des Produktes ohne Verstärkung mit der Polyesterfolie gegeneinander und versucht nach einer Minute die Verbindung zu lösen spaltet der Schaum.

### Beispiel 7

Die Herstellung erfolgt analog zu Beispiel 5, die Klebemasse bestehend aus folgenden Komponenten: 100 phr IN FUSE 9507, 250 phr Regalite 1100, 140 phr Oppanol B 10, 2 phr Irganox 1726.
Die Beschichtung erfolgt mit 50 g/m² auf einen 800 µm dicken viskoelastischen Träger aus Polyacrylat. Die Zusammensetzung sowie die Herstellung desselben sind in der WO 2006/027389 A1 als Beispiel Träger VT1 beschrieben. Die andere Seite wird ebenfalls mit 50 g/m² einer Acrylatlösungsmittelmasse (entsprechend Beispiel PA 1 der WO 2006/027389 A1) kaschiert.

Klebkraft auf Stahl der Ethylenpolymermasse 11 N/cm und der Acrylatmasse 15 N/cm. Klebkraft auf einer Polypropylenplatte der Ethylenpolymermasse > 10 N/cm und der Acrylatmasse 2 N/cm.

### Vergleichsbeispiel 1

Die Ausführung erfolgt wie in Beispiel 1 beschrieben, jedoch besteht die Masse entsprechend marktüblichen Rezepturen aus 100 phr Vector 4113, 97 phr Escorez 1310, 21 phr Ondina 933 und 1 phr Irganox 1726.

Rollenlagerung 1 Monat bei 70 °C: Die Rolle ist stark deformiert und sehr schwer abrollbar.
Verträglichkeitsprüfung: Die PVC- Isolierungen zeigen die ersten Risse nach 500 Stunden und die PE und PP-Isolierungen nach 1000 Stunden Lagerung bei 105 °C. Die Haftklebrigkeit ist nach 1000 Stunden verloren gegangen, die Masse ist vom Träger aufgesaugt worden und dort verlackt.
Fogging-Wert: 35.

### Vergleichsbeispiel 2

Die Ausführung erfolgt wie in Beispiel 1 beschrieben, jedoch mit einer Masse aus 100 phr LD 251, 78,4 phr Ondina 933, 212 phr Eastotac H130R (nicht hydriertes C₅-Kohlenwasserstoffharz, Polydispersität von 2,1, Schmelzpunkt 130 °C), 8 phr Irganox 1726. Die Beschichtung ist nicht haftklebrig, sondern hart mit öliger Oberfläche.

### Vergleichsbeispiel 3

Die Ausführung erfolgt wie in Beispiel 1 beschrieben, jedoch mit einer Masse aus 100 phr Engage 7467, 78,4 phr Ondina 933, 212 phr Escorez 1310, 8 phr Irganox 1726.
Die Beschichtung ist sehr weich und klebrig wie ein Fliegenfänger. Die Masse ist aufgrund der niedrigen Schmelzviskosität in den Träger eingedrungen. Der beschichtete Ballen konnte nicht zu Rollen aufgeschnitten werden, da bei Abrollen die Masse aufspaltet. Eine Klebkraftmessung ist aus diesem Grund ebenfalls nicht möglich (Kohäsionsbruch). Fogging-Wert: 37.

### Vergleichsbeispiel 4

Die Ausführung erfolgt wie in Beispiel 1 beschrieben, jedoch mit einer Masse aus 100 phr IN FUSE 9107, 78,4 phr Ondina 933, 212 phr Escorez 1310, 8 phr Irganox 1076. Die Beschichtung erfolgt mit 40 g/m² wie in Beispiel 3. Nach 4 Wochen Lagerung der Rollen bei 70 ° ist das Papier vom Öl durchfettet, der Tack der Masse erheblich reduziert und die Rolle deformiert (Hohlstellen). Die Beschichtung ist nicht haftklebrig.

### Vergleichsbeispiel 5

Die Ausführung erfolgt wie in Beispiel 1 beschrieben, jedoch mit einer Masse aus 100 phr IN FUSE 9107, 78,4 phr PB 0300 M, 212 phr Escorez 1310, 8 phr Irganox 1076. Die Beschichtung erfolgt wie in Beispiel 3. Die Beschichtung ist nicht haftklebrig.

### Vergleichsbeispiel 6

Die Ausführung erfolgt wie in Beispiel 5 beschrieben, jedoch mit LD 251 statt IN FUSE 9107. Die Beschichtung ist nicht haftklebrig, sondern hart mit öliger Oberfläche.

### Vergleichsbeispiel 7

Die Ausführung erfolgt wie in Beispiel 5 beschrieben, jedoch mit Engage 7467 statt IN FUSE 9107. Die Beschichtung ist sehr weich und klebrig. Eine Klebkraftmessung ist wegen Kohäsionsbruch nicht möglich.

### Vergleichsbeispiel 8

Die Ausführung erfolgt wie in Beispiel 5 beschrieben, Die Klebemasse besteht aus folgenden Komponenten: 100 phr IN FUSE 9107, 78,4 phr PB 0300 M, 212 phr Escorez 5400, 8 phr Irganox 1076. Die Masse ist kaum haftklebrig

Das erfindungsgemäße Klebeband eignet sich hervorragend für Verpackungsanwendungen, vorzugsweise Verstärkung von Kartonagen, insbesondere im Bereich von Stanzungen, als Aufreißstreifen, als Tragegriff, zur Palettensicherung, als Transportsicherung von Waren, zum Bündeln und insbesondere zum Verschließen von Faltkartons. Beispiele für solche Waren sind PC-Drucker oder Kühlschränke.

Bevorzugterweise ist die Klebemasse lösungsmittelfrei auf dem Träger aufgebracht.

Weiterhin hat es sich als vorteilhaft für die Verwendung als Verpackungsklebeband herausgestellt, wenn das Olefinpolymer ein Ethylenpolymer ist.

Styrolblockcopolymer-Klebemassen, in der Regel basierend auf Styrol-Isopren-Sytrol-Blockcopolymeren, sind nur auf Polypropylenfolien, aber nicht auf Hart-PVC-Folien beschichtbar.
Acrylatklebemassen sind wegen der schlechten Wiederentfernbarkeit für die Transportsicherung von Waren ungeeignet.

Hier schafft die erfindungsgemäße Verwendung des Klebebands als Verpackungsklebeband Abhilfe.

Das Ethylenpolymer weist vorzugsweise einen Schmelzindex von weniger als 6 g/10 min, besonders bevorzugt weniger als 1,5 g/10 min, vorzugsweise einen Biegemodul von weniger als 26 MPa, besonders bevorzugt weniger als 17 MPa auf und/oder enthält ein C₃- bis C₁₀-Olefin, vorzugsweise 1-Octen als Monomer.

Das erfindungsgemäße Ethylenpolymer kann mit Synthesekautschuken kombiniert werden. Dies sind zum Beispiel Polyisobutylen, Butylkautschuk, EPM, EPDM, ungesättigte oder hydrierte Styrolblockcopolymere.

Es stellte sich überraschenderweise heraus, dass Klebrigkeit (Tack) und Klebkraft bei der neuen polyethylenbasierten Klebemasse im Gegensatz zu konventionellen Kautschukmassen von der Polydispersität des Harzes extrem abhängig sind.

Als Klebharze werden Kohlenwasserstoffharze bevorzugt. Neben den bereits genannten sind auch Terpenphenolharze geeignet, führen aber zu nur mäßigem Tack, aber dafür zu sehr guter Scherfestigkeit und zu Alterungsbeständigkeit.

Die Klebemasse kann ohne Antioxidans auskommen. Dies hat den Vorteil, dass bei Anwendung als Transportsicherung von Waren kein Antioxidans auf dem verklebten Gegenstand Verfärbungen verursachen kann. Sodann ist das erfindungsgemäße Klebeband für Verklebungen mit Lebensmittelkontakt geeignet. Bei sehr hoher thermischer Belastung bei Herstellung und Beschichtung der Klebemasse empfiehlt sich der Einsatz eines phenolischen Antioxidans.

Bevorzugt ist der eingesetzte Weichmacher frei von Mineralöl, sondern ist gewählt aus der Gruppe der flüssigen Polymerisate aus Isobutenhomopolymer und/oder Isobuten-Buten-Copolymer und der Ester der Phthal-, Trimellit-, Zitronen- oder Adipinsäure, insbesondere deren Ester von verzweigten Octanolen und Nonanolen.

Weiter vorzugsweise enthält die Klebemasse ein Copolymer aus Ethylen und Buten-(1), Hexen-(1) oder Octen-(1) oder ein Terpolymer aus Ethylen, Propylen und Buten-(1), Hexen-(1) oder Octen-(1), wobei der Biegemodul des Copolymers oder Terpolymers vorzugsweise unter 10 MPa und der Kristallitschmelzpunkt vorzugsweise unter 50 °C liegt, oder ein EPM oder EPDM, vorzugsweise mit einem Ethylengehalt von 40 bis 70 Gew.-% und/oder einer Dichte unter 0,88 g/cm³, besonders bevorzugt unter 0,87 g/cm³, wobei die Menge an Copolymer oder Terpolymer vorzugsweise über 100 phr beträgt.

Als Beschichtungsverfahren werden Extrusionsbeschichtung mit Breitschlitzdüsen und Kalanderbeschichtung bevorzugt. In einer speziellen Ausführungsform besteht die Trägerfolie aus Polyolefin und wird mit der Klebemasse coextrudiert.

Die Klebemasse wird vorzugsweise mit zwischen 15 und 40 g/m², besonders bevorzugt mit zwischen 20 und 30 g/m² auf dem Träger aufgebracht.

Als Träger wird eine Folie aus Hart-PVC (Insbesondere aus Emulsions-PVC) oder aus Polyolefin bevorzugt. Die Folie ist besonders bevorzugt bei der Herstellung monoaxial oder biaxial verstreckt worden ist und/oder weist vorzugsweise eine Dicke zwischen 25 und 200 µm, besonders bevorzugt zwischen 30 und 80 µm auf.

Die Folie kann durch Kaschierung, Prägung oder Strahlenbehandlung modifiziert sein. Die Folien können mit Oberflächenbehandlungen versehen sein. Dies sind zum Beispiel zur Haftvermittlung Corona-, Flamm-, Fluor- oder Plasmabehandlung oder auf der der Trennlackierung abgewandten Seite Beschichtungen von Lösungen oder Dispersionen oder flüssige strahlenhärtbare Materialien.

Das Klebeband beinhaltet vorzugsweise eine auf der der Klebemasse gegenüber liegenden Seite des Trägers befindliche Trennlackung (Release-, Antihaftbeschichtung), zum Beispiel solche aus Silikon, Acrylaten (zum Beispiel Primal® 205), Stearylverbindungen wie Polyvinylstearylcarbamat oder Chromstearatkomplexen (zum Beispiel Quilon® C) oder Umsetzungsprodukten aus Maleinsäureanhydridcopolymeren und Stearylamin. Das Silikon kann lösungsmittelfrei oder lösungsmittelhaltig aufgetragen werden und durch Strahlen, eine Kondensations- oder Additionsreaktion oder physikalisch (zum Beispiel durch eine Blockstruktur) vernetzt sein. Der Trennlack ist vorzugsweise auf Basis Polyvinylstearylcarbamat oder Silikon. Für leise abrollbare Verpackungsklebebänder wird vorzugsweise keine Trennlackierung eingesetzt, sondern die Folienrückseite ist unbehandelt oder mit physikalischen Methoden wie Corona behandelt.

### Beispiel A1

Als Trägerfolie wird die Folie R240 (alte Bezeichnung GA 06) von Klöckner-Pentaplast, Gendorf verwendet. Sie weist die Prägung 441 (zur Verringerung der Abrollkraft), eine Dicke vor dem Prägen von 30 µm auf und ist farblos. Sie enthält E-PVC mit einem K-Wert von 78, ca. 0,6 Gew.-% Zinnstabilisator und ca. 3 Gew.-% Montansäureesterwachs. Die Folie wird im Luvitherm®-Verfahren hergestellt.

Die Unterseite (wo die Prägung nicht erhaben ist) wird coronabehandelt und mit einem Primer aus Naturkautschuk, Cyclokautschuk und 4,4'-Diisocyanato-diphenylmethan versehen.

Die Klebemasse besteht aus folgenden Komponenten

| | |
|---|---|
| 100 phr | IN FUSE 9107 |
| 78 phr | Ondina 933 |
| 212 phr | PRO 10394 |
| 2 phr | Irganox 1076 |

und wird aus der Schmelze mit 25 g/m² aufgetragen.

Die Klebkraft auf Stahl beträgt 2,8 N/cm.

Das Anfassverhalten dieses Beispiels ist gut.

### Beispiel A2

Die Trägerfolie besteht aus im Verhältnis 1:7 in Längsrichtung verstrecktem Polypropylencopolymer mit einer Dicke von 55 µm und rotbrauner Einfärbung. Sie wird auf der Rückseite mit einem kondensationsvernetzenden Silikon beschichtet. Ein Primer wird nicht verwendet.

Die Klebemasse besteht aus folgenden Komponenten

| | |
|---|---|
| 100 phr | IN FUSE 9507 |
| 140 phr | Oppanol B 10 |
| 250 phr | Escorez 1310 |
| 2 phr | Irganox 1076 |

und wird aus der Schmelze mit 28 g/m² aufgetragen.

Die Klebkraft auf Stahl beträgt 6,5 N/cm. Das Anfassverhalten ist gut.

### Beispiel A3

Die Trägerfolie ist Radil TM 35 µm aus biaxial verstrecktem Polypropylenhomopolymer. Sie wird auf der coronabehandelten Seite mit Polyvinylstearylcarbamat aus toluolischer Lösung beschichtet und auf der Vorderseite mit 28 g/m² einer Schmelzhaftklebermasse folgender Zusammensetzung:

| | |
|---|---|
| 100 phr | IN FUSE 9107 |
| 78 phr | Ondina 933 |
| 212 phr | Foral 85 |

Die Klebkraft auf Stahl beträgt 4,8 N/cm. Das Anfassverhalten ist gut.

### Vergleichsbeispiel A1

Die Ausführung erfolgt wie in Beispiel A3 beschrieben, jedoch mit einer Masse aus

| | |
|---|---|
| 100 phr | LD 251 |
| 78 phr | Ondina 933 |
| 212 phr | Escorez 1310 |
| 2 phr | Irganox 1076 |

Die Beschichtung ist nicht haftklebrig, sondern hart mit öliger Oberfläche.

### Vergleichsbeispiel A2

Die Ausführung erfolgt wie in Beispiel A3 beschrieben, jedoch mit einer Masse aus

| | |
|---|---|
| 100 phr | IN FUSE 9107 |
| 78 phr | PB 0300 M |
| 212 phr | Escorez 1310 |
| 2 phr | Irganox 1076 |

Die Beschichtung ist nicht haftklebrig.

Das erfindungsgemäße Klebeband eignet sich weiterhin hervorragend zum Abdecken von Oberflächen zum Lackieren, Sandstrahlen, Putzen mit Mörtel oder Transportieren insbesondere für Anwendungen mit Außenbewitterung und vor allem zum Schutz der Lackierung von Fahrzeugen.

Denn Kautschukklebemassen bestehen üblicherweise aus Naturkautschuk, einem Klebharz, einem Weichmacher und einem phenolischen Antioxidans und sind relativ wenig alterungs- und UV-beständig.
Acrylatklebemassen weisen eine hervorragende alterungs- und UV-Stabilität auf, leider haften sie auf unpolaren Untergründen schlecht. Sie sind von sehr polaren Untergründen wie Aluminium, Glas oder PVC nicht wieder entfernbar und daher für solche Maskierungsanwendungen ungeeignet. Insbesondere nach längerer Bewitterung sind fast alle Klebebänder nicht mehr völlig rückstandsfrei entfernbar.

Das erfindungsgemäße Abdeckklebeband ist alterungs- und UV-stabil, die Haftung für polare und unpolare Untergründe ist adjustierbar und ist auch lösungsmittelfrei verarbeitbar.

Bevorzugterweise ist die Klebemasse aus der Schmelze mindestens einseitig beschichtetet.

Weiterhin hat es sich als vorteilhaft für die Verwendung als Abdeckklebeband herausgestellt, wenn das Olefinpolymer ein Ethylenpolymer ist.

Das Ethylenpolymer weist vorzugsweise einen Schmelzindex von weniger als 6 g/10 min, besonders bevorzugt weniger als 1,5 g/10 min, vorzugsweise einen Biegemodul von weniger als 26 MPa, besonders bevorzugt weniger als 17 MPa auf und/oder enthält ein C₃- bis C₁₀-Olefin, vorzugsweise 1-Octen als Monomer.
Das Ethylenpolymer weist bevorzugt eine Struktur aus kristallinen Polyethylenblöcken und im Wesentlichen amorphen Blöcken aus Ethylen und einem C₃- bis C₁₀-Olefin auf. Das erfindungsgemäße Ethylenpolymer kann mit den bei Kautschukmassen bekannten Elastomeren wie Naturkautschuk oder Synthesekautschuken kombiniert werden. Vorzugsweise werden wegen der UV-Stabilität ungesättigte Elastomere wie Naturkautschuk, SBR, NBR oder ungesättigte Styrolblockcopolymere nur in geringen Mengen oder besonders bevorzugt gar nicht verwendet. In der Hauptkette gesättigte Synthesekautschuke wie Polyisobutylen, Butylkautschuk, EPM, EPDM oder hydrierte Styrolblockcopolymere werden für den Fall einer gewünschten Modifikation bevorzugt.

Es stellte sich überraschenderweise heraus, dass Klebrigkeit (Tack) und Klebkraft bei der neuen polyethylenbasierten Klebemasse im Gegensatz zu konventionellen Kautschukmassen von der Polydispersität des Harzes extrem abhängig sind.

Die Klebemasse enthält gemäß einer bevorzugten Ausführungsform
- ein primäres Antioxidans vorzugsweise in einer Menge von mindestens 2, besonders bevorzugt mindestens 6 phr und/oder mit einer sterisch gehinderten phenolischen Gruppe,
- ein sekundäres Antioxidans in einer Menge von 0 bis 5, vorzugsweise in einer Menge von 0,5 bis 1 phr und/oder aus der Klasse der Schwefelverbindungen oder der Klasse der Phosphite,
- ein Lichtschutzmittel, vorzugsweise ein HALS und/oder
- einen UV-Absorber.

Als Klebharz hat sich herausgestellt dass der Harze auf Basis von Kolophonium (zum Beispiel Balsamharz) oder Kolophoniumderivaten (zum Beispiel disproportioniertes, dimerisiertes oder verestertes Kolophonium), vorzugsweise partiell oder vollständig hydriert, gut geeignet sind.

Die Klebemasse enthält vorzugsweise einen flüssigen Mineralöl freien Weichmacher wie beispielsweise Ester der Phthal-, Trimellit-, Zitronen- oder Adipinsäure, Wollwachs, flüssige Kautschuke (zum Beispiel niedermolekulare Nitril-, Butadien- oder Polyisoprenkautschuke), flüssige Polymerisate aus reinem Isobuten oder Isobuten-Buten-Copolymer, Flüssig- und Weichharze mit einem Schmelzpunkt unter 40 °C auf Basis der Rohstoffe von Klebharzen, insbesondere der oben aufgeführten Klassen an Klebharz. Besonders bevorzugt werden flüssige Polymerisate aus Isobuten und vor allem Copolymere aus Isobuten und Buten.

Aus den erwähnten Gründen ist die Klebemasse daher im Wesentlichen frei von Mineralölen.

Für Außenanwendungen werden der Klebemasse vorzugsweise Lichtschutzmittel und/oder UV-Absorber eingesetzt wie sie zum Beispiel unter den Handelsnamen Chimassorb und Tinuvin bekannt sind. Besonders bevorzugt sind aminische Lichtschutzmittel die der Fachmann als HALS bezeichnet.

Als Träger wird Papier, Gewebe, Gewirke, Tissue, unverstreckte oder verstreckte Folie aus Polypropylen, Polyethylen, Polyester oder PVC, vorzugsweise ein Papier oder eine unverstreckte Polypropylenfolie, bevorzugt.

Die Herstellung und Verarbeitung der Haftklebemassen kann aus Lösung sowie aus der Schmelze erfolgen. Bevorzugte Herstell- und Verarbeitungsverfahren erfolgen aus der Schmelze. Für den letzteren Fall umfassen geeignete Herstellprozesse sowohl Batchverfahren als auch kontinuierliche Verfahren. Besonders bevorzugt ist die kontinuierliche Fertigung der Haftklebemasse mit Hilfe eines Extruders und anschließender Beschichtung direkt auf das zu beschichtende Substrat bei entsprechend hoher Temperatur der Klebmasse. Als Beschichtungsverfahren werden Extrusionsbeschichtung mit Breitschlitzdüsen und Kalanderbeschichtung bevorzugt.

Der Masseauftrag (Beschichtungsstärke) liegt vorzugsweise zwischen 10 und 120 besonders bevorzugt zwischen 20 und 70 g/m².

### Beispiel B1

Ein bevorzugtes Klebeband für diese Anwendung entspricht dem gemäß Beispiel 2.

### Beispiel B2

Ein bevorzugtes Klebeband für diese Anwendung entspricht dem gemäß Beispiel 4.

Das erfindungsgemäße Klebeband eignet sich weiterhin hervorragend als Wickelband zum Bündeln, Schützen, Kennzeichnen, Isolieren oder Abdichten von Lüftungsrohren oder Lüftungsleitungen in Klimaanlagen, von Drähten oder von Kabeln und vorzugsweise zum Ummanteln von Kabelsätzen in Fahrzeugen sowie von Feldspulen für Bildröhren.

Kabelwickelbänder und Isolierbänder bestehen üblicherweise aus weichgemachter PVC-Folie mit einer einseitigen Haftklebstoffbeschichtung. Entsprechende Nachteile beinhalten Ausdampfung von Weichmacher und hoher Halogengehalt. Wickelbänder auf Basis von Weich-PVC-Folien werden in Automobilen zur Bandagierung von elektrischen Leitungen zu Kabelbäumen eingesetzt. Stand in den Anfängen der technischen Entwicklung die Verbesserung der elektrischen Isolation bei Verwendung dieser ursprünglich als Isolierbänder entwickelten Wickelbänder im Vordergrund, müssen derartige Kabelsatzbänder mittlerweile weitere Funktionen erfüllen, wie die Bündelung und dauerhafte Fixierung einer Vielzahl von Einzelkabeln zu einem stabilen Kabelstrang, sowie den Schutz der Einzelkabel und des gesamten Kabelstrangs gegen mechanische, thermische und chemische Schäden.

Es gibt Bemühungen, statt Weich-PVC-Folie Gewebe oder Vliese zu verwenden, die daraus resultierenden Produkte werden aber in der Praxis nur wenig eingesetzt, da sie relativ teuer sind und sich in der Handhabung (zum Beispiel Handeinreißbarkeit, elastisches Rückstellvermögen) und unter Nutzungsbedingungen (zum Beispiel Beständigkeit gegen Betriebsflüssigkeiten, elektrische Eigenschaften) stark von den gewohnten Produkten unterscheiden, wobei im folgenden ausgeführt der Dicke eine besondere Bedeutung zukommt.

In der (Patent-)Literatur werden auch Wickelbänder mit Trägern aus Polyolefinen beschrieben. Sie sind mit Klebemassen aus Kautschuk oder Acrylat ausgerüstet.
Kautschuklebemassen haben den Vorteil, dass die Klebeigenschaften gut eingestellt werden können. Für Anwendungen im Motorraum sind Kautschukkleber nicht geeignet, sie verursachen unter den üblichen Prüfbedingungen je nach Kundenspezifikation nach 3000 Stunden bei 105 °C, 3000 Stunden 125 °C beziehungsweise 168 Stunden bei 140 °C eine Versprödung der Kabelisolierung aus Polyethylen und Polypropylen und insbesondere aus PVC und zum Teil auch eine Versprödung des Polyolefinträgers. Acrylatmassen weisen auf der Folienrückseite eine geringe Haftung auf, wodurch die Abrollkraft gering ausfällt, das heißt, sie liegt bei für mindestens einen Monat bei 25 °C gelagerten Rollen bei 300 mm/min Abrollgeschwindigkeit unter 1 N/cm, für die Anwendung sollte sie aber für eine faltenfreie Wicklung und ohne Ermüdung der verarbeitenden Person zwischen 1,6 und 3,0 N/cm liegen. Mit einer Coronabehandlung auf der Folienrückseite lässt sich die Abrollkraft erhöhen, sie liegt dann aber schon bei einer geringen Coronaleistung bei circa 4 N/cm und steigt bei längerer Lagerung weiter an.
Silikonhaftkleber könnten Abhilfe schaffen, wenn sie nicht extrem teuer und auch lösungsmittelfrei erhältlich wären.

Dispersionsbeschichtungen von Haftklebern sind potenziell durch Wassereinwirkungen gefährdet, was zu Klebkraftverlust (Flagging des Wickelendes) und Verschlechterung der elektrischen Eigenschaften führt. Lösungsmittelmassen sind diesbezüglich vorteilhaft entsprechen aber nicht neuen Anforderungen an VOC-Freiheit (VOC = volatile organic compounds) in Fahrzeugen und genügen nicht modernen Ansprüchen an Arbeitshygiene und -sicherheit.

Für den Fachmann überraschend und nicht vorhersehbar ist ein solches Wickelband aus einer Polyolefinfolie sowie einer Polyolefinhaftklebstoffschicht herstellbar.

Gemäß einer bevorzugten Ausführungsform des Wickelbands besteht der Träger aus einem halogenfreien Polyolefinträger, weiter vorzugsweise ist die Klebemasse lösungsmittelfrei aufgetragen.

Die Klebemasse enthält vorzugsweise mindestens ein Polyolefin auf Basis von Ethylen, Propylen, 1-Buten oder 1-Octen, weiter vorzugsweise eine Mischung von mindestens zwei solchen Polyolefinen.

Die Klebemasse enthält des Weiteren vorzugsweise ein sehr weiches und kaum kristallines Olefinpolymer. Dieses ist vorzugsweise ein Copolymer aus Ethylen, Propylen, Buten-(1), Hexen-(1) und/oder Octen-(1), die zum Beispiel unter den Handelsnamen Exact®, Engage®, Versify® oder Tafmer® bekannt sind, oder ein Terpolymer aus Ethylen, Propylen, Buten-(1), Hexen-(1) und/oder Octen-(1), wobei der Biegemodul vorzugsweise unter 20 MPa und der Kristallitschmelzpunkt vorzugsweise unter 50 °C liegt.

Der erfindungsgemäß im Wickelband bevorzugte Träger enthält ein Olefinpolymer ohne oxidationsempfindliche Doppelbindungen und könnte daher ohne Antioxidans auskommen. Für eine hohe Langzeitstabilität werden aber vorzugsweise ein primäres Antioxidans und besonders bevorzugt auch ein sekundäres Antioxidans verwendet. Die Träger enthalten in den bevorzugten Ausführungsformen mindestens 2 phr, besonders bevorzugt 6 phr primäres Antioxidans oder vorzugsweise mindestens 2 phr, insbesondere mindestens 6 phr einer Kombination aus primären und sekundären Antioxidans, wobei die primäre und sekundäre Antioxidansfunktion nicht in verschiedenen Molekülen vorliegen muss, sondern auch in einem Molekül vereinigt sein kann. Die Menge an sekundärem Antioxidans beträgt bevorzugt bis 5 phr, besonders bevorzugt 0,5 bis 1 phr. Überraschend wurde gefunden, dass eine Kombination von primären Antioxidantien (zum Beispiel sterisch gehinderten Phenolen oder C-Radikalfängern wie CAS 181314-48-7) und sekundären Antioxidantien (zum Beispiel Schwefelverbindungen, Phosphiten oder sterisch gehinderten Aminen) eine verbesserte Verträglichkeit ergibt. Vor allem wird die Kombination von einem primären Antioxidans, vorzugsweise sterisch gehinderten Phenolen mit einer relativen Molmasse von mehr als 500 Dalton, mit einem sekundären Antioxidans aus der Klasse der Schwefelverbindungen oder aus der Klasse der Phosphite bevorzugt mit einer relativen Molmasse von mehr als 500 Dalton bevorzugt, wobei die phenolische, die schwefelhaltigen und die phosphitische Funktionen nicht in drei verschiedenen Molekülen vorliegen müssen, sondern auch mehr als eine Funktion in einem Molekül vereinigt sein können.

Bei Anwendungen, bei denen das Wickelband längere Zeit dem Licht (zum Beispiel der Sonneneinstrahlung) ausgesetzt ist, wird vorzugsweise ein Lichtschutzmittel besonders bevorzugt ein HALS wie Tinuvin 111, ein UV-Absorber wie Tinuvin P oder deckendes Pigment eingesetzt.

Die Folie enthält vorzugsweise Polyolefine auf Basis von Ethylen, Propylen, 1-Buten oder 1-Octen, besonders bevorzugt eine Mischung von Polyolefinen.

Sie kann durch Kalandrieren oder Extrusion, vorzugsweise Coextrusion, wie zum Beispiel im Blas- oder Castprozess hergestellt sein. Durch Vernetzung ist das Wickelband sogar unschmelzbar. Dies ist zum Beispiel durch ionisierende Strahlung wie Elektronen- oder γ-Strahlung oder Peroxide möglich. Besonders bevorzugt ist das Verfahren der Coextrusion von Träger- und Haftkleberschicht.

Die Folie kann Flammschutzmittel wie Polyphosphate, Carbonate und Hydroxide des Aluminiums, Calciums oder des Magnesiums, Borate, Stannate, Flammschutzmittel auf Stickstoffbasis wie Melamincyanurat, Dicyandiamid, roter Phosphor oder sterisch gehinderte Amine wie zum Beispiel die Klasse der HA(L)S oder halogenhaltige Flammschutzmittel wie Decabromdiphenyloxid, Hexabomcyclododecan oder Polymere auf Basis Dibromstyrol enthalten.

Weitere bei Folien übliche Additive wie Füllstoffe, Pigmente, Licht- oder Alterungsschutzmittel, Nukleierungsmittel, Impactmodifier oder Gleitmittel und andere können zur Herstellung verwendet werden.

Die Dicke des Wickelbandes liegt vorzugsweise im Bereich von 30 bis 180 µm, bevorzugt 50 bis 150 µm, insbesondere 55 bis 100 µm. Die Oberfläche kann strukturiert oder glatt sein. Vorzugsweise ist die Oberfläche leicht matt eingestellt. Dies kann durch Verwendung eines Füllstoffs mit einer hinreichend hohen Teilchengröße oder durch eine Walze (zum Beispiel Prägewalze am Kalander oder mattierte Chill Roll oder Prägewalze bei der Extrusion) erreicht werden.

Die mechanischen Eigenschaften des erfindungsgemäßen Wickelbandes liegen in md (Maschinenrichtung) bevorzugt in den folgenden Bereichen:
- Kraft bei 1 %-Dehnung 0,6 bis 4 N/cm auf, besonders bevorzugt 1 bis 3 N/cm
- Kraft bei 100 %-Dehnung 5 bis 20 N/cm, besonders bevorzugt 8 bis 12 N/cm.
- Reißdehnung von 200 bis 1000 %, besonders bevorzugt von 300 bis 400 %,
- Reißkraft im Bereich von 6 bis 40 N/cm, besonders bevorzugt von 8 bis 15 N/cm,

Zur Ermittlung der Daten wird die Folie mit scharfen Klingen zugeschnitten.

Das erfindungsgemäße Wickelband weist vorzugsweise eine Wärmestabilität von mindestens 105 °C, besonders bevorzugt mindestens 125 °C nach 3000 Stunden auf, das heißt, dass nach dieser Lagerung noch eine Bruchdehnung von mindestens 100 % vorhanden ist und die umwickelten Drähte gemäß Norm LV 312 nicht verspröden.

Die Abrollkraft liegt zwischen vorzugsweise 1,0 und 3,8 N/cm, besonders bevorzugt zwischen 1,6 und 3,0 N/cm.

Das Wickelband ist ausgezeichnet zum Umwickeln von langgestrecktem Gut wie Feldspulen oder Kabelsätzen in Fahrzeugen geeignet. Herausragend ist die hohe Alterungsstabilität. Das Wickelband ist daher ebenfalls für andere Daueranwendungen geeignet wie zum Beispiel für Lüftungsrohre im Klimabau. Darüber hinaus ist erwünscht, dass das Wickelband den Kabelstrang elastisch zusammenzieht wofür eine hinreichende Dehnung des Trägers durch die Abrollkraft notwendig ist. Dieses Verhalten ist auch zur Abdichtung der Lüftungsrohre notwendig. Herausragend ist die hohe Alterungsstabilität. Diese Eigenschaften können von einem Wickelband basierend auf der erfindungsgemäßen Polyolefinmasse erreicht werden.

### Beispiel C1

Zur Herstellung des Trägerfilmes wird eine Blasfolie extrudiert. Sie besteht auf der Außenseite aus einem Ethylencopolymer mit Na-Ionen (Surlin 1601-2, DuPont) und auf der zu beschichtenden Seite aus LDPE (LD 251).
Die erhaltene Folie wird auf der Innenseite einseitig coronabehandelt und anschließend auf dieser Seite mit 20 g/m² eines Schmelzhaftklebers beschichtet. Das Schneiden erfolgt durch Abstechen der erhaltenen Stangen mittels rotierender Messer (round blade) in Rollen zu 15 mm Breite.

### Zusammensetzung des Schmelzhaftklebers:

| | |
|---|---|
| 100 phr | IN FUSE 9107, |
| 50 phr | Wingtack 10, |
| 180 phr | Foral 85, |
| 8 phr | Irganox 1726. |

Die Abrollkraft beträgt 2,0 N/cm, Kabelstränge lassen sich faltenfrei umwickeln, nach Lagerung 3000 Stunden bei 125 °C sind weder Trägerfolie noch die Drahtisolierungen versprödet und die Klebemasse ist noch haftklebrig.

### Beispiel C2

Zur Herstellung des Trägerfilmes werden zunächst in einem gleichläufigen Doppelschneckenextruder 100 phr Hifax CA10A, 10 phr Vinnapas B 10, 165 phr Magnifin H 5 GV, 10 phr Flammruß 101, 0,8 phr Irganox 1010, 0,8 phr Irganox PS 802 und 0,3 phr Irgafos 168 compoundiert. Das Magnifin wird zu je 1/3 in den Zonen 1, 3, und 5 zugegeben. Der Compound wird im Flachfolienverfahren mit der Haftklebemasse coextrudiert und zu Standen gewickelt welche anschließend abgestochen werden. Die Dicke der Trägerschicht beträgt 100 µm und die der Klebmasseschicht 22 g/m².

### Zusammensetzung der Klebemasse:

| | |
|---|---|
| 100 phr | Softell CA02A, |
| 70 phr | Oppanol B 10, |
| 180 phr | Regalite R1100, |
| 8 phr | Irganox 1726 |

Die Abrollkraft beträgt 2,5 N/cm, Kabelstränge lassen sich faltenfrei umwickeln, nach Lagerung 3000 Stunden bei 105 °C sind weder Trägerfolie noch die Drahtisolierungen versprödet und die Klebemasse ist noch haftklebrig.

### Vergleichsbeispiel C1

Eine Folie wie in Beispiel C1 wird mit 20 g/m² einer Acrylathaftklebemasse beschichtet und getrocknet. Die Abrollkraft beträgt 0,5 N/cm, der Kabelstrang ist faltig gewickelt. Nach Alterung 3000 Stunden bei 105 °C beziehungsweise 125 °C sind Trägerfolie und Drahtisolierungen aus PP, PE und PVC in Ordnung. Nach 3000 Stunden bei 105 °C ist die Klebrigkeit (Tack) der Masse wegen Nachvernetzung nur noch schwach.

### Vergleichsbeispiel C2

Eine Folie wie in Beispiel C1 wird mit 20 g/m² einer Naturkautschuklösungsmittelmasse mit einem Kolophoniumester beschichtet und getrocknet. Die Abrollkraft beträgt 2,5 N/cm, der Kabelstrang ist gut gewickelt. Nach Alterung 3000 Stunden bei 105 °C sind Trägerfolie und Drahtisolierungen aus PP und PE in Ordnung und Drahtisolierungen aus PVC versprödet. Nach Alterung 3000 Stunden bei 125 °C sind die Trägerfolie und alle Drahtisolierungen versprödet. Nach 3000 Stunden bei 105 °C ist die Masse völlig versprödet.

Das erfindungsgemäße Klebeband eignet sich neben der Verwendung als Wickelband ganz besonders vorteilhaft zum Umwickeln von Kabeln.

Bevorzugterweise ist die Klebemasse lösungsmittelfrei auf dem Träger aufgebracht.

Weiterhin hat es sich als vorteilhaft für die Verwendung als Kabelummantelungsklebeband herausgestellt, wenn das Olefinpolymer ein Ethylenpolymer ist und/oder der Träger ein textiler Träger.

Elektrische und elektromechanische Bauteile sowie die Umhüllungen von elektrischen Leitungen bestehen vielfach aus polymeren Werkstoffen, wobei Polyvinylchlorid (PVC) einen historisch bedingt und aufgrund seiner Verfügbarkeit sowie seiner exzellenten Werkstoffeigenschaften wichtigen Kunststoff darstellt. Insbesondere bestehen Ummantelungen von Kupferadern überwiegend aus PVC-Compounds, sofern nicht Rahmenbedingungen wie Hochtemperaturanforderungen oder Halogenfreiheit Alternativen erzwingen.

Für den mechanischen und elektrischen Schutz derartiger Kabel sind in der Vergangenheit Selbstklebebänder entwickelt worden, die allgemein für den Schutz und zur Isolierung sowie zum Bandagieren von elektrischen Leitungen und Bauteilen in erheblichem Umfang eingesetzt werden. Die Selbstklebebänder erlauben es, einen Langzeitverbund herzustellen, ohne dass durch Wechselwirkungen zwischen Klebeband und Kabelumhüllung Schäden an dem Kabel auftreten. Diese bestehen heute überwiegend aus einer Weich-PVC-Folie und einer Kautschukklebmasse. Für spezielle Anwendungen zum Beispiel in der Temperaturklasse T3 (siehe unten) oder bei Anforderungen an Dämpfung werden Klebbänder mit einem textilen Träger zum Beispiel einem Polyester- oder Zellwoll-Gewebe verwendet.

Im Rahmen der Diskussion über die Umweltverträglichkeit von PVC besteht die Tendenz, diesen Werkstoff durch Alternativen zu ersetzen. Elektrische Bau- und Hilfsteile sowie auch die Ummantelung von Kupferdrähten werden verstärkt mit anderen Kunststoffen ausgeführt, wobei für höhere Anforderungen Fluorpolymere, thermoplastische Polyester, Polyurethane, Polyphenylenoxid und vernetztes Polyethylen zum Einsatz kommen. Für den kostensensiblen Massenbereich mit geringeren Temperaturanforderungen finden verstärkt Werkstoffe auf Polypropylenbasis Verwendung.

Auch für Kabelsätze in Fahrzeugen ist die Tendenz zu derartigen PVC-freien Leitungen gegeben, während Bauteile wie Steckverbindungen, Schalter, Rillrohre etc. bereits überwiegend aus PVC-freien Werkstoffen gefertigt werden. Im Folgenden werden für die Prüfungen die Bezeichnungen Drahtisolierung, Ummantelung, Kabel, Kabelsatz und Leitungen synonym benutzt.

Elektrische Leitungsstränge oder Bauteile, die mit Selbstklebebändern umwickelt sind, müssen eine sichere Funktionsfähigkeit über die gesamte Lebensdauer des Gesamtproduktes, wie zum Beispiel die eines Fahrzeuges, gewährleisten. Bei Auswahl ungeeigneter Klebebänder können sich während des Produktlebens Unverträglichkeiten ergeben, die Schädigungen der Kabel bis hin zu extremer Versprödung nach sich ziehen. Korrosion und Kurzschlüsse mit der Gefahr des Ausfalls der gesamten Elektrik/Elektronik sind mögliche Folgen. Besonders bei Fahrzeugen wie Personenkraftwagen oder Lastkraftwagen werden an die Verträglichkeit sehr hohe Ansprüche gestellt; im Passagierraum können Spitzentemperaturen bis zu 80 °C auftreten, im Motorraum noch weit höhere Temperaturen. Deshalb hat sich für den Einsatzbereich der Kabelwickelbänder ein Langzeittest über 3000 Stunden, wie er in der Automobilprüfungsrichtlinie LV 312 beschrieben ist, als Standardprüfung durchgesetzt. Dieser beschreibt die Verträglichkeitsprüfung detailliert:
Musterkabelsätze werden bei den Prüftemperaturen gelagert und nach festgelegten Zeiträumen, meist alle 500 Stunden, um einen Dorn mit definiertem Durchmesser gebogen und anschließend auf Schädigungen untersucht. Dieser Test läuft über eine Gesamtdauer von 3000 Stunden. Die Prüftemperaturen richten sich nach den Temperaturklassen der Kabelsätze und betragen 90 °C bis 150 °C abhängig von dem Einsatzgebiet des Kabelbaums im Passagier- oder Motorraum. Die LV 312-Norm sieht vor, dass für ein Klebeband für den Temperaturbereich T2 nach 3000 Stunden bei 105 °C eine Verträglichkeit zwischen Klebeband und Drahtisolierung gewährleistet werden muss. Da in diesem Temperaturbereich in Europa hauptsächlich Kabel mit PVC-Ummantelung eingesetzt werden, muss auch der Test mit Klebeband auf solchen Kabeln durchgeführt werden. In der nächst höheren Temperaturklasse T3 kommen für die Tests hauptsächlich Drähte mit Isolierungen aus Polypropylen und strahlenvernetztem Polyethylen (XPE) zum Einsatz. Die Prüftemperatur beträgt dann 125 °C statt 105 °C. Zusätzlich zu den in LV 312 als Referenz festgelegten Leitungen bestimmter Hersteller kann der gleiche Test prinzipiell auch an Leitungen durchgeführt werden, die andere internationale Standards erfüllen wie zum Beispiel die SAE J1128 - TXL- oder die SAE J1128 - TWP-Norm in den USA.

Gemäß der Prüfmethode LV 312 werden wie nachfolgend beschrieben Musterkabelsätze hergestellt. Zwei gleiche Adern mit dem Leitungsquerschnitt 0,35 mm² werden mit einer Schlaglänge von ca. 2 cm verdrillt. Die Leitungsbündel werden mit dem zu prüfenden Klebeband (Breite 19 mm) mit ca. 50 % Überlappung schraubenförmig bewickelt. Als Leitungen werden dabei bei einer Prüftemperatur von 105 °C PVC-Leitungen (Herstellerbezeichnungen Gebauer & Griller 67218 oder Coroplast 46443) benutzt. Bei einer Prüftemperatur von 125 °C werden PP-Leitungen der Firma Tyco (Herstellerbezeichnung: AGP 0219) und XPE-Leitungen der Firmen Acome (Herstellerbezeichnung: T4104F) oder der Firma Draka (Herstellerbezeichnung: 971130) benutzt.

Die mit Klebeband bewickelten Leitungssätze aus entsprechenden Referenzleitungen sowie zusätzlich eine unbewickelte Nullprobe werden für die Dauer von 3000 h bei 105 °C beziehungsweise 125 °C freihängend in einem Ofen mit natürlicher Belüftung gelagert. Alle 500 h wird jeweils ein Prüfling entnommen. Der Kabelsatz wird mindestens 3 h, jedoch maximal 48 h bei Prüfklima konditioniert und anschließend wie folgt geprüft.

Ein Leitungssatzabschnitt wird um einen Dorn mit dem Durchmesser 20 mm gewickelt und visuell begutachtet. Danach wird der Prüfling vom Klebeband befreit und aufgedrillt. Dabei muss das Wickelband zunächst ohne offensichtliche Beschädigung der Leitung abgelöst werden können. Anschließend werden die Einzeladern geprüft. Eine Einzelader wird mindestens zweimal eng um einen Dorn mit dem Durchmesser 2 mm, die andere um einen Dorn mit dem Durchmesser 10 mm gewickelt, visuell begutachtet und jeweils eine Spannungsprüfung durchgeführt.
Wenn bei Prüfungen der Einzeladern um einen 2 mm Dorn die Drahtisolierungen keinerlei Risse, Brüche oder Versprödungen aufweisen und nicht aufgequollen oder geschrumpft sind, gilt das Klebeband als mit der Drahtisolierung verträglich. Eine Verfärbung der Leitung ist zulässig. Die Ursprungsfarbe muss aber noch erkennbar sein.

Bekannt für derartige Kabelwickelanwendungen sind Klebebänder mit einem bandförmigen Träger aus Weich-PVC-Folie oder Textilien auf Gewebe- oder Nähvliesbasis. Bänder mit einem Nähvliesträger werden beispielsweise in der DE 94 01 037 U1 beschrieben. Als Klebebeschichtung werden bevorzugt druckempfindliche Haftklebebeschichtungen eingesetzt. Bisher kommen auf textilen Trägern Haftklebmassen auf Basis von Naturkautschuk und Styrolblockcopolymeren zum Einsatz. Dabei zeigen diese Klebmassen auf Basis von Naturkautschuk praktisch immer Schwächen beim Verträglichkeitstest nach LV 312, sowohl auf PVC- als auch auf polyolefinischen Kabelummantelungen. Da Naturkautschuk-Klebmassen aus Lösung verarbeitet werden, ist diese Technologie nicht zukunftsweisend. Klebmassen auf Basis von ungesättigten Styrolblockcopolymeren, die auch aus der Schmelze ohne Lösungsmittel verarbeitet werden können, erreichen nur auf wenigen Sorten von Kabeln mit PVC-Drahtisolierungen eine Verträglichkeit für den Temperaturbereich T2 (3000 Stunden-Test bei 105 °C, wobei die eingesetzten Kabel ebenfalls nach der Temperaturklasse T2 spezifiziert sind. Die Bandbreite der auftretenden Schädigungen reicht von leichten Rissbildungen in den Kabelummantelungen durch Versprödung bis hin zum vollständigen Ausfall durch Zerbröckeln von Bauteilen und Drahtumhüllungen nach der Lagerung. Für die Temperaturklasse T3 (3000 Stunden-Test bei 125 °C) gibt es bisher keine guten Haftkleber, Acrylate sind zwar temperaturstabil jedoch lösungsmittelhaltig oder als Dispersion auf Textilträgern nicht beschichtbar, ein am Markt befindlicher Acrylathotmelt ist sehr teuer und verliert bei der Lagerung unter T2- und T3-Bedingungen durch Nachvernetzung seine Haftklebrigkeit.

Die bevorzugte Ausführungsform des Klebebands aus einem textilen Träger und aus dem erfindungsgemäßen Haftkleber weist gegenüber analogen Klebebändern auf Basis von Naturkautschuk oder ungesättigten Styrolblockcopolymeren nicht nur Vorteile in der Kabelverträglichkeit auf, sondern auch in der Verträglichkeit gegenüber Rillrohren aus Polypropylen und Polyamid, wie sie in Kabelbäumen im Automobilbau üblich sind.

Das erfindungsgemäße Ethylenpolymer weist vorzugsweise einen Schmelzindex von weniger als 6 g/10 min, besonders bevorzugt weniger als 1,5 g/10 min auf. Der Biegemodul des Ethylenpolymers beträgt vorzugsweise weniger als 26 MPa, besonders bevorzugt weniger als 17 MPa.

Das Ethylenpolymer enthält vorzugsweise ein C₃- bis C₁₀-Olefin insbesondere 1-Octen als Comonomer. Das Ethylenpolymer weist bevorzugt eine Struktur aus kristallinen Polyethylenblöcken und im Wesentlichen amorphen Blöcken aus Ethylen und einem C₃-bis C₁₀-Olefin auf.

Klassische textile Klebebänder neigen bei Lagerung einerseits zu Deformation (Bildung von Nasen und Hohlstellen) und andererseits steigen durch kalten Fluss der Klebemasse die Abrollkräfte immer weiter an, bis das Abrollen für den Anwender zu schwer wird oder die Klebemasse bei Abrollversuch gar aufspaltet. Daher ist ein weiterer überraschender Vorteil des erfindungsgemäßen Klebebands die Lagerstabilität der erfindungsgemäßen Klebebandrollen. Selbst nach einem Monat Lagerung bei 70 °C ist der Erfindungsgegenstand noch gut abwickelbar.

Darüber hinaus fordert die Norm LV 312, dass die Haftklebemasseschicht nach der Wärmelagerung analog der Verträglichkeitsprüfung noch Haftklebrigkeit aufweisen sollte. Klebebänder auf Basis von Naturkautschuk oder ungesättigten Styrolblockcopolymeren verlieren schon nach 500 bis 1500 Stunden ihr Klebvermögen völlig. Offenbar ist bei textilen Trägern der Sauerstoffdurchlass so hoch, dass die Klebemasse stark oxidiert. Im Fall von hydrierten Styrolblockcopolymeren, welche für solche Anwendungen nicht nur zu teuer sind, sondern auch im Wesentlichen zu geringe Klebkräfte aufweisen, geht das Klebvermögen ebenfalls fast vollständig zurück. Die Ursache dafür liegt hauptsächlich darin, dass diese Klebemassen bei Testtemperatur schmelzen und die Schmelze von dem Textilträger aufgesaugt wird, so dass sich der Haftkleber im Wesentlichen nicht mehr an der Oberfläche befindet. Dieser Effekt wird auch bei den ungesättigten Styrolblockcopolymeren beobachtet. Die erfindungsgemäße Klebemasse dringt überraschenderweise bei 105 °C nur geringfügig in den textilen Träger ein und bleibt gut haftklebrig, im Fall der Verwendung geeigneter Alterungsschutzmittel sogar mit noch recht guten klebtechnischen Daten.

Das erfindungsgemäße Ethylenpolymer kann mit den bei Kautschukmassen bekannten Elastomeren wie Naturkautschuk oder Synthesekautschuken kombiniert werden. Vorzugsweise werden ungesättigte Elastomere wie Naturkautschuk, SBR, NBR oder ungesättigten Styrolblockcopolymere nur in geringen Mengen oder besonders bevorzugt gar nicht verwendet. In der Hauptkette gesättigte Synthesekautschuke wie Polyisobutylen, Butylkautschuk, EPM, HNBR, EPDM oder hydrierte Styrolblockcopolymere werden für den Fall einer gewünschten Modifikation bevorzugt.

Die Klebemasse enthält vorzugsweise die genannten Weichmacher. Mineralöle eignen sich sehr gut, das Ethylenpolymer klebrig einzustellen, sind jedoch zu flüchtig um gute Fogging-Werte (DIN 75201), also zum Beispiel > 60, zu erreichen.

Klassische PVC-Klebebänder mit DOP als Weichmacher weisen einen Fogging-Wert von 30 bis 35 auf, der Erfindungsgegenstand soll diesbezüglich möglichst einem PVC-Klebeband überlegen sein. Darüber hinaus sind Klebemassen mit Trimellitatweichmacher (TOTM) oder flüssigem Polyisobutylen (zum Beispiel Oppanol® B 10) nach 3000 Stunden Lagerung bei 125 °C deutlich klebriger als bei Verwendung eines Mineralöls. Aus besagten Gründen ist die Klebemasse daher bevorzugt im Wesentlichen frei von Mineralölen.

Der Schmelzpunkt des Klebharzes (Bestimmung nach DIN ISO 4625) liegt vorzugsweise unter 90 °C.

Die erfindungsgemäße Klebemasse enthält jedoch ein Ethylenpolymer ohne oxidationsempfindliche Doppelbindungen und sollte daher ohne Antioxidans auskommen. Überraschenderweise wurde gezeigt, dass Antioxidantien die Kompatibilität der Klebemasse mit den Drahtisolierungen verbessern.
Daher wird erfindungsgemäß vorzugsweise ein primäres Antioxidans und besonders bevorzugt auch ein sekundäres Antioxidans verwendet. Die erfindungsgemäßen Klebemassen enthalten in den bevorzugten Ausführungsformen mindestens 2 phr, besonders bevorzugt 6 phr primäres Antioxidans oder vorzugsweise mindestens 2 phr, insbesondere mindestens 6 phr einer Kombination aus primären und sekundären Antioxidans, wobei die primäre und sekundäre Antioxidansfunktion nicht in verschiedenen Molekülen vorliegen muss, sondern auch in einem Molekül vereinigt sein kann.
Bei diesen Mengenangaben sind optionale Stabilisatoren wie Metalldesaktivatoren oder Lichtschutzmittel nicht eingerechnet. Die Menge an sekundärem Antioxidans beträgt bevorzugt bis 5 phr, besonders bevorzugt 0,5 bis 1 phr. Überraschend wurde gefunden, dass eine Kombination von primären Antioxidantien (zum Beispiel sterisch gehinderten Phenolen oder C-Radikalfängern wie CAS 181314-48-7) und sekundären Antioxidantien (zum Beispiel Schwefelverbindungen, Phosphiten oder sterisch gehinderten Aminen) eine verbesserte Verträglichkeit ergibt. Vor allem wird die Kombination von einem primären Antioxidans, vorzugsweise sterisch gehinderten Phenolen mit einer relativen Molmasse von mehr als 500 Dalton, mit einem sekundären Antioxidans aus der Klasse der Schwefelverbindungen oder aus der Klasse der Phosphite bevorzugt mit einer relativen Molmasse von mehr als 500 Dalton bevorzugt, wobei die phenolische, die schwefelhaltigen und die phosphitische Funktionen nicht in drei verschiedenen Molekülen vorliegen müssen, sondern auch mehr als eine Funktion in einem Molekül vereinigt sein können.

Die Herstellung und Verarbeitung der Haftklebemassen kann aus Lösung sowie aus der Schmelze erfolgen. Bevorzugte Herstell- und Verarbeitungsverfahren erfolgen aus der Schmelze. Für den letzteren Fall umfassen geeignete Herstellprozesse sowohl Batchverfahren als auch kontinuierliche Verfahren. Besonders bevorzugt ist die kontinuierliche Fertigung der Haftklebemasse mit Hilfe eines Extruders und anschließender Beschichtung direkt auf das zu beschichtende Substrat bei entsprechend hoher Temperatur der Klebmasse. Als Beschichtungsverfahren werden Extrusionsbeschichtung mit Breitschlitzdüsen und Kalanderbeschichtung bevorzugt.

Der Masseauftrag (Beschichtungsstärke) liegt vorzugsweise zwischen 30 und 120 g/m², besonders bevorzugt zwischen 50 und 70 g/m².

Als Trägermaterial können alle bekannten textilen Träger wie eine Schlingenware, ein Velour, ein Gelege, ein Gewebe, ein Gewirke, insbesondere ein PET-Filamentgewebe oder ein Polyamid-Gewebe, oder ein Vlies eingesetzt werden, wobei unter "Vlies" zumindest textile Flächengebilde gemäß EN 29092 (1988) sowie Nähwirkvliese und ähnliche Systeme zu verstehen sind.

Ebenfalls können Abstandsgewebe und -gewirke mit Kaschierung verwendet werden. Abstandsgewebe sind mattenförmige Schichtkörper mit einer Deckschicht aus einem Faser- oder Filamentvlies, einer Unterlagsschicht und zwischen diesen Schichten vorhandene einzelne oder Büschel von Haltefasern, die über die Fläche des Schichtkörpers verteilt durch die Partikelschicht hindurchgenadelt sind und die Deckschicht und die Unterlagsschicht untereinander verbinden. Die durch die Partikelschicht hindurchgenadelten Haltefasern halten die Deckschicht und die Unterlagsschicht in einem Abstand voneinander und sie sind mit der Deckschicht und der Unterlagsschicht verbunden.

Als Vliesstoffe kommen besonders verfestigte Stapelfaservliese, jedoch auch Filament-, Meltblown- sowie Spinnvliese in Frage, die meist zusätzlich zu verfestigen sind. Als mögliche Verfestigungsmethoden sind für Vliese die mechanische, die thermische sowie die chemische Verfestigung bekannt. Werden bei mechanischen Verfestigungen die Fasern meist durch Verwirbelung der Einzelfasern, durch Vermaschung von Faserbündeln oder durch Einnähen von zusätzlichen Fäden rein mechanisch zusammengehalten, so lassen sich durch thermische als auch durch chemische Verfahren adhäsive (mit Bindemittel) oder kohäsive (bindemittelfrei) Faser-Faser-Bindungen erzielen. Diese lassen sich bei geeigneter Rezeptierung und Prozessführung ausschließlich oder zumindest überwiegend auf Faserknotenpunkte beschränken, so dass unter Erhalt der lockeren, offenen Struktur im Vlies trotzdem ein stabiles, dreidimensionales Netzwerk gebildet wird.

Besonders vorteilhaft haben sich Vliese erwiesen, die insbesondere durch ein Übernähen mit separaten Fäden oder durch ein Vermaschen verfestigt sind.

Derartige verfestigte Vliese werden beispielsweise auf Nähwirkmaschinen des Typs "Malivlies" der Firma Karl Mayer, ehemals Malimo, hergestellt und sind unter anderem bei den Firmen Naue Fasertechnik und Techtex GmbH beziehbar. Ein Malivlies ist dadurch gekennzeichnet, dass ein Querfaservlies durch die Bildung von Maschen aus Fasern des Vlieses verfestigt wird.
Als Träger kann weiterhin ein Vlies vom Typ Kunitvlies oder Multiknitvlies verwendet werden. Ein Kunitvlies ist dadurch gekennzeichnet, dass es aus der Verarbeitung eines längsorientierten Faservlieses zu einem Flächengebilde hervorgeht, das auf einer Seite Maschen und auf der anderen Maschenstege oder Polfaser-Falten aufweist, aber weder Fäden noch vorgefertigte Flächengebilde besitzt. Auch ein derartiges Vlies wird beispielsweise auf Nähwirkmaschinen des Typs "Kunitvlies" der Firma Karl Mayer schon seit längerer Zeit hergestellt. Ein weiteres kennzeichnendes Merkmal dieses Vlieses besteht darin, dass es als Längsfaservlies in Längsrichtung hohe Zugkräfte aufnehmen kann. Ein Multiknitvlies ist gegenüber dem Kunitvlies dadurch gekennzeichnet, dass das Vlies durch das beidseitige Durchstechen mit Nadeln sowohl auf der Ober- als auch auf der Unterseite eine Verfestigung erfährt.

Schließlich sind auch Nähvliese als Vorprodukt geeignet, ein Klebeband zu bilden. Ein Nähvlies wird aus einem Vliesmaterial mit einer Vielzahl parallel zueinander verlaufender Nähte gebildet. Diese Nähte entstehen durch das Einnähen oder Nähwirken von durchgehenden textilen Fäden. Für diesen Typ Vlies sind Nähwirkmaschinen des Typs "Maliwatt" der Firma Karl Mayer, ehemals Malimo, bekannt.

Sodann ist das Caliweb® hervorragend geeignet. Das Caliweb® besteht aus einem thermisch fixierten Abstandsvliesstoff Multiknit mit zwei außenliegenden Maschenschichten und einer innenliegenden Polschicht, die senkrecht zu den Maschenschichten angeordnet sind.

Weiterhin besonders vorteilhaft ist ein Stapelfaservlies, das im ersten Schritt durch mechanische Bearbeitung vorverfestigt wird oder das ein Nassvlies ist, das hydrodynamisch gelegt wurde, wobei zwischen 2 Gew.-% und 50 Gew.-% der Fasern des Vlieses Schmelzfasern sind, insbesondere zwischen 5 Gew.-% und 40 Gew.-% der Fasern des Vlieses. Ein derartiges Vlies ist dadurch gekennzeichnet, dass die Fasern nass gelegt werden oder zum Beispiel ein Stapelfaservlies durch die Bildung von Maschen aus Fasern des Vlieses oder durch Nadelung, Vernähung beziehungsweise Luft- und/oder Wasserstrahlbearbeitung vorverfestigt wird. In einem zweiten Schritt erfolgt die Thermofixierung, wobei die Festigkeit des Vlieses durch das Auf- oder Anschmelzen der Schmelzfasern nochmals erhöht wird.

Die Verfestigung des Vliesträgers lässt sich auch ohne Bindemittel beispielsweise durch Heißprägen mit strukturierten Walzen erreichen, wobei über Druck, Temperatur, Verweilzeit und die Prägegeometrie Eigenschaften wie Festigkeit, Dicke, Dichte, Flexibilität u.ä. gesteuert werden können.

Als Ausgangsmaterialien für die textilen Träger sind insbesondere Polyester-, Polypropylen-, Viskose- oder Baumwollfasern vorgesehen. Die vorliegende Erfindung ist aber nicht auf die genannten Materialien beschränkt, sondern es können, für den Fachmann erkenntlich ohne erfinderisch tätig werden zu müssen, eine Vielzahl weiterer Fasern zur Herstellung des Vlieses eingesetzt werden. Insbesondere verschleißfeste Polymere wie Polyester, Polyolefine, Polyamide oder Glas- oder Carbonfasern finden Verwendung.

Als Trägermaterial eignet sich auch ein Träger aus einem Laminat, in dem zumindest die die Klebemasse tragende Schicht eine textile Schicht ist. Auf dieser Schicht können eine oder weitere Schichten aus beliebigem Material aufgebracht sein zum Beispiel Papier (gekreppt und/oder ungekreppt), Folie (zum Beispiel Polyethylen-, Polypropylen-, mono-oder biaxial orientierte Polypropylenfolien, Polyester-, PA-, PVC- und andere Folien), bahnförmige Schaumstoffe (beispielsweise aus Polyethylen und Polyurethan) sowie die genannten Textilien.

Auf der Streichseite (Beschichtungsseite) können die Oberflächen der Träger chemisch oder physikalisch vorbehandelt sein, sowie die Rückseite derselben einer antiadhäsiven physikalischen Behandlung oder Beschichtung unterzogen sein.

Das Klebeband wird dadurch gebildet, dass auf den textilen Träger partiell oder vollflächig vorzugsweise ein- oder gegebenenfalls beidseitig die Klebemasse aufgetragen wird. Die Beschichtung kann auch in Form eines oder mehrerer Streifen in Längsrichtung (Maschinenrichtung) erfolgen, gegebenenfalls in Querrichtung, sie ist insbesondere aber vollflächig. Weiterhin können die Klebemassen rasterpunktförmig mittels Siebdruck, wobei die Klebstoffpünktchen auch unterschiedlich groß und/oder unterschiedlich verteilt sein können, durch Tiefdruck in Längs- und Querrichtung zusammenhängenden Stegen, durch Rasterdruck oder durch Flexodruck aufgebracht werden. Die Klebemasse kann in Kalottenform (hergestellt durch Siebdruck) vorliegen oder auch in einem anderen Muster wie Gitter, Streifen, Zickzacklinien. Ferner kann sie beispielsweise auch aufgesprüht sein, was ein mehr oder weniger unregelmäßiges Auftragsbild ergibt.

Das Klebeband zeichnet sich dadurch aus dass es mit Drahtisolierungen auf PVC-Basis und auf Polyolefin-Basis verträglich ist, insbesondere bis zu 3000 Stunden bei 105 °C oder sogar auch bei 125 °C. In einem Fall ist es sogar gelungen, eine Verträglichkeit auf vernetztem PE unter T4-bedingungen (3000 Stunden bei 150 °C) zu erreichen.

### Beispiel D1

Die Klebemasse besteht aus folgenden Komponenten:

| | |
|---|---|
| 100 phr | IN FUSE 9107 |
| 78,4 phr | Ondina 933 |
| 212 phr | Escorez 1310 |
| 8 phr | Irganox 1726 |

Der Mischschmelzpunkt von Harz und Weichmacher beträgt 54 °C. Die Klebemasse wird in einem Extruder kontinuierlich hergestellt und mit 70 g/m² auf einem Polyestergewebe mittels Düsenbeschichtung aus der Schmelze aufgetragen. Das Filamentgewebe hat ein Flächengewicht von 130 g/m² aus Polyestergarn von 167 dtex mit 45 Fäden pro cm in Kettrichtung und 25 Fäden pro cm in Schussrichtung. Der beschichtete Ballen wird durch Schneiden in Rollen in 19 mm Breite und 10 m Lauflänge konfektioniert, der Kerninnendurchmesser beträgt 38 mm.

Klebkraft auf Stahl 6,6 N/cm
Klebkraft auf Rückseite 3,1 N/cm
Rollenlagerung 1 Monat bei 70 °C: Die Rolle ist leicht deformiert und gut abrollbar.
Verträglichkeitsprüfung: Das fertige Klebeband wird gemäß LV 312 um Drahtpaar mit unterschiedlichen Isolierungsmaterialien gewickelt und bei entsprechender Temperatur gelagert. Sechs solcher Prüflinge werden pro Isolierungsmaterial hergestellt. Alle 500 Stunden wird je eines der Muster kontrolliert, das Klebeband wieder abgewickelt und das Kabel um einen Dorn von 10 mm Durchmesser und um einen von 2 mm Durchmesser gewickelt. Es wird untersucht, ob die Isolierung beschädigt ist und ob die Klebemasse eine Haftklebrigkeit ausweist. Prüftemperaturen: PVC 105 °C und auf vernetztem PE bei 125 °C. Nach 3000 Stunden sind alle Drahtisolierungen noch unbeschädigt. Nach 3000 Stunden bei 105 °C ist die Masse kaum in den Träger eingedrungen und weist noch eine gute Haftklebrigkeit auf. Nach 3000 Stunden bei 125 °C ist die Masse teilweise in den Träger eingedrungen, ist aber noch haftklebrig.
Fogging-Wert: 36

### Beispiel D2

Klebemasse wie in Beispiel D1 jedoch mit Eastotac C 130 L statt Escorez 1310 und 5 phr Irganox 1076 und 3 phr Irganox PS 802 statt 8 phr Irganox 1726, Beschichtung wie in Beispiel D1 jedoch mit 60 g/m² auf folgendem Träger: Malivlies mit einem Flächengewicht von 150 g/m² bestehend aus Polyesterfasern einer Feinheit von 3,3 dtex und einer Faserlänge von 60 bis 80 mm und 5 Gew.-% eines thermisch aktivierten feinen Bindepulvers (VINNEX TM LL 2321). Der Mischschmelzpunkt von Harz und Weichmacher beträgt 90 °C.

Klebkraft auf Stahl 4,3 N/cm,
Klebkraft auf Rückseite 1,3 N/cm.
Rollenlagerung 1 Monat bei 70 °C: Die Rolle ist leicht deformiert und gut abrollbar. Verträglichkeitsprüfung auf PVC bei 105 °C und auf vernetztem PE und PP bei 125 °C: Nach 3000 Stunden sind alle Drahtisolierungen noch unbeschädigt. Nach 3000 Stunden bei 105 °C ist die Masse kaum in den Träger eingedrungen und weist noch eine gute Haftklebrigkeit auf. Nach 3000 Stunden bei 125 °C ist die Masse teilweise in den Träger eingedrungen, ist aber noch haftklebrig.

### Beispiel D3

Klebemasse wie in Beispiel D1 jedoch mit Eastotac C 115 L statt Escorez 1310, Beschichtung wie in Beispiel D1 mit 68 g/m² auf folgendem Träger: Maliwatt-Nähgewirke aus Polyesterfasern mit ca. 3,4 dtex und einer Faserlänge von ca. 80 mm, einem Flächengewicht von 72 g/m² und einer Feinheit F 22 mit einer Stichlänge von 1 mm eines Polyestergarns von 50 dtex. Der Mischschmelzpunkt von Harz und Weichmacher beträgt 75 °C

Klebkraft auf Stahl 4,2 N/cm,
Klebkraft auf Rückseite 1,6 N/cm.
Rollenlagerung 1 Monat bei 70 °C: Die Rolle ist leicht deformiert und gut abrollbar. Verträglichkeitsprüfung auf PVC bei 105 °C und auf vernetztem PE und PP bei 125 °C: Nach 3000 Stunden sind alle Drahtisolierungen noch unbeschädigt. Nach 3000 Stunden bei 105 °C ist die Masse kaum in den Träger eingedrungen und weist noch eine gute Haftklebrigkeit auf. Nach 3000 Stunden bei 125 °C ist die Masse teilweise in den Träger eingedrungen, ist aber noch haftklebrig.

### Beispiel D4

Klebemasse wie in Beispiel D1 jedoch mit Escorez 1102 statt Escorez 1310, Beschichtung wie in Beispiel D1 mit 70 g/m² auf folgendem Träger: Malivlies-Vlies aus Polypropylen mit einem Flächengewicht von 80 g/m² und einer Feinheit F 18. Der Mischschmelzpunkt von Harz und Weichmacher beträgt 60 °C.

Klebkraft auf Stahl 0,8 N/cm, Klebkraft auf Rückseite 0,2 N/cm.
Nach 4 Wochen Lagerung bei Raumtemperatur ist die Masse nicht mehr tackig. Rollenlagerung 1 Monat bei 70 °C: Die Rolle ist leicht deformiert und gut abrollbar. Verträglichkeitsprüfung auf PVC, vernetztem PE und PP bei 105 °C:
Nach 3000 Stunden bei 105 °C sind alle Drahtisolierungen noch unbeschädigt. Nach 3000 Stunden bei 105 °C ist die Masse kaum in den Träger eingedrungen und weist noch eine gute Haftklebrigkeit auf. Nach 3000 Stunden bei 125 °C ist der Vliesträger durch Versprödung zerfallen, daher können keine weiteren Prüfungen vorgenommen werden.

### Beispiel D5

Die Herstellung erfolgt wie in Beispiel D1, die Klebemasse besteht aus folgenden Komponenten:

| | |
|---|---|
| 100 phr | IN FUSE 9107 |
| 100 phr | Engage 7467 |
| 425 phr | Escorez 1310 |
| 16 phr | Irganox 1726 |

Klebkraft auf Stahl 5 N/cm, Klebkraft auf Rückseite 2,5 N/cm.
Rollenlagerung 1 Monat bei 70 °C: Die Rolle ist leicht deformiert und gut abrollbar. Verträglichkeitsprüfung: Prüftemperaturen: PVC 105 °C und auf vernetztem PE bei 125 und 150 °C. Nach 3000 Stunden sind alle Drahtisolierungen noch unbeschädigt. Nach 3000 Stunden bei 105 °C ist die Masse kaum in den Träger eingedrungen und weist noch eine gute Haftklebrigkeit auf. Nach 3000 Stunden bei 125 °C ist die Masse teilweise in den Träger eingedrungen, ist aber noch haftklebrig.
Fogging-Wert: 85.

### Beispiel D6

Die Herstellung erfolgt wie in Beispiel D1, die Klebemasse besteht aus folgenden Komponenten:

| | |
|---|---|
| 100 phr | IN FUSE 9507 |
| 250 phr | Regalite 1100 |
| 140 phr | Oppanol B 10 |
| 8 phr | Irganox 1726 |

Der Mischschmelzpunkt von Harz und Weichmacher beträgt 67 °C. Beschichtung erfolgt mit 70 g/m² auf einem Träger wie in Beispiel D3.

Klebkraft auf Stahl 8,9 N/cm, Klebkraft auf Rückseite 2,0 N/cm.
Verträglichkeitsprüfung: Prüftemperaturen: PVC 105 °C und auf vernetztem PE bei 125 und 150 °C. Nach 3000 Stunden sind alle Drahtisolierungen noch unbeschädigt. Nach 3000 Stunden bei 105 °C weist die Masse noch eine gute Haftklebrigkeit auf. Nach 3000 Stunden bei 125 °C ist die Masse noch etwas haftklebrig. Nach 3000 Stunden bei 150 °C ist die Masse deutlich abgebaut aber die Drahtisolierung ist noch unbeschädigt.
Fogging-Wert: 91.

### Beispiel D7

Die Herstellung erfolgt wie in Beispiel D1, die Klebemasse besteht aus folgenden Komponenten:

| | |
|---|---|
| 100 phr | IN FUSE 9107 |
| 212 phr | Foral 85 |
| 40 phr | TOTM |
| 8 phr | Irganox 1726 |

Der Mischschmelzpunkt von Harz und Weichmacher beträgt 67 °C. Beschichtung erfolgt mit 70 g/m² auf einem Träger wie in Beispiel D3.

Klebkraft auf Stahl 8,9 N/cm, Klebkraft auf Rückseite 2,0 N/cm.
Verträglichkeitsprüfung: Prüftemperaturen: PVC 105 °C und auf vernetztem PE bei 125 und 150 °C. Nach 3000 Stunden sind alle Drahtisolierungen noch unbeschädigt. Nach 3000 Stunden bei 105 °C weist die Masse noch eine gute Haftklebrigkeit auf. Nach 3000 Stunden bei 125 °C ist die Masse noch etwas haftklebrig. Nach 3000 Stunden bei 150 °C ist die Masse deutlich abgebaut aber die Drahtisolierung ist noch unbeschädigt.

### Vergleichsbeispiel D1

Die Ausführung erfolgt wie in Beispiel D1 beschrieben, jedoch besteht die Masse entsprechend marktüblichen Rezepturen aus

| | |
|---|---|
| 100 phr | Vector 4113 |
| 97 phr | Escorez 1310 |
| 21 phr | Ondina 933 |
| 1 phr | Irganox 1726 |

Rollenlagerung 1 Monat bei 70 °C: Die Rolle ist stark deformiert und sehr schwer abrollbar.
Verträglichkeitsprüfung: Die PVC- Isolierungen zeigen die ersten Risse nach 500 Stunden und die PE und PP-Isolierungen nach 1000 Stunden Lagerung bei 105 °C. Die Haftklebrigkeit ist nach 1000 Stunden verloren gegangen, die Masse ist vom Träger aufgesaugt worden und dort verlackt.

### Vergleichsbeispiel D2

Die Ausführung erfolgt wie in Beispiel D1 beschrieben, jedoch mit LD 251 statt IN FUSE 9107. Die Beschichtung ist nicht haftklebrig, sondern hart mit öliger Oberfläche.

### Vergleichsbeispiel D3

Die Ausführung erfolgt wie in Beispiel D1 beschrieben, jedoch mit Engage 7467 statt IN FUSE 9107. Die Beschichtung ist sehr weich und klebrig wie ein Fliegenfänger. Die Masse ist aufgrund der niedrigen Schmelzviskosität in den Träger eingedrungen. Der beschichtete Ballen konnte nicht zu Rollen aufgeschnitten werden, da bei Abrollen die Masse aufspaltet. Eine Klebkraftmessung ist aus diesem Grund ebenfalls nicht möglich (Kohäsionsbruch).

### Vergleichsbeispiel D4

Die Ausführung erfolgt wie in Beispiel D1, die Klebemasse besteht aus folgenden Komponenten:

| | |
|---|---|
| 100 phr | IN FUSE 9107, |
| 78,4 phr | PB 0300 M, |
| 212 phr | Escorez 5400, |
| 8 phr | Irganox 1076. |

Die Masse ist kaum haftklebrig

Das erfindungsgemäße Klebeband eignet sich des Weiteren ganz besonders vorteilhaft für Außenanwendungen, und zwar wenn gemäß einer weiteren vorteilhaften Ausführungsform der Erfindung das Klebeband einen textilen Träger mit einem Flächengewicht von 15 bis 150 g/m² aufweist, welcher auf der Oberseite mit einer Zusatzschicht versehen ist, die durch Extrusionsbeschichtung, durch Dispersionsbeschichtung oder durch Folienlaminierung aufgebracht ist, und welcher auf der Unterseite mit einer Klebemasse aus einem Ethylenpolymer mit einer Dichte zwischen 0,86 und 0,89 g/cm³ und einem Kristallitschmelzpunkt von mindestens 105 °C und aus einem Klebharz ausgerüstet ist.

Gewebeklebebänder, bestehend aus einem gewebten Textil als Trägermaterial und einer einseitig aufgebrachten Schicht einer Selbstklebemasse, gehören zu einer der ältesten Arten von selbstklebenden Systemen als Rollenware. Zuerst im medizinischen Bereich eingesetzt, lösten sie in der zweiten Hälfte des letzten Jahrhunderts teilweise Isolierbänder aus Weich-PVC bei der Bandagierung von Kabelsätzen in Automobilen ab. Wegen der außergewöhnlichen Eigenschaftskombination von Flexibilität und Anschmiegsamkeit, hoher mechanischer Festigkeit bei gleichzeitiger Quereinreißbarkeit per Hand erweiterte sich das Einsatzspektrum stark. Gewebeklebebänder können zum Bandagieren, Reparieren, Abdecken, Fixieren, Markieren etc. verwendet werden und lassen sich ohne Schere, Messer oder andere Hilfsmittel per Hand passend ablängen. Sie stellen deshalb Universal-Klebebänder (so genannte "Multi Purpose" oder "General Purpose" Tapes) dar, die auf einer Vielzahl von Untergründen, ob polar oder unpolar, rau oder glatt, kleben und für nahezu alle denkbaren Anwendungen genutzt werden.

Als Klebemasse wird fast ausschließlich auf Rezepturen aus Natur- oder Synthesekautschuk zurückgegriffen. Neben dem historischen Aspekt (Naturkautschuk als Hauptbestandteil der ersten industriell verfügbaren Selbstklebemassen) sind es insbesondere die klebtechnischen Eigenschaften, die hinsichtlich Adhäsion, Tack und Kohäsion ausbalanciert und für derartige Universalklebebänder bestens geeignet sind. Als Trägermaterialien werden dichte gewebte Textilien aus bevorzugt (modifizierten) Naturfasern wie Baumwolle, Zellwolle, Viskose etc. eingesetzt.

Anfänglich wurden Gewebeklebebänder aus unbeschichtetem Gewebe, als Rohgewebe oder aber im Garn gefärbt, nur einseitig mit Klebemasse beschichtet hergestellt. Durch die offene Gewebestruktur ist die Kautschukklebemasse auf der Rückseite jedoch leicht angreifbar: Sauerstoff, aggressive Substanzen wie Lösemittel, UV- oder Sonnenstrahlung etc. haben fast ungehinderten Zutritt.

Deshalb und auch zum Schutz des Gewebes selbst wurden Kunststoffbeschichtungen auf der Oberseite des Klebebandes aufgebracht. Dabei lassen sich drei Typen von Gewebeklebebändern anhand des Produktaufbaus unterscheiden:
- Die hochwertigsten Produkte nutzen ein dichtes Gewebe mit einem Flächengewicht von überwiegend 70 bis 150 g/m² bei einer Meshzahl (Summe der Fäden in Kett- und Schussrichtung, jeweils pro inch) in der Größenordnung von 100 bis 250 inch⁻² mit einer meist farbigen Kunststoffbeschichtung aus PVC, Acrylat, Polyurethan oder ähnlichem, die aus Dispersionen oder Organosolen einseitig aufgebracht wird. Diese Produkte haben ihren Ursprung in Mitteleuropa und werden überwiegend auch dort hergestellt. Als Beispiel für ein derartiges "Premium Tape" sei hier tesa® 4651 genannt.
- Eher asiatischen Ursprungs sind Gewebeklebebänder mit einem leichteren, offenen, netzartigen Gewebe von 40 bis 100 mesh, auf das eine 50 bis 200 µm dicke PE-Folie aufextrudiert wird. Gewebe und Folie bilden zumeist einen stabilen, belastbaren Verbund. Wegen ihrer Positionierung bezüglich Preises und der Eigenschaften werden sie auch als "Midgrades" bezeichnet. Als Beispiel kann hier tesa® 4688 gelten.
- Ursprünglich aus Nordamerika kommend haben sich die so genannten "Duct Tapes" global verbreitet. Hierbei kommen sehr offene Gewebe, Gelege oder Gewirke mit 25 bis 40 mesh mit einem Flächengewicht von 15 bis 40 g/m² zum Einsatz, auf die mit einem Teil der Selbstklebemasse eine meist farbige, undurchsichtige PE-Folie auflaminiert wird. Die Dauerhaftigkeit des Trägerverbunds aus Folie und Textil wird allein durch die Klebkraft und Alterungsstabilität der Klebemasse bestimmt. Diese Art von Gewebebändern stellt preislich die unterste Kategorie dar und wird meist in der Farbe Silber verwendet. Beispielhaft aus der Vielzahl der kommerziellen Duct Tapes kann hier tesa® 4662 genannt werden.

Derartige Klebebänder weisen im Regelfall eine Gesamtdicke von 200 bis 400 µm auf, wobei die Klebemasseschicht ca. 50 bis 250 µm beiträgt, und sind vom Aufbau her prinzipiell für Innenanwendungen konzipiert.

Als Universalklebebänder werden sie aber auch in Außenanwendungen eingesetzt. Durch die Einwirkung von Licht, direkter Sonneneinstrahlung, Feuchtigkeit, Wärme, Mikroorganismen etc. treten dann aber Schwächen zu Tage, die zu Schädigungen der Klebebänder bis hin zu deren vollständiger Zerstörung führen können:
- Kautschukklebemassen mit Doppelbindungen in den Elastomeren werden durch UV-Licht, Sauerstoff und Ozon angegriffen und geschädigt und verlieren dadurch ihre ursprünglichen Klebeigenschaften.
- Gewebe aus Baumwolle, Viskose, Zellwolle etc. werden durch Mikroorganismen angegriffen. Bei Anwesenheit von Feuchtigkeit, Wärme und Licht verrottet diese für die mechanischen Festigkeitseigenschaften des Klebebandes maßgebliche Komponente.
- Wasseraufnahme im Gewebe durch die Saugfähigkeit der Garne führt durch Aufquellen zur Verbundschwächung sowie zu Festigkeitsverlusten.

Bisherige Versuche, geeignete hochwertige Universal-Gewebeklebebänder für längerfristige Außenanwendungen als so genannte "Outdoor Tapes" zu entwickeln, sind bisher nicht oder nur mit Einschränkungen erfolgreich gewesen.

Ein hochwertiges, aber kostspieliges Gewebeklebeband mit einem dichtem 200 bis 250 mesh Viskoseactetat-Gewebe wird in US 3,853,598 A1 beschrieben. Das Gewebe ist mit einer Polyacrylat-Primerschicht versehen, auf die Klebemasse aus Synthese- und Naturkautschuk aufgebracht wird. Bedingt durch das Gewebe mit sehr hoher Meshzahl und die Gewebeausrüstung mit einem Polyacrylat-Primer weist das Klebeband gute und sehr leichte Handeinreißbarkeit auf. Hinweise auf Outdoor-Eignung finden sich jedoch nicht. Die gewählte Klebemasse und insbesondere das oberseitig ungeschützte Gewebe auf Basis von modifizierten Naturfasern sprechen auch dagegen. Explizit werden nur medizinische, das heißt Innenanwendungen genannt.

Ein technisches Klebeband, insbesondere für den Baubereich, wird in EP 1 548 080 A1 beschrieben. Zwar wird mit einer UV-vernetzten Acrylatklebemasse auf einem bandförmigen Träger eine witterungsstabile Klebemasse verwendet, aber die Trägerauswahl mit Papieren sowie Folien, Geweben oder Vliesstoffen aus PE, PP oder PET lässt eine Ausrichtung auf Outdoor-Anwendungen nicht erkennen. Eine leichte Quereinreißbarkeit per Hand, wie sie für ein General Purpose Tape zwingend ist, ist nicht gegeben. Des Weiteren ist bei UV-vernetzbaren Acrylatklebemassen das latente Risiko vorhanden, dass unter Einwirkung von Sonnenlicht eine während der Herstellung nicht vollständige eingestellte Vernetzung weiterläuft und sich damit die klebtechnischen Eigenschaften im Laufe der Verwendungszeit negativ verändern.

Ein spezielles Tape für langfristige Außenanwendungen, das nach 500 Stunden im Bewitterungstest nach ASTM G155 weniger als 10 % Klebemasserückstände aufweist, wird in WO 03/097758 A1 beschrieben. Wesentlich ist hierbei der mehrlagige PE-Kunststofffilm an der Oberseite, der bis zu 35 % Lichtschutzadditive enthält. Für die weiteren Klebebandkomponenten wie ein 10 bis 90 mesh Gelege (scrim) sowie die Selbstklebemasse werden keine besonderen Ausprägungen beschrieben. Deshalb ist davon auszugehen, dass großflächig durch den mehrlagigen Film an der Oberfläche ein Schutz gegen (UV-)Licht erreicht wird, aber Zugriff von Sauerstoff, Ozon etc. am Rand zu unerwünschten Veränderungen der Klebemasse an den Klebebandkanten führen kann. Außerdem ist bei dem beschriebenen Trägeraufbau aus einer 50 bis 100 µm dicken mehrlagigen PE-Folie und einem Gelege von 10 bis 90 mesh mit den für Duct Tapes typischen, ausgefransten Risskanten zu rechnen, die für ein hochwertiges Gewebeklebeband nicht akzeptiert werden. Des Weiteren lassen der hohe Anteil an Lichtschutzadditiven sowie die mehrlagige Folienstruktur entsprechend hohe Herstellkosten erwarten.

In EP 1 074 595 A1 wird ein Polyester-Gewebeband beschrieben, das durch die Auswahl von speziellen Garnen sowie definierter Gewebekonstruktion (maximal 2500 dtex/cm als Titer der Längsfäden pro Längeneinheit) sowie durch die als notwendig beschriebene Fixierung der Kettfäden durch die Kleberbeschichtung handeinreißbar wird. Somit müssen hier spezielle Bedingungen erfüllt sein, um zumindest eine Reißfestigkeit von weniger als 10 N in Querrichtung zu erzielen. Die Beschreibung der Garn- und Gewebeparameter weist für den Fachmann auf ein leichtes Gewebe merklich unter 100 g/m² hin, das nicht ganz überraschend per se allein durch die Reduzierung des Flächengewichtes bereits weniger Festigkeit besitzt, aber erst im Weiteren durch die Kieberschicht, die die Kettfäden an ihrem Platz fixieren muss, handeinreißbar wird. Des Weiteren wird hier fälschlicherweise eine Weiterreißfestigkeit von unter 10 N mit der Eigenschaft der Handeinreißbarkeit verknüpft. In der Praxis ist für eine einfache Handeinreißbarkeit aber neben der oben beschriebenen Weiterreißfestigkeit die Kraft zum initialen Einreißen des Trägers von hoher Wichtigkeit - diese wird aber maßgeblich durch weitere Parameter wie das Zug-Dehnungsverhalten des Trägers, die verwendete Schneidtechnologie und -güte etc. beeinflusst, über die in der Offenlegungsschrift keine Informationen zu finden sind.

In DE 10 2005 044 942 A1 wird ein quer einreißbares Klebeband mit einem unbeschichteten textilen Gewebeträger auf Basis von Polyester oder Polyamid beschrieben, wobei die Reduzierung der Faserfestigkeit und damit die Handeinreißbarkeit durch gezielte Schädigung des Garns (bei PET mit Alkalien, bei Polyamid mit Säuren) erfolgt. Durch zusätzliche Imprägnierung mit Schiebefestchemikalien wie Silikaten soll die Handeinreißbarkeit weiter verbessert werden. Die Alkalisierung von PET-Gewebe beispielsweise geht aber einher mit einem merklichen Festigkeitsverlust, der sich bei Alterung, thermischer Beanspruchung, Biege-und/oder Zugbelastungen negativ bemerkbar macht, und mit einer Erhöhung der Gas-und Dampfdurchlässigkeit. Letzterer bei medizinischen Anwendungen vorteilhafte Effekt kehrt sich bei technischen Anwendungen ins Gegenteil, da Sauerstoff, Ozon und vergleichbar aggressive Gase und Flüssigkeiten ungehindert bis zur Klebemasse durchdringen können und so diese stärker schädigen als bei unbehandelten oder gar beschichteten Geweben.

Obwohl es eine Vielzahl von Gewebeklebebändern sowohl im technischen, medizinischen und Consumer-Bereich gibt, ist ein handeinreißbares witterungsstabiles Universal-Gewebeband für längerfristige Außenanwendungen nicht bekannt.

Das erfindungsgemäße Klebeband mit dem Gewebeträger löst die gestellten Anforderungen.
- Es ist leicht handeinreißbar.
- Es klebt auf einer Vielzahl von im täglichen Gebrauch gängigen Untergründen, auch auf rauen und/oder verschmutzten Untergründen wie sägeraues Holz, Beton, Ziegel oder Putz.
- Auch bei längerer Anwendung von mindestens sechs Monaten im Außenbereich (Mitteleuropa) verliert es seine klebende Funktionalität nicht verliert.

Als Messlatte wird hierzu eine Abnahme der relevanten Messwerte um maximal 50 % angesetzt, beispielsweise für die Höchstzugkraft und Reißdehnung in Längsrichtung sowie die Klebkraft auf Stahl gemäß AFERA 5001. Auch Veränderungen der Optik wie deutlich erkennbare Zerstörungen oder Einrisse, merkliche Ver- oder Entfärbungen, Ablösungen von den Haftuntergründen sind erfindungsgemäß zu vermeiden.

Gemäß einer ersten vorteilhaften Ausführungsform der Erfindung weist das Klebeband einen textilen Träger aus einem sehr offenen Gewebe, Gelege oder Gewirke von 25 bis 40 mesh und mit einem Flächengewicht von 15 bis 40 g/m² auf. Auf der Oberseite ist eine UV-stabilisierte PE-Folie mit einer Dicke von 50 bis 200 µm vorhanden, die vorzugsweise durch Füllstoffe und Farbstoffpigmente nicht transparent und insbesondere UVundurchlässig ist. Durch vorteilhafte zusätzlich in der PE-Folie eingesetzte UV-Stabilisatoren und Alterungsschutzmittel sowie durch die UV-Undurchlässigkeit der PE-Folie wird die Klebemasse unterhalb des Trägers zusätzlich gegen photooxidative Angriffe geschützt.

Bei dieser Ausführungsform ist der eigentliche Träger ein Laminat, das aus dem Textil und der PE-Folie insbesondere in situ beim Klebemassenbeschichtungsprozess erzeugt wird. Ein geringer Teil der Klebemasse wird unter Druck durch das offene Textil durchgepresst und fungiert als Laminierkleber. Die Seite des textilen Trägers mit der geringen Menge an Klebemasse wird mit der PE-Folie kaschiert. Es entsteht so ein Verbund aus Folie, Kaschierkleber und Textil.
Bei Bedarf kann die PE-Folie auf der der Klebemasse abgewandten, offenen Seite vorher inline oder offline mit einem Release versehen werden, um ein leichtes Abrollen zu gewährleisten.
Prinzipiell führt die Vorab-Herstellung eines Laminates aus einer Folie, auf die der Laminierkleber aufgebracht und die anschließend mit dem Textil eingedeckt wird, bevor die Kleberbeschichtung auf der Gegenseite des Textils erfolgt, zu vergleichbaren Produkten.
Bei der hier beschriebenen Ausführungsform handelt es sich um einen Träger für die bereits oben beschriebene Kategorie der Duct Tapes wie beispielsweise tesa® 4662.

Gemäß einer weiteren vorteilhaften Ausführungsform der Erfindung besteht der textile Träger des Klebebands aus einem offenen, netzartigen Gewebe von 40 bis 100 mesh und mit einem Flächengewicht von 20 bis 60 g/m², auf das eine 50 bis 200 µm dicke PE-Folie aufextrudiert wird. Gewebe und Folie bilden zumeist einen stabilen, belastbaren Verbund. Eine geeignete UV-Stabilisierung kann auch hier wie bei den Duct Tapes über UV-Stabilisatoren, Alterungsschutzmittel und die Einfärbung erfolgen. Bei Bedarf kann auf der der Klebemasse abgewandten, offenen Seite der PE-Folie eine Releaseausrüstung aufgebracht werden.
Bei dieser Art von Träger handelt es sich um die bereits weiter oben beschriebene Kategorie der Midgrades wie beispielsweise tesa® 4688.

Gemäß einer weiteren vorteilhaften Ausführungsform der Erfindung besteht der textile Träger des Klebebands aus einem 80 bis 250 mesh PET-Gewebe mit einer Grammage von 50 bis 150 g/m², auf dessen Oberseite eine Dispersionspaste, insbesondere wässrige Acrylatpaste mit einem Auftragsgewicht von 15 bis 75 g/m² aufgebracht ist. Das Gewebe mit einer Grammage von 50 bis 150 g/m², insbesondere mit 70 bis 130 g/m², wird so ausgewählt, dass durch die besondere Konstruktion eine zumindest mäßige Ein- und Durchreißbarkeit per Hand in Querrichtung (auch als Schussrichtung oder CD bezeichnet) aufweist. Dieses Gewebe wird einseitig mit einer farbigen, wässrigen Acrylatpaste oder ähnlich mit einem Auftragsgewicht von 15 bis 75 g/m², insbesondere 25 bis 50 g/m², beschichtet.
Besonders vorteilhaft fällt der Gewebeträger aus, wenn die farbgebende Beschichtung in zwei Strichen nacheinander mit zwei unterschiedlichen Rezepturen aufgebracht wird. Der Hauptanteil wird als farbgebender Grundstrich mit 10 bis 60 g/m² direkt auf das Gewebe aufgetragen. Durch Verwendung eines Acrylatbindemittels mit einem Glasübergangspunkt von 0 °C und weniger wird eine weiche und elastische Beschichtung erzielt, die sich positiv auf die Flexibilität sowie den Griff des Trägers auswirkt und ein anschmiegsames Verkleben des Gewebeklebebandes fördert.
Auf diese manchmal etwas blockende (unter Druck klebende) Farbbeschichtung werden in einem zweiten Strich 5 bis 20 g/m² eines harten, widerstandsfähigen Decklack (Topcoat) aufgebracht. Damit erhöht sich die Widerstandsfähigkeit der Klebebandoberfläche sowohl gegen die eigene Klebemasse (direkter Kontakt während der Herstellung, Transport und bei der Lagerung als Klebebandrolle) sowie in der späteren Anwendung gegen alle möglichen Einflüsse wie mechanische Beanspruchungen, sichtbare, Infrarot- oder Ultraviolett-Strahlung, Wasser, Chemikalien etc.
Der so genannte Topcoat wird bevorzugt aus Acrylatdispersionen ausgewählt, in denen hart machende Comonomere wie beispielsweise Styrol, Methacrylat, Acrylnitril einpolymerisiert wurden.
Der erfindungsgemäße Träger als Verbundsystem aus einem PET-Gewebe und der bevorzugten Acrylatbeschichtung weist nicht nur sehr gute Beständigkeiten gegen unterschiedlichste Beanspruchungen auf, wie sie im Zusammenhang mit Außenanwendungen auftreten, sondern auch ein gegenüber dem Rohgewebe verbessertes Handling. Es stellen sich eine leichte Ein- und Durchreißbarkeit in Querrichtung ohne Verwendung von Schneidwerkzeugen sowie eine Flexibilität für konturenangepasste Verklebungen ein.
Mit diesem Verbundträger wird ein Gewebeklebeband der Premiumklasse erhalten, wie es beispielsweise tesa ®4651 darstellt.
Durch geschickte Wahl der Garne, der Gewebekonstruktion sowie der Prozessschritte ist es möglich, PET-Gewebe in dem angestrebten Grammagebereich von 50 bis 150 g/m², insbesondere 70 bis 130 g/m², bei Dicken von unter 100 bis 250 µm mit befriedigenden Handein- und weiterreißbarkeiten herzustellen. Mit dem in DE 10 2005 044 942 A1 beschriebenen Verfahren zur Schädigung des Garnes werden die Festigkeiten gezielt herabgesetzt, so dass sich ein ausgewogenes Verhältnis von verbleibender Höchstzugkraft in Kettrichtung und Quereinreißbarkeit einstellen lässt.
Alternativ dazu kann das Gewebe so konstruiert werden, dass die Kette, die im späteren Gewebe beim Querdurchreißen durchgetrennt werden muss, so gewählt wird, dass die einzelnen Kettfäden dies ohne unmäßigen Kraftaufwand erlauben.
Entweder wird der Fadenquerschnitt reduziert, so dass das Durchreißen ohne Probleme möglich ist, oder aber über die Materialauswahl für die Kette wird akzeptables Durchtrennverhalten eingestellt. Um genügend Gesamtfestigkeit in Kettrichtung (MD = machine direction) bei dem späteren Gewebe zu erreichen, ist die Anzahl der Fäden pro Längeneinheit so zu wählen, dass die gewünschte Höchstzugkraft MD von minimal 40 N/cm und maximal 100 N/cm erreicht wird. Idealerweise anzustreben ist für das Premium-Gewebeklebeband eine Höchstzugkraft MD von 60 bis 80 N/cm. Geeignet für die Kette ist beispielsweise PET-Garn von 75 den oder feinerem Titer, aber auch spröde Materialien, die bei impulsartigem Einbringen der Energie beim Reißvorgang ein Brechen des Kettfadens ergeben: PET-Fasern mit geeigneten Comonomeren oder Kristallisierung oder auch Kettgarn auf Basis von PA6,6. Um bei derartigen Ketten ein Gewebe mit angestrebter Haptik und Optik zu erhalten, müssen die Schussfäden entsprechend dicker und schwerer gewählt werden. Dadurch steigt zum einen das Flächengewicht in den Bereich von 70 g/m² und mehr, zum anderen werden die angestrebten Dicken für das Gewebe von 100 bis 250 µm erzielt; auch wirkt das Gewebe trotz der dünnen Kettfäden hochwertig, da die dickeren Schussfäden die Optik bestimmen. PET-Schussgarne ab 150 den sind möglich, besonders vorteilhaft hinsichtlich Optik und Haptik ist aber ein 300 den PET-Garn.
Analoges gilt für die Verwendung von anderen synthetischen Polymeren anstelle von PET als Werkstoff für das Garn wie beispielsweise andere Polyestertypen (PBT, PEN), Polyamid (PA), Polyacrylate, Polyimide, Polypropylen.

Eine weitere Möglichkeit, ein erfindungsgemäßes Grundgewebe mit einer akzeptablen Ein- und Durchreißbarkeit in Querrichtung zu erzeugen, ist, insbesondere für die Kette Garne aus einer Fasermischung zu verwenden, wobei zumindest eine dieser Faserarten löslich und damit nachträglich entfernbar ist. Somit würde ein Garn mit ausreichender Festigkeit für den Spinn- und Webprozess vorliegen und erst in einem nachgelagerten Prozessschritt eine Ausdünnung und Schwächung erfolgen, die die gewünschte Eigenschaft der Quereinreißbarkeit für das Gewebe zur Folge hat. Als Fasermischungen sind vielfältige Kombinationen denkbar, wobei sich die Verwendung von widerstandsfähigen Polymeren als permanentes Kettgarn wie beispielsweise PET-Fasern in Kombination mit wasserlöslichen beziehungsweise chemisch oder enzymatisch abbaubaren Materialien wie Polyvinylalkohol, Polylactate und ähnliches anbietet. Je nach gewählter Faserkombination sind die Mischungsanteile so zu wählen, dass die Endfestigkeit des (Kett-)Garns in dem Zielbereich zu liegen kommt.

Auch wenn mit derartigen, unbeschichteten Geweben Selbstklebebänder hergestellt werden können, so erfordert ein Premium-Universalgewebeband eine hochwertige einseitige Kunststoffbeschichtung, damit eine glatte, homogene Oberfläche erzielt und das Gewebe geschlossen wird, so dass aggressive Chemikalien von der Klebemasse und dem Verklebungsuntergrund ferngehalten werden. Des Weiteren erfolgt eine kostengünstige und flexible Farbgebung über diese Beschichtung, da ein Färben des Gewebes selbst aufwändiger ist. Überraschenderweise ergab sich neben diesen bekannten Aspekten, dass die erfindungsgemäße Farbbeschichtung die Handeinreißbarkeit des Rohgewebes bei geeigneten Rezepturen deutlich verbessert, so dass diesbezügliche Ansprüche an das Gewebe selbst reduziert werden können. Bei der einseitigen Beschichtung mit einer geeigneten Farbpaste auf der Oberseite dringt diese Beschichtung zumindest mit halber Gewebedicke in das Gewebe ein bedingt durch die dreidimensional strukturierte Oberfläche. Nach dem Trocken beziehungsweise Aushärten der Kunststoffschicht sind die Kett- und Schussfäden geometrisch fixiert, ähnlich wie es EP 1 074 595 A1 zwingend für die Kettfäden durch die Kleberbeschichtung fordert.

Für die Kunststoffbeschichtungen sind vom Grundsatz her eine Vielzahl von Systemen möglich wie Organosole, strahlenvernetzbare Präpolymersysteme, nicht-klebende Hotmelts, Polymerlösungen etc. Bevorzugt und etabliert sind dagegen wässrige Dispersionen aus Gründen der Kosten, Verfügbarkeit und vorhandenen Standardauftragstechnologien im Textilbereich.
Als Werkstoffe können beispielsweise Polyurethane, (Ethylen-)Vinylacetat-, PVC-, StyrolButadien- oder Acrylatsysteme gewählt werden. Aus Gründen der Ökologie, der Kosten, Verfügbarkeit und hinsichtlich der Anforderung "Außenanwendung/ Witterungsbeständigkeit' sind Acrylate zu bevorzugen. Diese werden je nach vorhandener Beschichtungstechnologie verdickt und mit entsprechenden Farbpasten/- pigmenten dispergiert, um die farbgebende einseitige Beschichtung zu erzeugen.

Als besonders vorteilhaft hat sich eine Zweistrich-Beschichtung erwiesen: Um einen guten "Griff" des finalen Gewebebandes zu erreichen, das heißt angenehmes Anfassen, anschmiegsames und flexibles Verhalten, so dass das Klebeband auch auf gewölbten, unebenen Flächen gut verklebt werden kann, sollte der farbgebende Grundstrich weich und flexibel sein; die Glasübergangstemperatur für das Bindemittel in der Farbpaste sollte unterhalb der Raumtemperatur liegen, insbesondere bei im Bereich von 0 °C oder tiefer.

Für eine gute Widerstandsfähigkeit des Klebebandes ist dagegen ein harter, chemisch resistenter Abschlusslack günstig. Ein derartiger Topcoat schützt nicht nur die Lagen darunter, sondern wirkt bei richtiger Auswahl auch als Barriereschicht gegen die Klebemasse, die bei der späteren Klebebandrolle in direktem Kontakt mit dem Topcoat liegt. Wechselwirkungen wie Migration von Bestandteilen des Klebers in die Kunststoffbeschichtung oder umgekehrt sind unerwünscht, da sie zu Veränderungen der jeweiligen Eigenschaften führen und im Extremfall die definierte Grenzfläche zwischen Klebemasse und Kunststoffoberfläche aufgelöst wird. Die Folge hiervon wäre ein starkes Aufziehen der Klebemasse und damit hohe Abrollkräfte. Topcoats, insbesondere auf Acrylatbasis, mit einer Glasübergangstemperatur oberhalb Raumtemperatur, insbesondere von 30 bis 50 °C und darüber, sind geeignet, ebenso chemisch oder thermisch vernetzende Systeme, wenn die finalen Filmeigenschaften in demselben Bereich liegen. Zu hart darf der Topcoat aber auch nicht ausfallen, damit bei Verklebungen um enge Radien durch Biegen oder Knicken im Topcoat keine Risse auftreten und damit die geschlossene Lackschicht geschädigt wird.

Die farbgebende Kunststoffbeschichtung ist mit 15 bis 75 g/m², insbesondere 20 bis 50 g/m², gesamthaft aufzubringen, um eine gute Farbgebung, geschlossene Schicht und eine gleichmäßige Oberflächenstruktur zu erreichen. Bei dem Zweistrichkonzept stellt der Grundstrich mit 50 bis 95 % den Hauptanteil dar. Aus Gründen geringer Komplexität hat es sich als günstig erwiesen, den Grundstrich pigmentiert mit 70 bis 95 % der Gesamtmenge als farbgebende Schicht aufzubringen und den Topcoat mit 5 bis 30 % als unpigmentierte, transparente Decklackierung. Hinsichtlich Rezeptierung und Prozessparametern ist darauf zu achten, dass einerseits die Haftung des Grundstrichs zu dem Rohgewebe hoch ist, andererseits aber auch die Verbundhaftung zwischen Grundstrich und Topcoat, damit es bei dem späteren Klebeband nicht zu Ausrissen oder Ablösen der farbgebenden Kunststoffschicht kommt, beispielsweise beim Abrollen von der Rolle.

Bei Bedarf, wenn beispielsweise eine leichte Abrollbarkeit von der Klebebandrolle erwünscht ist, können dem Decklack ein oder mehrere Releaseadditive zugemischt oder aber auf der der Klebemasse abgewandten, offenen Seite eine separate Releaselackierung/-bedruckung aufgebracht werden.

Das erfindungsgemäße Universal-Gewebeband mit Eignung für längerfristige Außenanwendungen ist gekennzeichnet durch folgenden Aufbau und Herstellung, wobei die Beschreibung als beispielhaft anzusehen ist und von einem Fachmann in modifizierter Form genutzt werden kann, ohne damit den Schutzrechtsbereich dieser Anmeldung zu verlassen. Auf den oben als vorteilhaft beschriebenen Trägern werden einseitig als Klebmasseschicht 50 bis 300 g/m², insbesondere 70 bis 150 g/m², der UV- und feuchtigkeitsbeständigen Selbstklebemasse aufgebracht, um eine sichere Verklebung bei Innen- und Außenanwendungen auf glatten, strukturierten sowie rauen Untergründen zu gewährleisten.

Die Klebemasse des hier beschriebenen Klebebands kann die offenbarten Antioxidantien enthalten, auch eine Kombination von primären Antioxidantien (zum Beispiel sterisch gehinderten Phenolen oder C-Radikalfängern wie CAS 181314-48-7) und sekundären Antioxidantien (zum Beispiel Schwefelverbindungen, Phosphiten oder sterisch gehinderten Aminen).

Aus Handhabungsgründen und wegen der hohen Klebkräfte ist es bei den erfindungsgemäßen Universal-Gewebebändern die Regel, die klebmassenabgewandte Oberseite des Trägers mit einem anti-adhäsiven Releasesystem zu versehen. Wie dem Fachmann bekannt ist, bieten Silikonsysteme die Option für eine leichte bis sehr leichte Abrollkraft, während Fettsäurederivate wie beispielsweise Polyvinylstearylcarbamat eher mittlere Abrollkräfte von einigen N/cm bewirken. Da sich bei Gewebebändern mittlere Abrollkräfte von 2 bis 8 N/cm etabliert haben, ist bevorzugt eine Oberflächenlackierung oder -bedruckung mit einem Releasemittel wie Polyvinylstearylcarbamat oder einem Umsetzungsprodukt aus Stearylisocyanat und Polyethylenimin zu wählen.

Mit diesem erfindungsgemäßen Produktaufbau werden Gewebeklebebänder erhalten, die auf den unterschiedlichen Haftuntergründen gut und sicher kleben.
Auf Stahl als Standardhaftuntergrund für polare Substrate wird eine Klebkraft im Frischzustand (maximal eine Woche nach Herstellung) von minimal 5 N/cm und auf Polyethylen als unpolares Substrat eine Klebkraft im Frischzustand von mindestens 4 N/cm erreicht, die wie gefordert zumindest zu 50 % über sechs Monate erhalten bleiben.
Aufgrund der hohen Klebkräfte und dem starken Aufziehen sowohl auf polaren als auch unpolaren Oberflächen haftet das erfindungsgemäße Klebeband bereits nach kurze Zeit so stark, dass es danach und verständlicherweise insbesondere nach bis zu sechsmonatiger Verwendung nicht mehr rückstandsfrei zu entfernen ist. Die Haftung und sonstige Funktionalität als Selbstklebeband wird jedoch nicht oder nur gering beeinflusst. Aus diesem Grunde bieten sich derartige Klebebänder insbesondere für längerfristige Permanentverklebungen im Außenbereich an.
Erfindungsgemäße Universal-Gewebebänder lassen sich leicht und mit einer geraden Risskante ohne Ausfransen per Hand in Querrichtung ablängen. In Maschinenrichtung weist das Gewebeband dagegen ausreichende Festigkeiten auf und kann somit für viele Bandagierungs- und Fixieranwendungen eingesetzt werden, bei denen es auf Zugfestigkeit ankommt. Meist wird eine leicht erhöhe Initialkraft zum Einreißen der Kante benötigt, wobei das weitere Durchreißen dann leicht und gleichmäßig erfolgen kann. Diese leicht erhöhte Einreißkraft schützt vorteilhaft das Gewebeband vor unbeabsichtigtem Durchreißen beim Handling sowie in der endgültigen Anwendung.

In Außenanwendungen erweist sich das erfindungsgemäße Universal-Gewebeklebeband als äußerst stabil und für Daueranwendungen von mindestens sechs Monaten geeignet. Während insbesondere die bisher bekannten Duct Tapes durchweg nach wenigen Wochen bei direkter Einwirkung von Sonnenlicht und Regen in ihre Bestandteile zerfallen, bleibt bei dem erfindungsgemäßen Gewebeband die Funktionalität und Produktintegrität erhalten:
- ausreichende Festigkeit für mechanische Beanspruchungen,
- Erhalt der Integrität des mehrlagigen Aufbaus des Klebebandes,
- gute dauerhafte Haftung auf dem Substrat.
Dieses ist bei den bekannten Naturkautschuk- und Synthesekauschuk-Gewebebändern nicht gegeben, da über den Angriff an den Doppelbindungen das Gerüstelastomer zerstört wird und teilweise auch die Trägerkomponente merklich irreversibel geschädigt wird.

Neben der Eignung für Außenanwendungen, bei denen bisher bekannte Gewebebänder deutliche Schwächen aufweisen, sind die erfindungsgemäßen Klebebänder als Universalklebebänder selbstverständlich auch für Innenanwendungen geeignet, was für den Fachmann nicht gesondert erwähnt werden muss.

Nahezu unabhängig von der Art der verwendeten Klebemasse benötigt ein erfindungsgemäßes Universal-Gewebeband eine gewisse Schichtdicke für die Klebemasse, um auch auf rauen oder strukturierten Haftuntergründen wie Holz, Stein, Beton etc. sicher zu kleben. Bei einem Masseauftrag von 50 bis 300 g/m², insbesondere 70 bis 150 g/m², wird das angestrebte Klebverhalten erzielt; die absolute Menge der klebtechnisch wirksamen Schichtdicke hängt unter anderem auch von der Struktur des Gewebes ab. Je nach Rauhigkeit der zu beschichtenden Seite werden Mengen bis zu 50 g/m² benötigt, allein um die Vertiefungen im Gewebe auszufüllen, ohne dass dieser Teil der Klebemasse über die "Gipfel des Gewebegebirges" hinausragt und für Verklebungen zur Verfügung steht. Als grobe Orientierung für den Massebedarf bei dem angestrebten Klebverhalten ist eine "wirksame" Schichtdicke von 50 bis 150 µm zu nennen.

Auf der Streichseite (Beschichtungsseite) können die Oberflächen der Träger haftfreundlich ausgerüstet werden, beispielsweise über einen Verankerungsstrich oder physikalisch vorbehandelt wie beispielsweise mittels Coronabestrahlung. Im Normalfall bietet aber die raue Struktur des Gewebes sowie die Affinität der Oberfläche der Klebemasse ausreichende Verankerung, so dass auf separate Prozessschritte verzichtet werden kann.

In Abhängigkeit von der Rezeptur und Viskosität der Klebemasse ist die Beschichtungstechnologie zu wählen. Hier kann auf bekannte Systeme wie Rakel, Walzen, Düsen etc. zurückgegriffen werden. Die geeignete Auswahl kann durch einen Fachmann ohne Probleme vorgenommen werden. Während in vielen Fällen die Kombination Klebemasse/Beschichtungstechnologie zu einem ausreichenden Eindringen der Klebemasse in die Vertiefungen des Gewebes und damit für eine gute Verankerung der Kleberschicht auf dem Träger führt, muss in Fällen, in denen eine Klebmasseschicht als Film beispielsweise aus der Düse ausgezogen und nur aufgelegt wird, durch zusätzlichen Einsatz von Druck und Temperatur für eine intensivere und dauerhafte Kontaktbildung zwischen den beiden Schichten gesorgt werden. Dies kann durch einen nachgelagerten Druck- und Anpressvorgang wie beispielsweise eine Kalanderstation erreicht werden. Alternativ lässt sich dies aber auch durch eine Trägervorbehandlung wie beispielsweise durch einen zusätzlichen Primerstrich erreichen, die die Haftung und Verankerung der Klebemasse auf dem Träger physikalisch/chemisch verstärkt.

### Beispiel E1

Ein schwarzes PET-Gewebe mit Leinwandbindung, einer Fadenzahl von 31 cm⁻¹ in der Kette, 22 cm⁻¹ im Schuss, mit 75 den-Garn in der Kette sowie 300 den-Garn im Schuss, weist nach der kontinuierlichen Alkalisierung gemäß DE 10 2005 044 942 A1 bei einem Flächengewicht von 100 g/m² eine Höchstzugkraft in Kettrichtung von 70 N/cm auf. Einseitig wird eine schwarz pigmentierte Acrylatdispersion mit einem Auftrag von 35 g/m² beschichtet. Die aufgrund ihres niedrigen T_{G}-Werts weiche und zum Blocken neigende Beschichtung wird sofort anschließend mit einem transparenten Topcoat auf Basis einer harten Acrylatdispersion mit einem Auftrag von 10 g/m² abdeckend beschichtet und so getrocknet, dass der selbstvernetzende Topcoat ausgehärtet ist.
Die Weiterreißbarkeit und insbesondere die Einreißbarkeit in Schussrichtung von der Kante her werden durch diese Beschichtung deutlich verbessert. Das Trägermaterial ist "handeinreißbar".

Die Klebemasse wird in einem Extruder kontinuierlich hergestellt und mit 80 g/m² auf den Träger mittels Düsenbeschichtung aus der Schmelze aufgetragen. Anstelle der Releaselackierung wird die Klebemasse für die Herstellung und Untersuchung von Labormustern mit silikonisiertem Trennpapier eingedeckt.

### Klebemasse mit folgender Rezeptur:

| | |
|---|---|
| 100 phr | IN FUSE 9107, |
| 78,4 phr | Ondina 933, |
| 212 phr | Foral 85 |
| 2 phr | Irganox 1076 |
| 5 phr | Tinuvin 111. |

Die Klebkraft auf Stahl beträgt 9,8 N/cm. Von PE lässt sich nach ein- bis zweistündigem Aufziehen das Gewebeband nur noch unter Umspulen von Teilmengen der Klebemasse abziehen.
In dem UV-Test nach 7d sowie im SunTest nach 2 Wochen sind geringfügige visuelle Veränderungen zu erkennen, aber das verklebte Klebeband weist kaum Hinweise auf Zersetzungen und Ablösungen auf und klebt weiterhin sicher und fest.

### Beispiel E2

Als Träger wird ein PE-extrudiertes Gewebe gewählt. Der Träger wird fertig ausgerüstet mit Polyvinylstearylcarbamat-Lackierung aus Japan bezogen. Es handelt sich um einen 0,18 mm dicken Verbundträger aus einem 55 mesh VIS/PET-Mischgewebe (30 x 25 inch⁻²) mit einer damit fest verbundenen 65 g/m² schwarz eingefärbten LDPE-Beschichtung.

Die Klebemassenherstellung und -beschichtung erfolgt analog Beispiel E1 mit folgender Rezeptur:

| | |
|---|---|
| 100 phr | IN FUSE 9507, |
| 140 phr | Oppanol B10, |
| 250 phr | Regalite R 1100 |
| 2 phr | Irganox 1076 |
| 5 phr | Tinuvin 111. |

Die Klebkraft auf Stahl beträgt 9,0 N/cm bei einem Masseauftrag von 70 g/m². Aufziehverhalten, UV- und Bewitterungsteste werden wie Beispiel E1 beschrieben geprüft und durchgeführt, wobei tendenziell eine leichte Schädigung des Trägers zu erkennen ist. Hier ist eine etwas stärkere UV-Stabilisierung der PE-Folie sinnvoll und für den Fachmann ohne Probleme umzusetzen. Die Klebemasse selbst weist keine Hinweise auf Schädigungen auf.

### Gegenbeispiel E1

Gegenbeispiel E1 entspricht einem kommerziellen Gewebeband aus Zellwolle mit einer Standard-Naturkautschukklebemasse.
Ein 150 mesh Zellwollgewebe (ca. 110 g/m² Rohgewebe; symmetrische Leinwandbindung mit Nm 50-Garnen in Kette und Schuss) mit oberseitiger, pigmentierter Acrylatbeschichtung (60 g/m²) und rückseitiger Naturkautschukbeschichtung (110 g/m²; keine spezielle UV-Stabilisierung) lässt sich leicht einreißen, klebt gut auf unterschiedlichen Untergründen, weist aber in den UV- und Bewitterungstesten schwerwiegende Mängel bereits nach kurzer Expositionsdauer auf. Insbesondere im Suntester sind deutliche Ablösungserscheinungen vom Haftuntergrund und Zersetzungen der Klebemasse zu erkennen. Da aufgrund seiner Zusammensetzung das Klebeband auch von Mikroorganismen leicht angegriffen und zerstört wird, ist es für Außenanwendung denkbar ungeeignet.

### Gegenbeispiel E2

Gegenbeispiel E2 entspricht einem kommerziellen Duct Tape mit einer Standard-Naturkautschukklebemasse.
Ein 30 mesh PET/VIS-Gewirke (20x10 inch⁻²) und eine silberne 50 µm PE-Folie stellen die Trägerkomponenten dar, die mit insgesamt 160 g/m² einer sehr weich und tackig eingestellten Naturkautschuk-Klebemasse ausgerüstet werden, wobei ca. 5 bis 10 g/m² als Laminierkleber fungieren.
Das Duct Tape klebt gut auf unterschiedlichen Haftuntergründen (zum Beispiel auf Stahl 5 N/cm, auf PE 2,5 N/cm); nach 1 bis 2 Wochen Freibewitterung treten erste massive Zersetzungserscheinungen, nach 2 Monaten ist der Träger nahezu vollständig delaminiert und die Klebemasse verlackt, das heißt keine Selbstklebeeigenschaften mehr. In den UV- und Bewitterungstesten treten diese Effekte entsprechend früher bereits nach kurzen Prüfintervallen auf, erst starke Faltenbildung, dann partielles Ablösen der PE-Folie vom Gewirke bzw. nur noch geringe Verbundfestigkeit.
Derartige Duct Tapes sind für länger andauernde Außenanwendungen ungeeignet.

Das erfindungsgemäße Klebeband eignet sich des Weiteren ganz besonders vorteilhaft auf rauen oder verschmutzten Untergründen in der Bauindustrie, und zwar wenn gemäß einer weiteren vorteilhaften Ausführungsform der Erfindung das Klebeband einen Träger und eine darauf zumindest einseitig aus der Schmelze beschichtete Klebemasse aus einem Ethylenpolymer mit einer Dichte zwischen 0,86 und 0,89 g/cm³ und einem Kristallitschmelzpunkt von mindestens 105 °C und einem Klebharz aufweist.

Beim Hausbau finden Klebebänder vielfältige Anwendungen, zum Beispiel als Dichtungsband für Fugen, als Putzband oder als Montageband zum Verkleben von Winddichtungen, Dampfbremsen und Dampfsperren.
Dichtungsbänder für Fugen haben den Zweck, die Fugen umgehend luftdicht und optimal abzudichten. Diese Dichtungsbänder sind bevorzugt als selbstklebende Bänder ausgebildet und werden nach dem Zusammenfügen der Bauelemente auf der Wandinnenseite fugenüberdeckend auf die Fugenränder aufgeklebt. Putzband wird als Außenabdeckung zum Schutz von Profilen, Türzargen, Fensterrahmen und -bänken verwendet. Besonders geeignet ist es beim Auftragen und Abreiben von Putzen. Das Klebeband schützt empfindliche Untergründe, auch Edelstahl und eloxierte Metalle, vor mechanischer Belastung und Verschmutzung. Montagebänder für Winddichtungen, Dampfbremsen und Dampfsperren werden beim Innenausbau von Häusern nach dem Befestigen von wärmedämmenden Materialien and Wänden, Dachflächen und ähnlichem verwendet, um die in Form von Folien vorliegenden Winddichtungen, Dampfbremsen und Dampfsperren zu verkleben. Zum Befestigen auf verschiedensten Untergründen sowie zum Verkleben der entstehenden Überlappungsstellen der entsprechenden Dampfbremsen, Dampfsperren und Winddichtungen werden einseitig oder doppelseitig klebende Montagebänder eingesetzt.

An alle im Baubereich verwendeten Klebebänder werden hohe Anforderungen im Bezug auf ihre Resistenz gegenüber Chemikalien und Wasser, Klebefähigkeit, insbesondere auch bei Temperaturen bis 0 °C, Alterungsbeständigkeit und Abdichtungsfähigkeit gestellt. Gemeinsam ist allen Anwendungen, dass die Verklebung auf verschmutzten und/oder rauen Untergründen wie zum Beispiel Betonoberflächen oder Holzsparren sicher haften muss. Anforderungen, die vor allem Montagebänder zum Verkleben von Winddichtungen betreffen, sind Alterungsbeständigkeit und gute Klebeeigenschaften auf PE-Folie.

Die DE 103 12 13 A1 beschreibt ein Dichtungsband für Fugen mit einer Klebemasse auf Basis Acrylat. Als besondere Anforderungen werden die Verarbeitbarkeit bei tiefen Temperaturen sowie Alterungsbeständigkeit genannt.

Im Handel erhältliche Putzbänder enthalten meist eine Klebemasse aus Kautschuk. Da diese Klebebänder häufig zur Befestigung von unpolaren Schutzfolien eingesetzt werden, bieten unpolare Klebemassen hier Vorteile. Kautschuk-Klebemassen sind allerdings im Bezug auf ihre Alterungsbeständigkeit eingeschränkt. Sie sollten daher im Außenbereich nach spätestens sechs Wochen wieder entfernt werden.

Ein einseitig klebendes Montageband für die Verklebung von Winddichtungen, Dampfbremsen und Dampfsperren wird in der DE 297 23 454 U1 beschrieben. Das Montageband besteht aus einer Folie und einer Acrylat-Klebemasse mit einem hohen Masseauftrag von mehr als 80 g/m². In der Praxis werden Klebebänder mit Masseaufträgen von ca. 200 g/m² angeboten. Da diese hohen Masseaufträge nach Trocknen einer Lösung einer Acrylat-Klebemassen erhalten werden müssen, ist die Herstellung eines solchen Klebebandes sehr zeitaufwändig und damit teuer. Für Verklebung von Winddichtungen wird vom Hersteller zudem oft eine Alterungsbeständigkeit von mindestens fünf Jahren garantiert, daher ist der Einsatz einer alterungsbeständigen Klebemasse sehr wichtig. Die Verwendung einer lösungsmittelfrei herstellbaren Kautschuk-Klebemasse kommt aufgrund dessen nicht in Frage.

Dies Klebeband ist lösungsmittelfrei herstellbar und alterungsstabil und kann auf rauen oder verschmutzten Untergründen in der Bauindustrie eingesetzt werden.

Bei Nichtverwendung eines Weichmachers weist das Klebharz vorzugsweise einen Schmelzpunkt von unter 90 °C auf.

Als Klebharze haben sich Harze auf Basis von Kolophonium (zum Beispiel Balsamharz) oder Kolophoniumderivaten (zum Beispiel disproportioniertes, dimerisiertes oder verestertes Kolophonium), vorzugsweise partiell oder vollständig hydriert, als gut geeignet erwiesen.

Es werden vorzugsweise ein primäres Antioxidans und besonders bevorzugt auch ein sekundäres Antioxidans in den genannten Mengen verwendet.

Die Herstellung und Verarbeitung der Haftklebemassen kann aus Lösung sowie aus der Schmelze erfolgen. Der Vorteil der Verarbeitung der Haftklebemasse aus der Schmelze liegt in der Möglichkeit, sehr hohe Schichtdicken (Masseaufträge) in sehr kurzer Zeit erreichen zu können, da nach der Beschichtung kein Lösungsmittel entfernt werden muss. Bevorzugte Herstell- und Verarbeitungsverfahren erfolgen daher aus der Schmelze. Für den letzteren Fall umfassen geeignete Herstellprozesse sowohl Batchverfahren als auch kontinuierliche Verfahren. Besonders bevorzugt ist die kontinuierliche Fertigung der Haftklebemasse mit Hilfe eines Extruders und anschließender Beschichtung direkt auf das zu beschichtende Substrat oder ein Trennpapier beziehungsweise eine Trennfolie bei entsprechend hoher Temperatur der Klebmasse. Als Beschichtungsverfahren werden Extrusionsbeschichtung mit Breitschlitzdüsen und Kalanderbeschichtung bevorzugt.

Der Masseauftrag (Beschichtungsstärke) liegt je nach Anwendung zwischen 10 und 300 g/m², besonders bevorzugt zwischen 20 und 250 g/m². Für Anwendungen als Putzband liegt der Masseauftrag eher im unteren Bereich dieser Werte; Dichtungsbänder für Fugen und Montagebänder für Winddichtungen, Dampfbremsen und Dampfsperren weisen in der Regel Masseaufträge zwischen 50 und 250 g/m² auf.

Als Trägermaterial können Kunststoff-Folien wie zum Beispiel Folien aus Polyolefin wie Polyethylen, Polypropylen, Polybuten, deren Copolymeren, Blends dieser Polymeren zum Beispiel mit Polyethylenvinylacetat oder lonomeren sowie Folien aus Polyvinylchlorid oder Polyester eingesetzt werden. Dehnbare Folien können durch eine Armierung, vorzugsweise ein Fadengelege, verstärkt werden. Weiterhin ist der Einsatz von Papier-Kunststoff-Verbünden, die zum Beispiel durch Extrusionsbeschichtung oder Laminierung erhalten werden, möglich. Textilmaterialien können je nach Anwendung offenporig oder als Textil-Kunststoff-Verbund als Trägermaterial verwendet werden. Die verwendeten Kunststoffe können Flammschutzmittel wie zum Beispiel Antimontrioxid oder bromhaltige Flammschutzmittel wie zum Beispiel Saytex® 8010 enthalten. Das Trägermaterial kann Dicken zwischen 30 und 150 µm aufweisen, bevorzugt zwischen 50 und 100 µm.

Auf der Streichseite (Beschichtungsseite) können die Oberflächen der Träger chemisch oder physikalisch (zum Beispiel Corona) vorbehandelt sein, sowie die Rückseite derselben einer antiadhäsiven physikalischen Behandlung oder Beschichtung unterzogen sein.

Für die Verwendung als Haftklebeband können die ein- oder doppelseitigen Haftklebebänder mit einem oder zwei Trennfolien oder Trennpapieren abgedeckt sein. In einer bevorzugten Auslegung werden silikonisierte oder fluorierte Folien oder Papiere, wie zum Beispiel Glassine, HPDE oder LDPE gecoatete Papiere eingesetzt, die wiederum mit einer Releaseschicht basierend auf Silikonen oder fluorierten Polymeren versehen sind.

Diese Ausführungsform des Klebebands ist für den Einsatz als alterungsstabiles Klebeband insbesondere für die Verklebung auf rauen Untergründen wie Beton, Putz, Stein oder Holz und auf unpolaren Oberflächen wie Polyethylen-Folie geeignet. Es kann zum Beispiel als Dichtungsband für Fugen, als Putzband oder als ein- oder beidseitig klebendes Montageband für Winddichtungen, Dampfbremsen oder Dampfsperren eingesetzt werden. Bevorzugt ist aufgrund der Alterungsbeständigkeit der Klebemasse der Einsatz als Montageband für Winddichtungen, Dampfbremsen oder Dampfsperren.

### Beispiel F1

Die Klebemasse besteht aus folgenden Komponenten:

| | |
|---|---|
| 100 phr | IN FUSE 9107, |
| 78,4 phr | Ondina 933, |
| 212 phr | PRO 10394, |
| 2 phr | Irganox 1726. |

Die Klebemasse wird in einem Extruder kontinuierlich hergestellt und mit 30 g/m² auf den Träger mittels Düsenbeschichtung aus der Schmelze aufgetragen. Der Träger ist eine Folie aus 100 Gewichtsteilen PVC (K-Wert 65), 45 Gewichtsteilen Polymerweichmacher (Palamoll 652), 15 Gewichtsteilen Füllstoff (Kreide), 0,2 Gewichtsteilen Gleitmittel (Stearinsäure), 5 Gewichtsteilen Pigment (Titandioxid) und 3 Gewichtsteilen Stabilisator (Typ Calcium-Zink) mit einer Rückseitenbeschichtung aus einem Silikon-PMMA-Copolymer.

Die Klebkraft auf Stahl beträgt 8,1 N/cm. Das Klebeband lässt sich auch bei 10 °C auf Mauerwerk verkleben.

### Beispiel F2

Klebemasse wie in Beispiel F1 jedoch mit folgender Rezeptur:

| | |
|---|---|
| 100 phr | IN FUSE 9507, |
| 78,4 phr | Ondina 933, |
| 212 phr | Escorez 1310, |
| 2 phr | Irganox 1076. |

Die Klebemasse wird in einem Extruder kontinuierlich hergestellt und mit 200 g/m² auf ein Trennpapier mittels Düsenbeschichtung aus der Schmelze aufgetragen. Die Trägerfolie besitzt eine Dicke von 70 µm und besteht aus 91,3 % (w/w) aus Blockcopolymer Novolen 2309 L (BASF, Schmelzindex 6 g/10 min bei 230 °C und 2,16 kg, Ethylengehalt ca. 6,5 % (w/w)), 8,4 % (w/w) Titandioxid und 0,3 % (w/w) des HALS-Stabilisators Tinuvin 770. Sie wird vor der Beschichtung einseitig Corona-behandelt. Der Klebmassenauftrag erfolgt auf die Corona-behandelte Seite des Trägermaterials durch Kaschieren von beschichtetem Trennpapier. Das Klebeband wird, ohne das Trennpapier wieder auszudecken, zu Stangen gewickelt.

Die Klebkraft auf Stahl beträgt 14,2 N/cm. Die Klebkraft auf Polyethylen beträgt 7,9 N/cm. Nach Alterung beträgt die Klebkraft auf Polyethylen noch 90 % der ursprünglichen Klebkraft. Das Klebeband lässt sich auch bei 0 °C auf Mauerwerk, sägerauem Holz, Polyethylen-Folie oder Polyamid-Folie verkleben.

### Beispiel F3

Klebemasse wie in Beispiel F1 jedoch mit folgender Rezeptur:

| | |
|---|---|
| 100 phr | IN FUSE 9107, |
| 78,4 phr | Ondina 933, |
| 212 phr | Foral 85, |
| 2 phr | Irganox 1076 |
| 5 phr | Tinuvin 111. |

Die Klebemasse wird wie in Beispiel F2 beschichtet. Das Klebeband wird analog hergestellt, allerdings werden beide Seiten des Trägers Corona-behandelt und mit der Klebemasse beschichtet. Nach der zweiten Transferbeschichtung wird das zweite Trennpapier ausgedeckt und das Klebeband zu Stangen gewickelt.

Die Klebkraft auf Stahl beträgt 16,9 N/cm. Die Klebkraft auf Polyethylen beträgt 10,5 N/cm. Nach Alterung beträgt die Klebkraft auf Polyethylen noch 97 % der ursprünglichen Klebkraft. Das Klebeband lässt sich auch bei 0 °C auf Mauerwerk, sägerauem Holz, Polyethylen-Folie oder Polyamid-Folie verkleben.

### Beispiel F4

Klebemasse wie in Beispiel F1 jedoch mit folgender Rezeptur:

| | |
|---|---|
| 100 phr | IN FUSE 9107, |
| 78,4 phr | Ondina 933, |
| 212 phr | Regalite R1100 |
| 2 phr | Irganox 1076. |

Die Klebemasse wird wie in Beispiel F2 beschichtet und, ohne das Trennpapier auszudecken, zu Stangen gewickelt. Die Anwendung erfolgt als trägerloses doppelseitig klebendes Transfertape zum Beispiel zum Befestigen von Winddichtungen, Dampfbremsen und Dampfsperren auf sägerauem Holz.

Die Klebkraft auf Stahl beträgt 15,0 N/cm. Die Klebkraft auf Polyethylen beträgt 8,1 N/cm. Nach Alterung beträgt die Klebkraft auf Polyethylen noch 96 % der ursprünglichen Klebkraft. Das Klebeband lässt sich auch bei 0 °C auf Mauerwerk, sägerauem Holz, Polyethylen-Folie oder Polyamid-Folie verkleben.

### Beispiel F5

Klebemasse wie in Beispiel F1 jedoch mit folgender Rezeptur:

| | |
|---|---|
| 100 phr | IN FUSE 9107, |
| 78,4 phr | Ondina 933, |
| 212 phr | Regalite R1100 |
| 2 phr | Irganox 1076. |

Die Klebemasse wird wie in Beispiel F2, allerdings mit einem Masseauftrag von lediglich 70 g/m², beschichtet. Das Klebeband wird, ohne das Trennpapier wieder auszudecken, zu Stangen gewickelt.

Die Klebkraft auf Stahl beträgt 9,4 N/cm. Die Klebkraft auf Polyethylen beträgt 5,3 N/cm. Nach Alterung beträgt die Klebkraft auf Polyethylen noch 95 % der ursprünglichen Klebkraft. Das Klebeband lässt sich auch bei 0 °C auf Mauerwerk, sägerauem Holz, Polyethylen-Folie oder Polyamid-Folie verkleben.

### Beispiel F6

Klebemasse wie in Beispiel F1 jedoch mit folgender Rezeptur:

| | |
|---|---|
| 100 phr | IN FUSE 9107, |
| 78,4 phr | Ondina 933, |
| 212 phr | Wingtack extra |
| 2 phr | Irganox 1076. |

Die Klebemasse wird in einem Extruder kontinuierlich hergestellt und mit 200 g/m² auf ein Trennpapier mittels Düsenbeschichtung aus der Schmelze aufgetragen. Das Trägermaterial besitzt eine Dicke von 100 µm und besteht aus mit Polyethylen beschichtetem Kraftpapier (20 g/m² Polyethylen). Der Klebmassenauftrag erfolgt auf die Seite des Trägermaterials aus Kraftpapier durch Kaschieren von beschichtetem Trennpapier. Das Klebeband wird, ohne das Trennpapier wieder auszudecken, zu Stangen gewickelt.

Klebkraft auf Stahl beträgt 16,3 N/cm. Die Klebkraft auf Polyethylen beträgt 10,1 N/cm. Nach Alterung beträgt die Klebkraft auf Polyethylen noch 92 % der ursprünglichen Klebkraft. Das Klebeband lässt sich auch bei 0 °C auf Mauerwerk, sägerauem Holz, Polyethylen-Folie oder Polyamid-Folie verkleben.

### Beispiel F7

Klebemasse wie in Beispiel F1 jedoch mit folgender Rezeptur:

| | |
|---|---|
| 100 phr | IN FUSE 9107, |
| 78,4 phr | Wingtack 10 |
| 212 phr | Wingtack extra |
| 2 phr | Irganox 1076. |

Die Klebemasse wird wie in Beispiel F2 beschichtet und das Klebeband analog hergestellt.

Klebkraft auf Stahl beträgt 5,3 N/cm. Die Klebkraft auf Polyethylen beträgt 3,6 N/cm. Nach Alterung beträgt die Klebkraft auf Polyethylen noch 89 % der ursprünglichen Klebkraft. Das Klebeband lässt sich auch bei 0 °C auf Mauerwerk, sägerauem Holz, Polyethylen-Folie oder Polyamid-Folie verkleben.

### Vergleichsbeispiel F1

Die Ausführung erfolgt wie in Beispiel F1 beschrieben, jedoch besteht die Masse entsprechend marktüblichen Rezepturen aus

| | |
|---|---|
| 100 phr | Vector 4113, |
| 97 phr | Escorez 1310, |
| 21 phr | Ondina 933 und |
| 1 phr | Irganox 1726. |

Die Klebkraft auf Polyethylen beträgt 8,1 N/cm. Nach Alterung beträgt die Klebkraft auf Polyethylen noch 74 % der ursprünglichen Klebkraft, was einem deutlichen Klebkraft-Abfall und einer starken Alterung entspricht.

### Vergleichsbeispiel F2

Die Ausführung erfolgt wie in Beispiel F1, Die Klebemasse besteht aus folgenden Komponenten:

| | |
|---|---|
| 100 phr | IN FUSE 9107 |
| 78,4 phr | PB 0300 M |
| 212 phr | Escorez 5400 |
| 8 phr | Irganox 1076. |

Die Masse ist kaum klebrig.

### Vergleichsbeispiel F3

Als Klebemasse wird eine wässrige Acrylatdispersion der Firma Rohm and Haas mit der Bezeichnung Primal PS83D (Feststoffgehalt 53 Gew.-%; Ammoniakgehalt < 0,2 Gew.-%; pH-Wert 9,1 bis 9,8) eingesetzt.
Die Beschichtung der Trennfolie mit der Klebemasse erfolgt durch ein Drahtrakel. Das Drahtrakel und die Beschichtungsgeschwindigkeit werden so eingestellt, dass nach der Trocknung der beschichteten Folie ein Masseauftrag von ca. 100 g/m² gemessen wird. Beschichtungsgeschwindigkeit und Trocknerleistung werden so eingestellt, dass nach der Trocknung in der Klebemasse ein Wassergehalt von 0,03 bis 0,13 Gew.-% gemessen wird. Die in Beispiel F2 beschriebene Folie wird einseitig Corona-behandelt. Der Klebemassenauftrag erfolgt auf die Corona-behandelte Seite durch Kaschieren von beschichtetem Trennpapier. Nach dem ersten Auftrag einer Schichtdicke von 100 g/m² wird das Trennpapier ausgedeckt und eine zweite Schicht Klebmasse auf die erste Schicht kaschiert, so dass ein Masseauftrag von ca. 200 g/m² erreicht wird.
Durch die schwierige Trocknung der Acrylatdispersion wird ein erhöhter Prozessaufwand notwendig, da die Herstellung einer Schichtdicke von 200 g/m² in einem Arbeitsgang unwirtschaftlich lange Trocknungsdauern der Beschichtung zur Folge hat. Wird die Klebemasse Wasser ausgesetzt, so quillt sie auf und verliert an Festigkeit und Klebkraft. Das erfindungsgemäße Klebeband eignet sich des Weiteren ganz besonders vorteilhaft als Rollen- oder Einzelpflaster, als Stanzling zum Verkleben von Kolostomiebeuteln oder Elektroden, als Wirkstoffpflaster, als Wundabdeckung, als orthopädische oder phlebologische Bandage oder als Inzisionsfolie, und zwar wenn gemäß einer weiteren vorteilhaften Ausführungsform der Erfindung das Klebeband einen Träger und eine darauf zumindest einseitig beschichtete Klebemasse aus einem Olefinpolymer mit einer Dichte zwischen 0,86 und 0,89 g/cm³ und einem Kristallitschmelzpunkt von mindestens 105 °C und einem Klebharz aufweist.

Stark klebende orthopädische Bandagen und andere medizinische Produkte werden üblicherweise vollflächig mit einer Zink-Kautschuk-Klebemasse beschichtet. Das Verkleben derartiger Produkte auf der Haut zeigt dem Ablösen deutliche Hautirritationen und eine mechanische Beanspruchung der Haut. Die Verklebung lässt sich ohne Hilfsmittel nur unter Schmerzen lösen. Fallweise kommt es zu allergischen Reaktionen. Die verwendeten Klebemassen führen darüber hinaus oft zu einem Massetransfer, das heißt einem Umspulen der Klebemasse auf die Haut.

Die Verwendung von hautfreundlichen Klebemassen wie Acrylatklebemassen ist aufgrund der geringen Scherstabilität und Anfassklebrigkeit nicht erwägenswert. Eine Verbesserung durch eine Nachbehandlung, insbesondere Vernetzung, ist möglich, jedoch bleibt das Ergebnis insgesamt unbefriedigend. Weiterhin reicht die Klebkraft auf der Trägerrückseite solcher Systeme bei zirkulär angelegten Verbänden mit mehreren Lagen nicht für einen stabilen funktionalen Verband aus. Die propriozeptive Wirkung ist gegenüber den Systemen mit einer Zink-Kautschuk-Klebemasse geringer.

Andere bekannte Klebesysteme auf Basis von herkömmlichen Blockcopolymeren sind zum einen durch hohen Stabilisatorzusatz nicht hautfreundlich beziehungsweise zeigen durch die hohe Kohäsivität bislang nur eine Eignung für technische Anwendungsfälle, zum anderen sind sie nicht stark hautklebend und hauthaftend einzustellen.
Bei der partiellen Beschichtung zeigt sich aufgrund eines begrenzt möglichen Masseauftrags eine zu geringe Klebkraft, insbesondere bei schweren Trägermaterialien.

Die zuvor genannten Klebemassen sind druckempfindliche Selbstklebemassen, wobei die Massen für die Verarbeitung in einer Trägermatrix vorliegen können. Als Trägermatrix werden gängige organische oder anorganische Lösemittel oder Dispergiermittel verstanden.

Systeme ohne Trägermatrix werden als 100%-Systeme bezeichnet und sind ebenfalls nicht unbekannt. Sie werden im thermoplastischen Zustand verarbeitet. Eine gängige Verarbeitungsweise ist die Schmelze.
Auch solche Haftschmelzklebemassen sind im Stande der Technik bereits vorbeschrieben. Sie basieren auf natürlichen oder synthetischen Kautschuken und/oder anderen synthetischen Polymeren. Aufgrund ihrer hohen Härte ist für solche 100%-Systeme die Hauthaftung problematisch.

Es ist ferner bekannt, derartige Selbstklebemassen nicht nur vollflächig sondern auch rasterpunktförmig aufzubringen, beispielsweise durch Siebdruck (DE 42 37 252 C1), wobei die Klebstoffpünktchen auch unterschiedlich groß und/oder unterschiedlich verteilt sein können (EP 0 353 972 B1), oder durch Tiefdruck von in Längs- und Querrichtung zusammenhängenden Stegen (DE 43 08 649 C1). Der Vorteil des rasterförmigen Auftrags zeigt sich darin, dass die Klebematerialien bei entsprechend porösem Trägermaterial luft- und wasserdampfdurchlässig sowie in der Regel leicht wieder ablösbar sind.

Ein Nachteil dieser Produkte besteht jedoch in der Tatsache, dass bei zu hoher Flächendeckung der an sich undurchlässigen Klebeschicht die Luft- und Wasserdampfdurchlässigkeit sich entsprechend verringert sowie der Klebemassenverbrauch steigt und bei geringer Flächendeckung der Klebeschicht die Klebeeigenschaften leiden, das heißt, das Produkt löst sich insbesondere bei schweren, textilen Trägermaterialien zu leicht vom Untergrund.

An medizinische Produkte, beispielsweise eine orthopädische Binde, werden hohe Anforderungen bezüglich der Klebeeigenschaften gestellt. Für eine ideale Anwendung sollte die Selbstklebemasse eine hohe Anfassklebrigkeit besitzen. Die funktionsangepasste Klebkraft auf der Haut und auf der Trägerrückseite sollte vorhanden sein. Weiterhin ist, damit es zu keinem Verrutschen der Lagen kommt, eine hohe Scherfestigkeit der Selbstklebemasse notwendig.

Die erfindungsgemäße Klebemasse zeigen hervorragende Klebeeigenschaften auf Haut.

Als Klebharze haben sich Harze auf Basis von Kolophonium (zum Beispiel Balsamharz) oder Kolophoniumderivaten (zum Beispiel disproportioniertes, dimerisiertes oder verestertes Kolophonium), vorzugsweise partiell oder vollständig hydriert, als gut geeignet erwiesen.

Vorteilhaft insbesondere für die Verwendung bei medizinischen Produkten ist weiterhin, wenn die Haftschmelzklebemasse partiell auf dem Trägermaterial aufgetragen ist, beispielsweise durch Rasterdruck, Thermosiebdruck oder Tiefdruck, denn im Vollstrich selbstklebend ausgerüstete Trägermaterialien können bei der Applikation mechanische Hautirritationen hervorrufen.

Der partielle Auftrag ermöglicht durch geregelte Kanäle die Abführung des transepidermalen Wasserverlustes und verbessert das Ausdampfen der Haut beim Schwitzen insbesondere bei der Verwendung von luft- und wasserdampfdurchlässigen Trägermaterialien. Hierdurch werden Hautirritationen wie Mazeration, die durch Stauungen der Körperflüssigkeiten hervorgerufen werden, vermieden. Die angelegten Abführungskanäle ermöglichen ein Ableiten auch unter Verwendung eines mehrlagigen Verbandes.

Bevorzugt wird der Auftrag in Form von polygeometrischen Kalotten und ganz besonders von solchen Kalotten, bei denen das Verhältnis Durchmesser zu Höhe kleiner 5:1 ist. Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen und Zickzacklinien.

Ferner kann sie beispielsweise auch aufgesprüht sein, was ein mehr oder weniger unregelmäßiges Auftragsbild ergibt.

Die Klebemasse kann gleichmäßig auf dem Trägermaterial verteilt sein, sie kann aber auch funktionsgerecht für das Produkt über die Fläche unterschiedlich stark oder dicht aufgetragen sein.

Eine Hotmeltselbstklebemasse kann im Thermosiebdruck aufgebracht werden. Das Prinzip des Thermosiebdrucks besteht in der Verwendung einer rotierenden beheizten, nahtlosen, trommelförmigen perforierten Rundschablone, die über eine Düse mit der Haftschmelzklebemasse beschickt wird. Eine speziell geformte Düsenlippe (Rund- oder Vierkantrakel) presst die über einen Kanal eingespeiste Selbstklebemasse durch die Perforation der Schablonenwand auf die vorbei geführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen Klebstoffkalotten geschieht dabei nach folgendem Mechanismus:
Der Düsenrakeldruck fördert die Selbstklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Selbstklebemasse und die innere Kohäsion des Hotmelts wird von der auf dem Träger bereits haften den Basis der Kalotten der in den Löchern begrenzte Vorrat an Haftschmelzklebemasse konturenscharf abgezogen beziehungsweise durch den Rakeldruck auf den Träger gefördert.

Nach Beendigung dieses Transportes formt sich, abhängig von der Rheologie der Haftschmelzklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fliessverhalten, Oberflächenspannung und Benetzungswinkel auf dem Trägermaterial) der Selbstklebemasse ab.

Bei der Siebschablone im Thermosiebdruck kann das Steg/Loch-Verhältnis kleiner 2:1 sein, bevorzugt kleiner oder gleich 1:1.

Der beschriebene Bildungsmechanismus der Kalotten erfordert bevorzugt saugfähige oder zumindest von Haftschmelzklebemasse benetzbare Trägermaterialien. Nichtbenetzende Trägeroberflächen müssen durch chemische physikalische Verfahren vorbehandelt werden. Dies kann durch zusätzliche Maßnahmen wie zum Beispiel Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 µm bis 5000 µm gewählt werden, die Höhe der Kalotten von 20 µm bis ca. 2000 µm, bevorzugt 50 µm bis 1000 µm, wobei der Bereich kleiner Durchmesser für glatte Träger, der mit größerem Durchmesser und größerer Kalottenhöhe für raue oder stark porige Trägermaterialien vorgesehen ist.

Die Positionierung der Kalotten auf dem Träger wird durch die in weiten Grenzen variierbare Geometrie des Auftragswerkes, zum Beispiel Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Der Träger wird bevorzugt mit einer Geschwindigkeit von größer 2 m/min, bevorzugt 20 bis 100 m/min, beschichtet, wobei die Beschichtungstemperatur größer als die Erweichungstemperatur zu wählen ist.

Der prozentuale Anteil der mit der Haftschmelzklebemasse beschichteten Fläche sollte - wie bereits erwähnt - mindestens 20 % betragen und kann bis zu 95 % reichen, für spezielle Produkte bevorzugt 40 % bis 60 % sowie 70 % bis 95 %. Dieses kann gegebenenfalls durch Mehrfachapplikation erreicht werden, wobei gegebenenfalls auch Klebemassen mit unterschiedlichen Eigenschaften eingesetzt werden können.

Das Klebeband weist gemäß einer vorteilhaften Ausführungsform der Erfindung eine Klebkraft auf der Trägerrückseite von mindestens 1,5 N/cm auf, besonders eine Klebkraft zwischen 2,5 N/cm und 5 N/cm. Auf anderen Untergründen können höhere Klebkräfte erreicht werden.

Die Kombination der Selbstklebemasse und der partiellen Beschichtung sichert auf der einen Seite eine sichere Verklebung insbesondere des medizinischen Produktes auf der Haut, auf der anderen Seite sind zumindest visuell erkennbare allergische oder mechanische Hautirritationen ausgeschlossen, auch bei einer Anwendung, die sich über mehrere Tage erstreckt.

Die Epilation entsprechender Körperregionen und der Massetransfer auf die Haut sind aufgrund der hohen Kohäsivität des Klebers vernachlässigbar, weil der Kleber nicht an Haut und Haar verankert, vielmehr ist die Verankerung der Klebemasse auf dem Trägermaterial mit bis zu 12 N/cm (Probenbreite) sehr gut, insbesondere für medizinische Anwendungen.

Durch die ausgeformten Sollbruchstellen in der Beschichtung werden Hautlagen beim Ablösen nicht mehr mit- oder gegeneinander verschoben. Das Nichtverschieben der Hautlagen und die geringere Epilation führen zu einem bisher nicht gekannten Grad der Schmerzfreiheit bei solchen stark klebenden Systemen. Weiter unterstützt die individuelle biomechanische Klebkraftsteuerung, welche eine nachweisliche Absenkung der Klebkraft des Klebebands aufweist, die Ablösbarkeit. Der angelegte Verband zeigt gute propriozeptive Wirkungen.

Je nach Trägermaterial und dessen Temperaturempfindlichkeit kann die Selbstklebemasse direkt aufgetragen sein oder zuerst auf einen Hilfsträger aufgebracht und dann auf den endgültigen Träger transferiert werden.

Als Trägermaterialien eignen sich alle starren und elastischen Flächengebilde aus synthetischen und natürlichen Rohstoffen. Bevorzugt sind Trägermaterialien, die nach Applikation der Klebemasse so eingesetzt werden können, dass sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt.

Das Klebeband kann eine Luftdurchlässigkeit von größer 1 cm³/(cm²*s), bevorzugt größer 15 cm³/(cm²*s), ganz besonders bevorzugt größer 70 cm³/(cm²*s) aufweisen, des weiteren eine Wasserdampfdurchlässigkeit von größer 500 g/(m²*24 h), bevorzugt größer 1000 g/(m²*24 h)), ganz besonders bevorzugt größer 2000 g/(m²*24 h).

Das Klebeband kann darüber hinaus auch im Lagenverbund noch eine Luftdurchlässigkeit von 1 g/(m²*24 h) und eine Wasserdampfdurchlässigkeit von 500 g/(m²*24 h) aufweisen.

Schließlich kann das Klebeband nach der Applikation eingedeckt oder mit einer Wundauflage, Polsterung versehen werden.

Besonders vorteilhaft ist, dass das Klebeband sterilisierbar, insbesondere strahlensterilisierbar, ist, da das Polymer der Klebmasse keine Doppelbindungen enthält, welche zur Vernetzung neigen.

Weiterhin kann der Träger auf der Seite, die der mit Klebemasse beschichteten Seite gegenüberliegt, mit einer wasserabweisenden Schicht oder Imprägnierung ausgerüstet sein, die eine schnelle Durchnässung bei Kontakt mit Wasser oder Schweiß verhindert. Neben den bekannten Imprägnierungen kann dies auch durch das Aufnähen einer Folie, vorteilhafterweise einer wasserdampfdurchlässigen Folie, erfolgen.

Der Träger kann darüber hinaus mit einer die Klebkraft der Klebemasse vermindernden Releaseschicht oder -imprägnierung und/oder Lackierung ausgerüstet sein. Auch hierbei kann neben den bekannten Releasematerialien eine Folie, vorteilhafterweise eine wasserdampfdurchlässige Folie, verwendet werden.

Das erfindungsgemäße Klebeband ist für Anwendungen auf menschlicher Haut hervorragend geeignet, Beispiele sind Rollen- und Einzelpflaster, Stanzlinge zum Verkleben von Kolostomiebeuteln und Elektroden, Wirkstoffpflaster (transdermale Pflaster), Wundabdeckungen und orthopädische oder phlebologische Bandagen, Inzisionsfolien. Die Eignung ergibt sich aus den Klebeigenschaften, aber auch der Möglichkeit, hautreizende beziehungsweise hautirritierende oder anders chemisch wirkende Substanzen wie Antioxidantien zu vermeiden. Die erfindungsgemäßen Klebmasse weist eine ausgezeichnete Balance zwischen Haftung auf der Haut und leichter Ablösbarkeit von der Haut nach der Verwendung ohne Hautirritationen auf.

### Beispiel G1

Die Klebemasse besteht aus folgenden Komponenten:

| | |
|---|---|
| 100 phr | IN FUSE 9107, |
| 78,4 phr | Ondina 933, |
| 212 phr | Wingtack extra |

Die Klebemasse wird in einem Extruder kontinuierlich hergestellt und mit 70g/m² auf den Träger mittels Düsenbeschichtung aus der Schmelze aufgetragen. Der Träger ist eine hautfarbene Folie aus Polyethylen und Polypropylen, welche auf der Unterseite (Beschichtungsseite) mit einem Polypropylenvlies kaschiert ist. Die Klebemasse wird nach dem Aufbringen auf den Träger mit Wundabdeckungsmaterial versehen und mit Liner aus Silikonpapier abgedeckt. Daraus werden Einzelpflaster mit Luftlöchern gestanzt. Die Klebkraft auf Stahl beträgt 9 N/cm. Das Klebeband (Pflaster) zeigte ein reversibles Ablösen von der Haut sowie eine gute Luft- und Wasserdampfdurchlässigkeit. Es werden keine Hautirritationen und eine vernachlässigbar geringe Epilation nach dem Abnehmen des Pflasters beobachtet.

### Beispiel G2

Die Ausführung erfolgt wie in Beispiel G1 beschrieben, jedoch mit folgender Rezeptur:

| | |
|---|---|
| 100 phr | NOTIO PN-0040, |
| 78,4 phr | Wingtack 10, |
| 212 phr | Escorez 1310 und |
| 1 phr | Irganox 1076. |

Sie wird im Schmelzsiebdruck (Siebstärke 300 µm, Mesh-Zahl 25) auf ein Baumwollgewebe (Höchstzugkraft 60 N/cm, Bruchdehnung 10 %) aufgetragen. Der Masseauftrag ist 120 g/m². Die Klebkraft auf Stahl beträgt 11 N/cm. Das Klebeband (Bandage) zeigte ein reversibles Ablösen von der Haut sowie eine gute Luft- und Wasserdampfdurchlässigkeit. Es werden keine Hautirritationen und eine vernachlässigbar geringe Epilation nach dem Abnehmen des Pflasters beobachtet.

### Beispiel G3

Die Ausführung erfolgt wie in Beispiel G1 beschrieben, jedoch mit folgender Rezeptur:

| | |
|---|---|
| 100 phr | Softell CA02, |
| 50 phr | Ondina 933, |
| 212 phr | Regalite R1100 und |
| 20 phr | Salicylsäure. |

Die Masse wird mit 70 g/m² auf ein Celluloseacetatgewebe aufgetragen. Es ist als Warzenpflaster geeignet.

### Vergleichsbeispiel G1

Die Ausführung erfolgt wie in Beispiel G1 beschrieben, jedoch mit LD 251 statt IN FUSE 9107. Die Beschichtung ist nicht haftklebrig sondern hart mit öliger Oberfläche.

## Patentansprüche

1. Klebeband aus einem Träger und einer darauf zumindest einseitig beschichteten Klebemasse aus einem Olefinpolymer mit einer Dichte zwischen 0,86 und 0,89 g/cm³ und einem Kristallitschmelzpunkt von mindestens 105 °C und aus einem Klebharz, wobei die Menge an Klebharz 130 bis 350 phr beträgt.

2. Klebeband nach Anspruch 1,
**dadurch gekennzeichnet, dass**
die Dichte des Olefinpolymers zwischen 0,86 und 0,88 g/cm³, bevorzugt zwischen 0,86 und 0,87 g/cm³ liegt und/oder das Olefinpolymer einen Kristallitschmelzpunkt von mindestens 105 °C, vorzugsweise mindestens 115 °C, besonders bevorzugt mindestens 135 °C aufweist.

3. Klebeband nach Anspruch 1 oder 2,
**dadurch gekennzeichnet, dass**
das Olefinpolymer einen Schmelzindex von weniger als 8 g/10 min, vorzugsweise weniger als 1,5 g/10 min aufweist, und/oder
einen Biegemodul von weniger als 50 MPa, vorzugsweise weniger als 26 und besonders bevorzugt weniger als 17 MPa aufweist.

4. Klebeband nach zumindest einem der Ansprüche 1 bis 3,
**dadurch gekennzeichnet, dass**
das Olefinpolymer Ethylen oder Propylen und mindestens ein weiteres Comonomer ausgewählt aus den C₂- bis C₁₀-Olefinen, vorzugsweise C₂- bis C₁₀-α-Olefinen enthält, besonders bevorzugt ein Copolymer aus Ethylen und Propylen, Ethylen und Buten-(1), Ethylen und Octen-(1), Propylen und Buten-(1), oder ein Terpolymer aus Ethylen, Propylen und Buten-(1) ist.

5. Klebeband nach zumindest einem der vorherigen Ansprüche,
**dadurch gekennzeichnet, dass**
das Olefinpolymer ein Blockcopolymer, ein Pfropfpolymer oder ein heterophasisches Polymer auf Basis Polypropylen ist.

6. Klebeband nach zumindest einem der vorherigen Ansprüche, **dadurch gekennzeichnet, dass**
die Klebmasse ein Klebharz enthält, welches vorzugsweise eine Polydispersität von weniger als 2,1, vorzugsweise weniger als 1,8, besonders bevorzugt weniger als 1,6, ganz besonders bevorzugt zwischen 1,0 und 1,4 aufweist.

7. Klebeband nach zumindest einem der vorherigen Ansprüche,
**dadurch gekennzeichnet, dass**
das Klebharz gewählt ist aus der Gruppe
- der Harze auf Basis von Kolophonium oder Kolophoniumderivaten vorzugsweise partiell oder vollständig hydriert,
- der Kohlenwasserstoffharze auf Basis von C₅-Monomeren, vorzugsweise partiell oder vollständig hydriert,
- der Kohlenwasserstoffharze aus Hydrierung von aromatenhaltigen Kohlenwasserstoffharzen,
- der Kohlenwasserstoffharze auf Basis von hydrierten Cyclopentadien-Polymeren, und/oder
- der Harze auf Basis von Polyterpenen, vorzugsweise partiell oder vollständig hydriert und/oder,
- der Terpenphenolharze,
wobei die Menge an Klebharz in der Klebemasse vorzugsweise 200 bis 240 phr beträgt.

8. Klebeband nach zumindest einem der vorherigen Ansprüche,
**dadurch gekennzeichnet, dass**
die Klebemasse einen Weichmacher enthält gewählt aus der Gruppe der Mineralöle, der flüssigen Polymerisate aus Isobutenhomopolymer und/oder Isobuten-Buten-Copolymer.

9. Klebeband nach zumindest einem der vorhergehenden Ansprüche, **dadurch gekennzeichnet, dass**
die Klebemasse ein Copolymer oder ein Terpolymer aus Ethylen, Propylen, Buten-(1), Hexen-(1) und/oder Octen-(1) enthält, wobei der Biegemodul des Copolymers oder Terpolymers vorzugsweise unter 10 MPa und der Kristallitschmelzpunkt vorzugsweise unter 50 °C liegen, oder ein EPM oder EPDM enthält, vorzugsweise mit einem Ethylengehalt von 40 bis 70 Gew.-% und/oder einer Dichte unter 0,88, besonders bevorzugt unter 0,87 g/cm³, wobei die Menge an Copolymer oder Terpolymer vorzugsweise über 100 phr beträgt.

10. Klebeband nach zumindest einem der vorherigen Ansprüche,
**dadurch gekennzeichnet, dass**
die Klebemasse
i. ein primäres Antioxidans, vorzugsweise in einer Menge von mindestens 2 phr, besonders bevorzugt mindestens 6 phr und/oder mit einer sterisch gehinderten phenolischen Gruppe,
ii. ein sekundäres Antioxidans in einer Menge von 0 bis 5 phr, vorzugsweise in einer Menge von 0,5 bis 1 phr und/oder aus der Klasse der Schwefelverbindungen oder der Klasse der Phosphite
iii. ein Lichtschutzmittel vorzugsweise ein HALS und/oder
iv. einen UV-Absorber enthält.

11. Klebeband nach zumindest einem der vorherigen Ansprüche,
**dadurch gekennzeichnet, dass**
die Klebemasse im Wesentlichen Mineralöl frei ist, insbesondere durch Verwendung eines Mineralöl freien Weichmachers.

12. Klebeband nach zumindest einem der vorherigen Ansprüche,
**dadurch gekennzeichnet, dass**
die Klebemasse mit 10 bis 300 g/m², vorzugsweise mit 70 bis 160 g/m² auf dem Träger aufgebracht ist.

13. Verwendung eines Klebebandes nach zumindest einem der vorhergehenden Ansprüche als Rollen- oder Einzelpflaster, Stanzling zum Verkleben von Kolostomiebeuteln oder Elektroden, Wirkstoffpflaster, Wundabdeckungen oder orthopädische oder phlebologische Bandagen, Inzisionsfolien.

14. Verwendung eines Klebebandes gemäß einem der vorhergehenden Ansprüche für Verpackungsanwendungen vorzugsweise zur Verstärkung von Kartonagen, insbesondere im Bereich von Stanzungen, als Aufreißstreifen, als Tragegriff, zur Palettensicherung, als Transportsicherung von Waren, zum Bündeln und insbesondere zum Verschließen von Faltkartons.

15. Verwendung nach Anspruch 14,
**dadurch gekennzeichnet, dass**
die Klebemasse lösungsmittelfrei auf dem Träger aufgebracht ist.

16. Verwendung nach Anspruch 14 oder 15,
**dadurch gekennzeichnet, dass**
das Olefinpolymer ein Ethylenpolymer ist.

17. Verwendung eines Klebebandes gemäß einem der vorhergehenden Ansprüche als Wickelband zum Bündeln, Schützen, Kennzeichnen, Isolieren oder Abdichten von Lüftungsrohren oder Drähten oder Kabeln und vorzugsweise zum Ummanteln von Kabelsätzen in Fahrzeugen.

18. Verwendung nach Anspruch 17,
**dadurch gekennzeichnet, dass**
der Träger
- ein primäres Antioxidans, vorzugsweise in einer Menge von mindestens 2 phr, besonders bevorzugt mindestens 6 phr und/oder
- ein sekundäres Antioxidans in einer Menge von 0 bis 5 phr, vorzugsweise in einer Menge von 0,5 bis 1 phr enthält.

19. Verwendung eines Klebebandes gemäß einem der vorhergehenden Ansprüche als Kabelbandagierungsband.

20. Verwendung nach Anspruch 19,
**dadurch gekennzeichnet, dass**
die Klebemasse lösungsmittelfrei auf dem Träger aufgebracht ist.

21. Verwendung nach Anspruch 19 oder 20,
**dadurch gekennzeichnet, dass**
das Olefinpolymer ein Ethylenpolymer ist.

22. Verwendung nach zumindest einem der Ansprüche 19 bis 21,
**dadurch gekennzeichnet, dass**
der Träger ein textiler Träger ist.

23. Verwendung eines Klebebandes gemäß einem der vorhergehenden Ansprüche für Außenanwendungen, wobei das Klebeband einem textilen Träger mit einem Flächengewicht von 15 bis 150 g/m² aufweist, welcher auf der Oberseite mit einer Zusatzschicht versehen ist, die durch Extrusionsbeschichtung, durch Dispersionsbeschichtung oder durch Folienlaminierung aufgebracht ist, und welcher auf der Unterseite mit einer Klebemasse aus einem Ethylenpolymer mit einer Dichte zwischen 0,86 und 0,89 g/cm³ und einem Kristallitschmelzpunkt von mindestens 105 °C und aus einem Klebharz ausgerüstet ist.

24. Verwendung eines Klebebandes nach mindestens einem der vorherigen Ansprüche zum Verkleben auf rauen und/oder verschmutzten Oberflächen, bevorzugt zum Abdichten von Fugen, als Putzband, wobei das Klebeband aus einem Träger und einer darauf zumindest einseitig aus der Schmelze beschichteten Klebemasse aus einem Ethylenpolymer mit einer Dichte zwischen 0,86 und 0,89 g/cm³ und einem Kristallitschmelzpunkt von mindestens 105 °C und einem Klebharz besteht.

25. Verwendung nach Anspruch 24 als ein- beziehungsweise doppelseitig klebendes Montageband für Winddichtungen, Dampfbremsen und Dampfsperren.

26. Verwendung eines Klebebandes nach mindestens einem der vorherigen Ansprüche zum als Rollen- oder Einzelpflaster, als Stanzling zum Verkleben von Kolostomiebeuteln oder Elektroden, als Wirkstoffpflaster, als Wundabdeckung, als orthopädische oder phlebologische Bandage oder als Inzisionsfolie, wobei das Klebeband einen Träger und eine darauf zumindest einseitig beschichtete Klebemasse aus einem Olefinpolymer mit einer Dichte zwischen 0,86 und 0,89 g/cm³ und einem Kristallitschmelzpunkt von mindestens 105 °C und einem Klebharz aufweist.

## Claims

1. Adhesive tape comprising a backing and an adhesive which is coated at least one-sidedly thereon and comprises an olefin polymer having a density of between 0.86 and 0.89 g/cm³ and having a crystallite melting point of at least 105°C, and comprises a tackifier resin, the amount of tackifier resin being 130 to 350 phr.

2. Adhesive tape according to Claim 1,
**characterized in that**
the density of the olefin polymer is between 0.86 and 0.88 g/cm³, preferably between 0.86 and 0.87 g/cm³, and/or the olefin polymer has a crystallite melting point of at least 105°C, preferably at least 115°C, more preferably at least 135°C.

3. Adhesive tape according to Claim 1 or 2,
**characterized in that**
the olefin polymer has a melt index of less than 8 g/10 min, preferably less than 1.5 g/10 min, and/or
has a flexural modulus of less than 50 MPa, preferably less than 26 MPa, and more preferably less than 17 MPa.

4. Adhesive tape according to at least one of Claims 1 to 3,
**characterized in that**
the olefin polymer comprises ethylene or propylene and at least one further comonomer selected from the C₂ to C₁₀ olefins, preferably C₂ to C₁₀ α-olefins, more preferably a copolymer of ethylene and propylene, of ethylene and but-1-ene, of ethylene and oct-1-ene, of propylene and but-1-ene, or a terpolymer of ethylene, propylene, and but-1-ene.

5. Adhesive tape according to at least one of the preceding claims,
**characterized in that**
the olefin polymer is a block copolymer, a graft polymer or a heterophasic polymer based on polypropylene.

6. Adhesive tape according to at least one of the preceding claims,
**characterized in that**
the adhesive comprises a tackifier resin which preferably has a polydispersity of less than 2.1, preferably less than 1.8, more preferably less than 1.6, very preferably between 1.0 and 1.4.

7. Adhesive tape according to at least one of the preceding claims,
**characterized in that**
the tackifier resin is selected from the group
- of resins based on rosin or rosin derivatives, preferably partially or completely hydrogenated,
- of hydrocarbon resins based on C₅ monomers, preferably partially or completely hydrogenated,
- of hydrocarbon resins from hydrogenation of aromatics-containing hydrocarbon resins,
- of hydrocarbon resins based on hydrogenated cyclopentadiene polymers, and/or
- of resins based on polyterpenes, preferably partially or completely hydrogenated, and/or
- of terpene-phenolic resins,
the amount of tackifier resin in the adhesive being 200 to 240 phr.

8. Adhesive tape according to at least one of the preceding claims,
**characterized in that**
the adhesive comprises a plasticizer selected from the group of mineral oils, of liquid polymers of isobutene homopolymer and/or isobutene-butene copolymer.

9. Adhesive tape according to at least one of the preceding claims,
**characterized in that**
the adhesive comprises a copolymer or terpolymer of ethylene, propylene, but-1-ene, hex-1-ene and/or oct-1-ene, the flexural modulus of the copolymer or terpolymer being preferably below 10 MPa and the crystallite melting point being preferably below 50°C, or comprises an EPM or EPDM, preferably having an ethylene content of 40% to 70% by weight and/or a density below 0.88, more preferably below 0.87 g/cm³, the amount of copolymer or terpolymer being preferably above 100 phr.

10. Adhesive tape according to at least one of the preceding claims,
**characterized in that**
the adhesive comprises
i. a primary antioxidant, preferably in an amount of at least 2 phr, more preferably at least 6 phr, and/or with a sterically hindered phenolic group,
ii. a secondary antioxidant in an amount of 0 to 5 phr, preferably in an amount of 0.5 to 1 phr, and/or from the class of the sulphur compounds or from the class of the phosphites,
iii. a light stabilizer, preferably a HALS, and/or
iv. a UV absorber.

11. Adhesive tape according to at least one of the preceding claims,
**characterized in that**
the adhesive is substantially mineral oil-free, more particularly through use of a mineral oil-free plasticizer.

12. Adhesive tape according to at least one of the preceding claims,
**characterized in that**
the adhesive is applied at 10 to 300 g/m², preferably at 70 to 160 g/m², on the carrier.

13. Use of an adhesive tape according to at least one of the preceding claims as a roll plaster or individual plaster, diecut for bonding colostomy bags or electrodes, active-substance patch, wound covering or orthopaedic or phlebological bandage, or incision film.

14. Use of an adhesive tape according to any of the preceding claims for packaging applications, preferably for reinforcing cardboard packaging, more particularly in the region of diecuts, as a tear-open strip, as a carry handle, for pallet securement, as transmit securement of goods, for bundling, and more particularly for the sealing of folding cartons.

15. Use according to Claim 14,
**characterized in that**
the adhesive is applied solventlessly on the carrier.

16. Use according to Claim 14 or 15,
**characterized in that**
the olefin polymer is an ethylene polymer.

17. Use of an adhesive tape according to any of the preceding claims as a winding tape for bundling, protecting, labelling, insulating or sealing ventilation pipes or wires or cables, and preferably for wrapping cable harnesses in vehicles.

18. Use according to Claim 17,
**characterized in that**
the carrier comprises
- a primary antioxidant, preferably in an amount of at least 2 phr, more preferably at least 6 phr, and/or
- a secondary antioxidant in an amount of 0 to 5 phr, preferably in an amount of 0.5 to 1 phr.

19. Use of an adhesive tape according to any of the preceding claims as cable bandaging tape.

20. Use according to Claim 19,
**characterized in that**
the adhesive is applied solventlessly on the carrier.

21. Use according to Claim 19 or 20,
**characterized in that**
the olefin polymer is an ethylene polymer.

22. Use according to at least one of Claims 19 to 21,
**characterized in that**
the carrier is a textile carrier.

23. Use of an adhesive tape according to any of the preceding claims for exterior applications, the adhesive tape having a textile carrier having a basis weight of 15 to 150 g/m², which is provided on the top face with an additional layer which is applied by extrusion coating, by dispersion coating or by film lamination, and which is equipped on the bottom face with an adhesive comprising an ethylene polymer having a density of between 0.86 and 0.89 g/cm³ and a crystallite melting point of at least 105°C, and comprising a tackifier resin.

24. Use of an adhesive tape according to at least one of the preceding claims for adhesive bonding on rough and/or soiled surfaces, preferably for sealing joints, as plaster tape, the adhesive tape being composed of a carrier and an adhesive which is coated from the melt at least one-sidedly thereon and comprises an ethylene polymer having a density of between 0.86 and 0.89 g/cm³ and a crystallite melting point of at least 105°C, and of a tackifier resin.

25. Use according to Claim 24 as a single- or double-sidedly adhesive assembly tape for wind seals, vapour diffusion retarders, and vapour barriers.

26. Use of an adhesive tape according to at least one of the preceding claims as a roll plaster or individual plaster, as a diecut for bonding colostomy bags or electrodes, as an active substance patch, as a wound cover, as an orthopaedic or phlebological bandage or as an incision film, the adhesive having a carrier and an adhesive which is coated at least one-sidedly thereon and comprises an olefin polymer having a density of between 0.86 and 0.89 g/cm³ and a crystallite melting point of at least 105°C, and comprises a tackifier resin.

## Revendications

1. Bande adhésive constituée par un support et une masse adhésive, revêtue sur au moins une face de celui-ci, constituée par un polymère oléfinique présentant une densité entre 0,86 et 0,89 g/cm³ et un point de fusion des cristaux d'au moins 105°C et par une résine adhésive, la quantité de résine adhésive étant de 130 à 350 phr.

2. Bande adhésive selon la revendication 1, **caractérisée en ce que** la densité du polymère oléfinique se situe entre 0,86 et 0,88 g/cm³, de préférence entre 0,86 et 0,87 g/cm³ et/ou le polymère oléfinique présente un point de fusion des cristaux d'au moins 105°C, de préférence d'au moins 115°C, de manière particulièrement préférée d'au moins 135°C.

3. Bande adhésive selon la revendication 1 ou 2, **caractérisée en ce que** le polymère oléfinique présente un indice de fusion inférieur à 8 g/10 min, de préférence inférieur à 1,5 g/10 min et/ou présente un module de flexion inférieur à 50 MPa, de préférence inférieur à 26 MPa et de manière particulièrement préférée inférieur à 17 MPa.

4. Bande adhésive selon au moins l'une quelconque des revendications 1 à 3, **caractérisée en ce que** le polymère oléfinique contient de l'éthylène ou du propylène et au moins un autre comonomère, choisi parmi les C₂-C₁₀-oléfines, de préférence les C₂-C₁₀-α-oléfines, et est de manière particulièrement préférée un copolymère d'éthylène et de propylène, d'éthylène et de butène-(1), d'éthylène et d'octène-(1), de propylène et de butène-(1) ou un terpolymère d'éthylène, de propylène et de butène-(1).

5. Bande adhésive selon au moins l'une quelconque des revendications précédentes, **caractérisée en ce que** le polymère oléfinique est un copolymère à blocs, un polymère greffé ou un polymère hétérophasique à base de polypropylène.

6. Bande adhésive selon au moins l'une quelconque des revendications précédentes, **caractérisée en ce que** la masse adhésive contient une résine adhésive, qui présente de préférence une polydispersité inférieure à 2,1, de préférence inférieure à 1,8, de manière particulièrement préférée inférieure à 1,6, de manière tout particulièrement préférée entre 1,0 et 1,4.

7. Bande adhésive selon au moins l'une quelconque des revendications précédentes, **caractérisée en ce que** la résine adhésive est choisie dans le groupe formé par
- les résines à base de colophonium ou de dérivés de colophonium, de préférence partiellement ou totalement hydrogénées,
- les résines hydrocarbonées à base de monomères en C₅, de préférence partiellement ou totalement hydrogénées,
- les résines hydrocarbonées provenant de l'hydrogénation de résines hydrocarbonées contenant des aromatiques,
- les résines hydrocarbonées à base de polymères hydrogénés de cyclopentadiène, et/ou
- les résines à base de polyterpènes, de préférence partiellement ou totalement hydrogénées et/ou
- les résines de terpènephénol,
la quantité de résine adhésive dans la masse adhésive étant de préférence de 200 à 240 phr.

8. Bande adhésive selon au moins l'une quelconque des revendications précédentes, **caractérisée en ce que** la masse adhésive contient un plastifiant, choisi dans le groupe des huiles minérales, des polymères liquides d'homopolymère d'isobutène et/ou de copolymère d'isobutène-butène.

9. Bande adhésive selon au moins l'une quelconque des revendications précédentes, **caractérisée en ce que** la masse adhésive contient un copolymère ou un terpolymère d'éthylène, de propylène, de butène-(1), d'hexène-(1) et/ou d'octène-(1), le module de flexion du copolymère ou du terpolymère étant de préférence inférieur à 10 MPa et le point de fusion des cristaux étant de préférence inférieur à 50°C, ou contient un EPM ou un EPDM, présentant de préférence une teneur en éthylène de 40 à 70% en poids et/ou une densité inférieure à 0,88 g/cm³, de manière particulièrement préférée inférieure à 0,87 g/cm³, la quantité de copolymère ou de terpolymère étant de préférence supérieure à 100 phr.

10. Bande adhésive selon au moins l'une quelconque des revendications précédentes, **caractérisée en ce que** la masse autoadhésive contient
i. un antioxydant primaire, de préférence en une quantité d'au moins 2 phr, de manière particulièrement préférée d'au moins 6 phr et/ou présentant un groupe phénolique stériquement encombré,
ii. un antioxydant secondaire en une quantité de 0 à 5 phr, de préférence en une quantité de 0,5 à 1 phr et/ou de la classe des composés soufrés ou de la classe des phosphites,
iii. un agent de protection contre la lumière, de préférence un HALS (stabilisant à la lumière de type amine stériquement encombrée) et/ou
iv. un absorbant des UV.

11. Bande adhésive selon au moins l'une quelconque des revendications précédentes, **caractérisée en ce que** la masse adhésive est essentiellement exempte d'huile minérale, en particulier par l'utilisation d'un plastifiant exempt d'huile minérale.

12. Bande adhésive selon au moins l'une quelconque des revendications précédentes, **caractérisée en ce que** la masse adhésive est appliquée sur le support à raison de 10 à 300 g/m², de préférence de 70 à 160 g/m².

13. Utilisation d'une bande adhésive selon au moins l'une quelconque des revendications précédentes, comme pansement en rouleau ou individuel, pièce découpée pour le collage de poches de colostomie ou d'électrodes, pansement à principe actif, recouvrements de plaies ou bandes orthopédiques ou phlébologiques, feuilles d'incision.

14. Utilisation d'une bande adhésive selon au moins l'une quelconque des revendications précédentes pour des utilisations d'emballage, de préférence pour le renforcement de cartonnages, en particulier dans le domaine des découpages, comme bandelettes d'ouverture par déchirure, comme poignée de transport, pour la sécurisation de palettes, pour la sécurisation lors du transport de marchandises, pour empaqueter et en particulier pour fermer des boîtes en carton pliables.

15. Utilisation selon la revendication 14, **caractérisée en ce que** la masse adhésive est appliquée sans solvant sur le support.

16. Utilisation selon la revendication 14 ou 15, **caractérisée en ce que** le polymère oléfinique est un polymère d'éthylène.

17. Utilisation d'une bande adhésive selon l'une quelconque des revendications précédentes comme bande d'enroulement pour empaqueter, protéger, caractériser, isoler ou étanchéifier des tuyaux d'aération ou des fils ou des câbles et de préférence pour envelopper des ensembles de fils dans des véhicules.

18. Utilisation selon la revendication 17, **caractérisée en ce que** le support contient
- un antioxydant primaire, de préférence en une quantité d'au moins 2 phr, de manière particulièrement préférée d'au moins 6 phr et/ou
- un antioxydant secondaire en une quantité de 0 à 5 phr, de préférence en une quantité de 0,5 à 1 phr.

19. Utilisation d'une bande adhésive selon l'une quelconque des revendications précédentes comme bande d'enveloppement de câbles.

20. Utilisation selon la revendication 19, **caractérisée en ce que** la masse adhésive est appliquée sans solvant sur le support.

21. Utilisation selon la revendication 19 ou 20, **caractérisée en ce que** le polymère oléfinique est un polymère d'éthylène.

22. Utilisation selon au moins l'une quelconque des revendications 19 à 21, **caractérisée en ce que** le support est un support textile.

23. Utilisation d'une bande adhésive selon l'une quelconque des revendications précédentes pour des applications extérieures, la bande adhésive présentant un support textile présentant un poids surfacique de 15 à 150 g/m², qui est muni, sur sa face supérieure, d'une couche supplémentaire qui est appliquée par revêtement par extrusion, par revêtement à partir d'une dispersion ou par laminage de feuilles et qui est apprêté sur la face inférieure avec une masse adhésive constituée par un polymère d'éthylène présentant une densité entre 0,86 et 0,89 g/cm³ et un point de fusion des cristaux d'au moins 105°C et par une résine adhésive.

24. Utilisation d'une bande adhésive selon au moins l'une quelconque des revendications précédentes pour le collage sur des surfaces rugueuses et/ou souillées, de préférence pour étanchéifier des joints, comme bande à enduit, la bande adhésive étant constituée par un support et une masse adhésive revêtue à partir de la masse fondue sur au moins une face de celui-ci, constituée par un polymère d'éthylène présentant une densité entre 0,86 et 0,89 g/cm³ et un point de fusion des cristaux d'au moins 105°C et par une résine adhésive.

25. Utilisation selon la revendication 24 comme bande de montage adhésive monoface ou double face pour les isolations contre le vent, les pare-vapeur et les écrans anticondensation.

26. Utilisation d'une bande adhésive selon au moins l'une quelconque des revendications précédentes, comme pansement en rouleau ou individuel, comme pièce découpée pour le collage de poches de colostomie ou d'électrodes, comme pansement à principe actif, comme recouvrement de plaies, comme bandage orthopédique ou phlébologique, ou comme feuille d'incision, la bande adhésive présentant un support et une masse adhésive revêtue sur au moins une face de celui-ci, constituée par un polymère oléfinique présentant une densité entre 0,86 et 0,89 g/cm³ et un point de fusion de cristaux d'au moins 105°C et par une résine adhésive.
